(19) Europäisches Patentamt
European Patent Office
Office européen des brevets

(11) EP 3 015 864 A1

(12) **EUROPEAN PATENT APPLICATION**
published in accordance with Art. 153(4) EPC

(43) Date of publication:
**04.05.2016 Bulletin 2016/18**

(21) Application number: **14811734.4**

(22) Date of filing: **13.06.2014**

(51) Int Cl.:
***G01N 33/574*** *(2006.01)* ***G01N 33/68*** *(2006.01)*

(86) International application number:
**PCT/ES2014/070495**

(87) International publication number:
**WO 2014/198995 (18.12.2014 Gazette 2014/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.06.2013 ES 201330897**
**14.06.2013 ES 201330896**

(71) Applicants:
- **Universidad de Granada**
**E-18071 Granada (ES)**
- **Servicio Andaluz de Salud**
**41071 Sevilla (ES)**
- **Universidad De Jaen**
**23071 Jaen (ES)**

(72) Inventors:
- **TORRES PERALES, Carolina**
**18071 Granada (ES)**
- **PERALES ROMERO, Sonia**
**18071 Granada (ES)**
- **DELGADO PÉREZ, Juan Ramón**
**18014 Granada (ES)**
- **IRIGOYEN MEDINA, Antonio**
**18014 Granada (ES)**
- **ALEJANDRE PÉREZ, María José**
**18071 Granada (ES)**
- **IGLESIAS GÓMEZ, José**
**18071 Granada (ES)**
- **PALOMINO MORALES, Rogelio**
**18071 Granada (ES)**
- **CABA PÉREZ, Octavio**
**23071 Jaén (ES)**
- **PRADOS SALAZAR, José Carlos**
**18071 Granada (ES)**
- **ARÁNEGA JIMÉNEZ, Antonia**
**18071 Granada (ES)**
- **LINARES GIL, Ana**
**18071 Granada (ES)**

(74) Representative: **Hoffmann Eitle**
**Hoffmann Eitle SLU**
**Paseo de la Habana 9-11**
**28036 Madrid (ES)**

(54) **BIOMARKERS FOR THE DIAGNOSIS AND THE RESPONSE TO TREATMENT OF PANCREATIC CANCER**

(57) Biomarkers, method and early diagnosis kit for cancer of the pancreas, in particular, of ductal adenocarcinoma of the pancreas (DACP) and biomarkers, method and kit or device to predict or prognosticate the response of an individual to combination treatment with a nucleoside analogue (preferably gemcitabine) and with a growth receptor factor (preferably erlotinib) in patients with ductal adenocarcinoma of the pancreas.

EP 3 015 864 A1

## Description

### FIELD OF INVENTION

This invention is within the field of Molecular Biology and Medicine. Specifically, it relates to a method for obtaining data useful for the early diagnosis of ductal adenocarcinoma of the pancreas. Early diagnosis is performed by analysis of biomarkers *FGF-10, CXCL11, OSM, GPNMB* and *SCF.* It also relates to a method for obtaining data useful for predicting or prognosticating response to combined treatment with a nucleoside analogue (gemcitabine) with a growth receptor factor (erlotinib) in patients with ductal adenocarcinoma of the pancreas. The prognosis is performed by analysis of serum biomarkers CD80, EG-VEGF, IL-29, NRG1-beta1 and PD-ECGF.

### BACKGROUND OF THE INVENTION

Ductal adenocarcinoma of the pancreas

Ductal adenocarcinoma of the pancreas (DACP), though it only accounts for 2.68% of all cancers, has one of the highest mortality rates among all types of cancer.. The new cases of this cancer reached 44,000 individuals last year (2012) and 37,400 of them died (85%). Men and women have approximately a similar risk of suffering it (Siegel et al., 2012. C.A. Cancer J. Clin. 62, 10-29).

The reasons for low treatment response include late diagnosis, as in most cases when cancer is detected metastasis and intrinsic mechanisms of resistance to chemotherapy have occurred (Hidalgo, 2010. N. Engl. J. Med. 362, 1605-1617; Sakar et al. 2007. Toxicol. Appl. Pharmacol. 224, 326-336). Due to the impact of early diagnosis in patient survival (Agarwal et al., 2008. Pancreas 36, 15-20), better diagnostic tools are required for detection and evaluation of the disease. Serum is the best option to obtain samples where to study malignant tumours as it can be taken by non-invasive methods. In the case of cancer of the pancreas it is particularly useful, as access to the target organ is very difficult. For these reasons, biomarkers of cancer based on serum are the easiest screening tests. To date, the only biomarker based on serum for DACP used is carbohydrate antigen 19-9 (CA), with sensitivity ranging from 70 to 90% and specificity from 70 to 98%, depending on the tumour size (Chan et al., 2012. J. Proteomics*).* However, this biomarker is not detected if tumour size is under 2 centimetres and for lack of expression in 10% of the patients. This also occurs in other diseases. Therefore, since recently the American Society of Oncology does not recommend CA 19-9 for DACP screening tests (Duffy et al., 2010. Ann. Oncl. 21, 441-447). There is a close relationship between inflammatory conditions and carcinogenesis (Mantovani et al., 2008. Nature 454, 436-444; Germano et al., 2008. Cytokine 43, 374-379). Cancer of the pancreas is characterised by a dense desmoplastic reaction, which represents a major barrier that prevents effective release of chemotherapy agents at the main site of the disease. In addition, the tumour complex microenvironment nourishes invasion and metastasis by cancer of the pancreas (Shields et al., 2012. Biochem J. 441, 541-542; Nesse et al., 2011. Gut. 60, 861-868; Luo et al., 2012. Biochim. Biophys Acta. 1826, 170-178). Cytokines released by cancer cells or by cells inside the tumour microenvironment are the architects of this reaction. Based on the above, we consider that a modulation of cytokine levels could reflect the processes leading to the development of the disease or chemoresistance. Antibody-based arrays represent a useful tool for the discovery of cancer biomarkers. This method is remarkable for its capacity to provide fast, adequate perceptions for early detection of cancer biomarkers, providing new approaches for therapies against cancer (Breman et al., 2010. Nat. Rev. Cancer 10, 605-617). Therefore, the identification and validation of individual biomarkers or sets of biomarkers for fast detection of DACP (diagnostic biomarkers) would help increase patient survival.

On the other hand, the prognosis for patients with this disease is discouraging. Less than 5% of the patients reach 5 years of survival after the diagnosis due to the very low effect of chemotherapy in the treatment of disease and the metastatic condition of the tumour at the time of diagnosis. The disease is developed through a poorly known complex process, that covers a mix of mutations and multiple disorders in the cell signalling pathways. All of this explains the heterogeneity of this condition and the differences observed in the results between the different patients. In recent years no improvements have occurred to increase survival of patients with DACP (Hidalgo, 2010. Pancreatic cancer. N. Engl. J. Med. 362, 1605-1617; Costello et al., 2012. Nat. Rev. Gastroenterol Hepatol. 9, 435-444.)

Biomarkers

A biological marker (biomarker), according to NIH Biomarker, is a characteristic that can be objectively measured and evaluated as an indicator of normal biological processes, pathological conditions or of pharmacological responses after a therapeutic procedure (Fong et al., 2012. Cancer J. 18, 530-538.).

In DACP three types of biomarkers are useful: those helping to detect the disease (diagnostic biomarkers); those predicting the response to treatments (predictive biomarkers) and those predicting the most probable course of the disease, including survival and recurrence pattern (prognostic biomarkers). The prognostic biomarkers can guide the treatment plants, helping to identify patients that could benefit from more aggressive interventions, new chemotherapy regimens and also help to establish new expectations for physicians and patients. In recent years, multiple investigations have been performed on biomarkers for the prognosis of DACP using immunhistochemistry, Western Blot, CRP, mRNA, proteomics and methods of methylation of DNA (Jamieson et al., 2011. Clin Cancer Res 17, 3316-333; Garcea et al., 2005. Eur. J. Cancer 41, 2213-2236; Luo et al., 2013. Hum. Pathol. 44, 69-76; Mann et al., 2012. PLoS One 7, e51119; Dallol et al., 2012. Cancer Epidemiol. Biomarkers. Prev. 21, 2069-2075).

This study was focused on the inflammatory mediators that could be useful biomarkers for the prognosis of patients with DACP even before they are treated. Disorders have been found in inflammatory cytokine levels of patients with cancer, even in early stages of development. The immune response plays a significant role during carcinogenesis, and circulatory inflammatory markers can be useful biomarkers for the diagnosis and prognosis of cancer (Schetter et al., 2010. Carcinogenesis 31, 37-49; Germano et al., 2008. Cytokine 43, 374-379). Cytokines are signalling molecules and act as key mediators of inflammation or of immune response. The starting hypothesis is based on the essential role of the microenvironment and the desmoplastic reaction in the development and progression of DACP so the pattern of expression of cytokines inside the tumour and the surrounding microenvironment could be potential prognostic biomarkers for DACP.

The objective of the study was to investigate the prognostic power of serum cytokines in response to the tumour in patients with DACP. To identify the most significantly adequate combination of biomarkers a conditional algorithm was used, based on a probabilistic analysis, adjusted to the Cox regression model. An equation was obtained for specific survival in this cohort.

## SHORT DESCRIPTION OF THE INVENTION

Early diagnosis biomarkers

A first aspect of the invention relates to the use of genes (and/or proteins) *FGF-10, CXCL11, OSM, GPNMB* and *SCF*, for the early diagnosis of cancer of the pancreas. The use can be simultaneous or any of the genes (and/or proteins), or any of the combination thereof can be selected.

In a preferred embodiment of this aspect of the invention the cancer of the pancreas is ductal adenocarcinoma of the pancreas (DACP)

Another aspect of the invention relates to a method for obtaining useful data, hereinafter the first method of the invention, for the early diagnosis of cancer of the pancreas, that comprises:

a) Obtaining an isolated sample from the individual.
b) Measuring the product of expression of genes that are selected from the list consisting of: *FGF-10, CXCL11, OSM, GPNMB* and SCF.

The product of expression of all genes can be measured simultaneously or any of the genes (and/or proteins), or any of the combinations thereof can be chosen.

In another preferred embodiment, the method of the invention also comprises:

c) comparing the amounts obtained in step (b) with a reference amount.

In a preferred embodiment of the method of the invention, steps (b) and/or (c) the methods described above can be completely or partially automated.

Another aspect of the invention relates to a diagnostic method for cancer of the pancreas that comprises steps (a)-(c) according to any of claims 3-5, and also comprises assigning the individual of step (a) to the group of individuals suffering cancer of the pancreas when they show an amount of expression of product of genes *FGF-10, CXCL11, OSM, GPNMB and SCF,* higher than the reference amount.

In a preferred embodiment of this aspect of the invention the cancer of the pancreas is ductal adenocarcinoma of the pancreas (DACP)

In a preferred embodiment of the method of the invention, the isolated biological method from an individual of step (a) is a blood sample.

In another preferred embodiment, the detection of the amount of expression of any of genes FGF-10, CXCL11, OSM, GPNMB and SCF is performed by immunoassay.

In another preferred embodiment, the immunoassay is an Enzyme-Linked ImmunoSorbent Assay (ELISA).

Another aspect of the invention relates to an antibody to treat an individual suffering cancer of the pancreas (or alternatively to the use of an antibody for the preparation of a medicine for the treatment of cancer of the pancreas),

that can be identified by a method of the invention, wherein the antibody is selected from anti-FGF-10, anti-CXCL11, anti-OSM, anti-GPNM and anti-SCF, or any of its combinations. Preferably a combination of all the above antibodies is used. In another preferred embodiment of this aspect of the invention, the cancer of the pancreas is ductal adenocarcinoma of the pancreas (DACP).

Another aspect of the invention relates to a pharmaceutical composition that comprises a modulatory agent of at least one of genes that are selected from *FGF-10, CXCL11, OSM; GPNMB* and *SCF*, to treat an individual that suffers cancer of the pancreas (or alternatively to the use of a pharmaceutical composition that comprises a modulatory agent of at least one of genes that are selected from *FGF-10, CXCL11, OSM, GPNMB* and SCF in the preparation of a medicine for the treatment of cancer of the pancreas), and more preferably of ductal adenocarcinoma of the pancreas (DACP), that can be identified by a method of the invention.

Another aspect of this invention relates to a kit or device, hereinafter "first kit of the invention", that comprises the elements necessary to measure the amount of products of expression of genes that are selected from *FGF-10, CXCL11, OSM, GPNMB* and *SCF* or any of its combinations.

In a preferred embodiment, the first kit of this invention comprises the antibodies that are selected from the list consisting of antibodies: anti-FGF-10, anti- CXCL11, anti-OSM, anti-GPNM and anti-SCF, or any of its combinations. Preferably, the kit of the invention comprises simultaneously at least one antibody of each type: anti-FGF-10, anti-CXCL11, anti-OSM, anti-GPNM and anti-SCF.

In another preferred embodiment, the first kit of the invention comprises secondary antibodies or positive and/or negative controls. The kit can also include, with no type of limitation, buffers, protein extraction solutions, agents for preventing contamination, protein degradation inhibitors, etc.

Another aspect of the invention relates to a solid support, or protein chip, that comprises at least one of the antibodies anti-FGF-10, anti- CXCL11, anti-OSM, anti- GPNM and anti-SCF, or any of its combinations, to perform any of the methods of the invention.

Another aspect of the invention relates to a solid support, or DNA chip, that comprises oligonucleotides or single-channel microarrays designed from a known sequence or a mRNA of at least one of genes *FGF-10, CXCL11, OSM, GPNM and SCF.* Preferably, it comprises oligonucleotides that can detect the mRNA of all genes *FGF-10,* EG-VEGF, IL-29, NRG1-beta1 and PD-ECGF.

<u>Treatment response biomarkers</u>

Another aspect of the invention relates to the use of genes (and/or proteins) CD80, EG-VEGF, IL-29, NRG1-beta1 and PD-ECGF, to predict or prognosticate the response of an individual to combination treatment with a nucleoside analogue plus an epidermal growth factor receptor inhibitor, wherein the individual suffers cancer of the pancreas.

In a preferred embodiment, in the treatment the nucleoside analogue is gemcitabine. In another preferred embodiment, in the treatment the epidermal growth factor receptor inhibitor is erlotinib.

In another preferred embodiment, the cancer is ductal adenocarcinoma of the pancreas (DACP).

Another aspect of the invention relates to a method for obtaining useful data, hereinafter third method of the invention, to predict or prognosticate the response of an individual to the treatment of cancer of the pancreas, that comprises:

a) Obtaining an isolated sample from the individual.
b) measuring the product of expression of genes that are selected from the list consisting of: *CD80, EG-VEGF, IL-29, NRG1-beta1* and *PD-ECGF.*

In another preferred embodiment, the third method of the invention also comprises:

c) finding a prognostic index value (PI) with the values of cytokines of step b), applying the formula:

$$IP_{PDAC} = 4.351 \times \textbf{B7-1/CD80} + 0.003 \times \textbf{EG-VEGF / PK1} + 0.081 \times \textbf{IL-29} + 0.020 \times \textbf{NGR1-beta1/HRG1-beta1} + 0.264 \times \textbf{PD-ECGF}$$

Where the individuals with PI>17 show a poor prognosis of the disease. In a preferred embodiment of this aspect, the individuals with PI>17 show a survival of less than five months.

In another preferred embodiment, individuals with PI≤17 show a better prognosis of the disease. In another preferred embodiment of this aspect, individuals with PI≤17 show a survival longer than five months.

In a preferred embodiment of the third method of the invention, the nucleoside analogue is gemcitabine. In another preferred embodiment, the epidermal growth factor receptor inhibitor is erlotinib.

In another preferred embodiment, the cancer is ductal adenocarcinoma of the pancreas (DACP).

In another preferred embodiment of this aspect of the invention, the biological sample (a) is a blood sample, and more preferably serum.

In another preferred embodiment, the detection of the amount of expression of any of genes *CD80, EG-VEGF, IL-29, NRG1-beta1* and *PD-ECGF* is performed by an immunoassay.

In another preferred embodiment, the immunoassay is an Enzyme-Linked ImmunoSorbent Assay (ELISA).

Another aspect of the invention relates to an antibody for treating an individual that suffers cancer of the pancreas, that can be identified by a method of the invention, wherein the antibody is selected from anti-CD80, anti-EG-VEGF, anti-IL-29, anti-NRG1-beta1 and anti-PD-ECGF, or any of its combinations.

Another aspect of the invention relates to a pharmaceutical composition that comprises a modulatory agent of at least one of genes that are selected from *CD80, EG-VEGF, IL-29, NRG1-beta1* and *PD-ECGF,* to treat an individual that suffers cancer of the pancreas (or alternatively to the use of a pharmaceutical composition that comprises a modulatory agent of at least one of the genes that are selected from *CD80, EG-VEGF, IL-29, NRG1-beta1* and *PD-ECGF* in the preparation of a medicine for the treatment of cancer of the pancreas), that can be identified by a method of the invention.

Another aspect of this invention relates to a kit or device, hereinafter second kit of the invention, that comprises the elements necessary to measure the amount of products of expression of genes that are selected from *CD80, EG-VEGF, IL-29, NRG1-beta1* and *PD-ECGF* or any of its combinations.

Even more preferably, the second kit of this invention comprises the antibodies that are selected from the list consisting of antibodies: anti-CD80, anti-EG-VEGF, anti-IL-29, anti-NRG1-beta1 and anti-PD-ECGF, or any of its combinations. Preferably, the second kit of the invention comprises simultaneously at least one antibody of each type: anti-CD80, anti-EG-VEGF, anti-IL29, anti-NRG1-beta1 and anti-PD-ECGF.

Another aspect of the invention relates to a solid support, or protein chip, that comprises at least one of the anti-CD80, anti-EG-VEGF, anti-IL-29, anti-NRG1-beta1 and anti-PD-ECGF antibodies, or any of its combinations or any of its combinations, to perform any of the methods of the invention.

Another aspect of the invention relates to a solid support, or DNA chip, that comprises oligonucleotides or single-channel microarrays designed from a known sequence or a mRNA of at least one of genes *CD80, EG-VEGF, IL-29, NRG1-beta1* and *PD-ECGF.*

Implementation of the methods of the invention

Another aspect of the invention relates to a computer-readable storage medium or a transmissible signal that comprises software instructions that can lead a computer to perform steps of any of the methods of the invention.

## DESCRIPTION OF INVENTION

In the description and the claims the word "comprises" and its variants do not intend to exclude other technical characteristics, additives, components or steps. For the experts in the matter, other objects, advantages and characteristics of the invention will be concluded in part of the description and in part of the practice of the invention.

Definitions

An "isolated biological sample" includes, amongst others, cells, tissues and/or biological fluids of a body, obtained by any method known by an expert in the art.

The term "individual", as used in the description, relates to animals, preferably mammals, and more preferably, humans. The term "individual" does not intend to be limiting in any aspect, and this can be of any age, gender and physical condition.

The expression levels of genes will give a specific profile of genetic expression. The term "expression level", also called "amount of gene product" or "amount of expression product" relates to the biochemical material, wither RNA or protein, result of the expression of a gene. Sometimes a measure of the amount of gene product is used to conclude how active a gene is. "Genetic expression profile" is defined as the genetic profile obtained after measurement of the mRNA and/or protein produced by the genes of interest of biomarkers, that is, by the genes *FGF-10, CXCL11, OSM, GPNMB, SCF, CD80, EG-VEGF, IL-29, NRG1-beta1* and *PD-ECGF,* in an isolated biological sample. The gene expression profile is performed, preferably, determining the levels of mRNA derived from transcription, after extraction of the total RNA present in the isolated biological sample, which can be performed through protocols known in the state of the art.

The detection of the amount of expression product of *FGF-10, CXCL11, OSM, GPNMB, SCF, CD80, EG-VEGF, IL-29, NRG1-beta1* and *PD-ECGF* can be performed by any means known in the state of the art. The authors of this invention have shown that the detection of the amount or the concentration of antibodies against these cytokines on a semi-quantitative or quantitative basis allow to diagnose early individuals suffering ductal adenocarcinoma of the pancreas.

The measurement of the amount or concentration of expression product of these genes, preferably on a semi-quantitative or quantitative basis, can be performed directly or indirectly. The direct measure relates to the measurement of the amount or concentration of the product of expression of genes, based on a signal that is obtained directly from the transcripts of these genes or of the proteins, and that it is related directly to the number of RNA molecules or proteins produced by genes. This signal - that can be also referred to as signal of intensity - can be obtained, for instance, measuring a value of intensity of a chemical or physical property of said products. The indirect measurement includes the measure obtained from a secondary component or a biological measurement system (for instance, the measurement of cell response, ligands, "label" or enzyme reaction products).

The term "amount", as used in the description, relates to, but not limited, the absolute or relative amount of the products of expression of genes or to the amount of antibodies, as well as to any value or parameter related to them or that can be derived from these. These values or parameters comprise values of signal of intensity obtained from any of the physical or chemical properties of aid products of expression obtained by direct measure. In addition, said values or parameters include all those obtained by indirect measurement, for instance, any of the systems of measurement described in another part of this document.

The term "comparison", as used in the description, refers to but is not limited to, the comparison of the amount of products of expression of genes or of the biological sample to be analysed, also called test biological sample, with an amount of products of expression of genes of one or several desired reference samples. The reference sample may be analysed, for instance, simultaneously or consecutively, together with the test biological sample.

The term "marker compound", as used in this description, relates to a compound that can lead to a chromogenic, fluorogenic, radioactive and/or chemoluminiscent signal which allows detection and quantitation of the amount of antibodies against FGF-10, CXCL11, OSM, GPNMB, SCF, *CD80, EG-VEGF, IL-29, NRG1-beta1* and *PD-ECGF.* The marker compound is selected from the list that comprises radioisotopes, enzymes, fluorophores or any molecule susceptible of being conjugated with another molecule or detected and/or measured directly. This marker compound can bind to the antibody directly or through another compound. Some examples of marker compounds binding directly are, though not limited to, enzymes such as alkaline phosphatase or peroxidase, radioactive isotopes such as $^{32}P$ or $^{35}S$, fluorochromes such as fluorescein or metal particles, for direct detection by colourimetry, auto-radiography, fluorometry or metallography respectively.

The term "immunoassay", as used in this description relates to any analytical technique that is based on the reaction of conjugation of an antibody with an antigen. Examples of known immunoassays in the state of the art are, for instance, but not limited to: *inmunoblot,* Enzyme-Linked ImmunoSorbent Assay (ELISA), lineal immunoassay (LIA), radioimmunoassay (RIA), immunofluorescence, x-map or *protein* chips.

The terms "polynucleotide" and "nucleic acid" are used herein exchangeably, referring to polymer forms of nucleotides of any length, both ribonucleotides (RNA) and deoxyribonucleotides (DNA).

The terms "amino acid sequence", "peptide", "oligopeptide", "polypeptide" and "protein" are used herein exchangeably, and relate to a polymer form of amino acids of any length, that can be coding or non-coding, chemically or biochemically modified.

<u>Early diagnosis biomarkers</u>

The authors of this invention have analysed the family members of cytokines in healthy individuals, in untreated individuals with DACP and in individuals suffering DACP that have been subject to treatment with gemcitabine and erlotinib. A number of markers have been found that allow the early diagnosis of individuals with DACP. Therefore, this invention provides a method for obtaining useful data for the early diagnosis of individuals with DACP.

Therefore, a first aspect of the invention relates to the use of genes (and/or proteins) *FGF-10, CXCL11, OSM, GPNMB* and SCF, for the early diagnosis of cancer of the pancreas. The use can be simultaneous or any of the genes (and/or proteins), or any the combination thereof can be selected.

In a preferred embodiment of this aspect of the invention the cancer of the pancreas is ductal adenocarcinoma of the pancreas (DACP)

Another aspect of the invention relates to a method for obtaining useful data, hereinafter the first method of the invention, for the early diagnosis of cancer of the pancreas, that comprises:

a) Obtaining an isolated sample from the individual.
b) Measuring the product of expression of genes that are selected from the list consisting of: *FGF-10, CXCL11, OSM, GPNMB* and SCF.

The product of expression of all genes can be measured simultaneously or any of the genes (and/or proteins), or any of the combinations thereof can be chosen.

In another preferred embodiment, the first method of the invention also comprises:

c) comparing the amounts obtained in step (b) with a reference amount.

In a preferred embodiment, steps (b) and/or (c) of the methods described above can be completely or partially automated, for instance, by means of a sensor robotic equipment for the detection of the amount in step (b) or the computerised comparison in step (c).

A preferred embodiment of this aspect of the invention relates to a diagnostic method for cancer of the pancreas that comprises steps (a)-(c) of the first method of the invention, and also comprises assigning the individual of step (a) to the group of individuals suffering cancer of the pancreas when they show an amount of expression products of genes *FGF-10, CXCL11, OSM, GPNMB and SCF,* higher than the reference amount. An amount of expression product of any of genes *FGF-10, CXCL11, OSM, GPNMB and SCF,* or all of them simultaneously, above the reference amount, can occur.

In a preferred embodiment the method of the invention, the isolated biological sample of an individual of step (a) is a blood sample, and even more preferably serum.

The comparison described in section (c) of the method of this invention can be performed manually or computer-assisted.

The adequate reference amounts can be measured by the method of this invention from a reference sample that can be analysed, for instance, simultaneously or consecutively, together with the test biological sample. Therefore, for instance, but not limited to, the reference sample can be the negative controls, that is, the amounts detected by the method of the invention in samples of individuals not suffering the disease.

The characteristics of the cytokines that are object of evaluation are described below:

The gene *FGF-10* or *fibroblast growth factor 10* (KGF-2: *keratinocyte growth factor* 2) is in chromosome 5 (5p13-p12). This factor has been described as a promoter of morphogenesis in primary stages of organogenesis as well as a regulator of pancreatic epithelial stem cell proliferation *(*Bhushan et al., 2001. Development 128, 5109-5117). A link has been also recently described between signalling FGF10/FGFR2-IIIb and migration and invasion of pancreatic cancer cells through induction of metalloproteinases of type 1 membrane matrix and transforming the growth factor TGF-β1 which is an important regulator of mesenchymal epithelial transition (Nomura et al., 2008. Br. J. Cancer 99, 305-313).

In this invention, *FGF-10* is also defined by a nucleotide or polynucleotide sequence, forming the coding sequence of the protein included in SEQ ID NO: 8, and that would comprise several variants from:

a) nucleic acid molecules coding a polypeptide that comprises the amino acid sequence of SEQ ID NO: 8,

b) nucleic acid molecules where the complementary hybrid chain with the polynucleotide sequence of a),

c) nucleic acid molecules where the sequence differs from a) and/or b) due to degeneration of the gene code,

d) nucleic acid molecules coding a polypeptide that comprises the amino acid sequence with an identity of at least 80%, 90%, 95%, 98% or 99% with SEQ ID NO: 8, and wherein the polypeptide coded by said nucleic acids has the activity and the structural characteristics of the *FGF-10* protein. These nucleic acid molecules include that of sequence SEQ ID NO: 1.

The gene *CXCL11* or *hemokine (C-X-C motif) ligand 11* (C-X-C motif chemokine 11; beta-R1; interferon gamma-inducible protein 9; interferon-inducible T-cell alpha chemoattractant; small inducible cytokine B11; small inducible cytokine subfamily B (Cys-X-Cys), member 11; small inducible cytokine subfamily B (Cys-X-Cys), member 9B; small-inducible cytokine B11, H174, I-TAC, PI-9, IP9, SCYB11, SCYB9B, b-R1) is in chromosome 4 (4q21.2). CXCL11 is a chemokine that interacts specifically with the receptor CXCR3. It stimulates phosphorylation of the MAPK kinase pathways leading to proliferation and prevention of apoptosis (Miekus et al., 2010. Folia Histochem. Cytobiol. 48, 104-111) In addition, its role has been described in several cancer tumorigenesis (Lo et al., 2010. Am. J. Pathol. 176, 2435-2446; Furuya et al., 2011. Gynecol. Oncol. 122, 648-655). Although we report herein, for the first time, CXCL11 as a possible biomarker in patients with DACP, it has been recently proposed as a serum biomarker for adenocarcinoma of the prostate (Klee et al., 2012. Clin. Toxicol. 58, 599-609)

In this invention, *CXCL11* is also defined by a nucleotide or polynucleotide sequence, forming the coding sequence of the protein included in SEQ ID NO: 9, and that would comprise several variants from:

a) nucleic acid molecules coding a polypeptide that comprises the amino acid sequence of SEQ ID NO: 9,

b) nucleic acid molecules where the complementary hybrid chain with the polynucleotide sequence of a),

c) nucleic acid molecules where the sequence differs from a) and/or b) due to degeneration of the gene code,

d) nucleic acid molecules coding a polypeptide that comprises the amino acid sequence with an identity of at least 80%, 90%, 95%, 98% or 99% with SEQ ID NO: 9, and wherein the polypeptide coded by said nucleic acids has the activity and the structural characteristics of the CXCL11 protein. These nucleic acid molecules include that of sequence SEQ ID NO: 2.

The gene OMS or *oncostatin M* (C-X-C motif chemokine 11; beta-R1; interferon gamma-inducible protein 9; interferon-inducible T-cell alpha chemoattractant; small inducible cytokine B11; small inducible cytokine subfamily B (Cys-X-Cys), member 11; small inducible cytokine subfamily B (Cys-X-Cys), member 9B; small-inducible cytokine B11, H174, I-TAC, PI-9, IP9, SCYB11, SCYB9B, b-R1) is contained in chromosome 4 (4q21.2). Oncostatin M belongs to the same family as interleukin-6 and stimulates several functions involved in wound repair. Although it was initially described as a leukaemia cell growth inhibitor (Zarling et al., 1986. Proc. Nat. Acad. Sci. USA. 83, 9739-43), it has been recently seen that it can have a double role, also as promoter in cell proliferation, migration and invasion (Fossey et al. 2011, BMC Cancer 11, 125; Li et al., 2011. Int. J. Mol. Med. 28, 101-108; Winder et al., 2011. J. Pathol. 225, 448-462; Tiffen et al., 2008. Mol. Endocrinol. 22, 2677-2688). Once the OMS binds its receptor, it promotes mutual phosphorylation and activation of the Janus kinase family pathway (JAK)/STAT. Several transcriptional objectives of STAT3 are important contributing factors to the biology of DACP (Corcoran et al., 2011. Cancer Res. 71, 5020-5029)

In this invention, OMS is also defined by a nucleotide or polynucleotide sequence, forming the coding sequence of the protein included in SEQ ID NO: 10, and that would comprise several variants from:

a) nucleic acid molecules coding a polypeptide that comprises the amino acid sequence of SEQ ID NO: 10,

b) nucleic acid molecules where the complementary hybrid chain with the polynucleotide sequence of a),

c) nucleic acid molecules where the sequence differs from a) and/or b) due to degeneration of the gene code,

d) nucleic acid molecules coding a polypeptide that comprises the amino acid sequence with an identity of at least 80%, 90%, 95%, 98% or 99% with SEQ ID NO: 10, and wherein the polypeptide coded by said nucleic acids has the activity and the structural characteristics of the OMS protein. These nucleic acid molecules include that of sequence SEQ ID NO: 3.

The gene *GPNMB* or *glycoprotein (transmembrane) nmb* (UNQ1725/PRO9925, HGFIN, NMB, glycoprotein NMB; glycoprotein nmb-like protein; osteoactivin; transmembrane glycoprotein HGFIN; transmembrane glycoprotein, NMB NQ1725/PRO9925, HGFIN, NMB) is contained in chromosome 7 (7p15). Glycoprotein GPNMB, also known as osteoactivin (OA), DC-HIL or HGFIN is a type 1 transmembrane protein, which was described in the beginning as low at undetectable levels in malignant cells (Weterman et al., 1995. Int. J. Cancer 60, 73-81) However, more recent studies have described GPNMB/osteoactivin as promoter of metastasis and invasion (Rose et al., 2010. PLos One 5, 12093) in some cancers such as melanoma (Tomihari et al., 2010. Cancer Res 70, 5778-5787; Tse et al., 2006. Clin. Cancer Res. 12, 1373-1382), uveal melanoma (Williams et al., 2010. Melanoma res. 20, 184-190), glioma (Kuan et al., 2006. Clin. Cancer Res. 12, 1970-1982), hepatocellular carcinoma (Onaga et al., 2003. J. Hepatol. 39, 779-785 and breast cancer, where it has been also described as a prognostic indicator of recurrence (Rose et al., 2010. Clin. Cancer Res. 16, 2147-2156). An induction of GPNMB in the bone marrow of patients with amyotrophic lateral sclerosis as inducer of motor neuron degeneration has been recently described (Tanaka et al., 2012. Sci. Rep. 2, 573).

In this invention, *GPNMB* is also defined by a nucleotide or polynucleotide sequence, forming the coding sequence of the protein included in SEQ ID NO: 11 or of SEO ID NO: 12, and that would comprise several variants from:

a) nucleic acid molecules coding a polypeptide that comprises the amino acid sequence of SEQ ID NO: 11 or of SEQ ID NO: 12,

b) nucleic acid molecules where the complementary hybrid chain with the polynucleotide sequence of a),

c) nucleic acid molecules where the sequence differs from a) and/or b) due to degeneration of the gene code,

d) nucleic acid molecules coding a polypeptide that comprises the amino acid sequence with an identity of at least 80%, 90%, 95%, 98% or 99% with SEQ ID NO: 11 or of SEQ ID NO: 12, and wherein the polypeptide coded by said nucleic acids has the activity and the structural characteristics of the GPNMB protein. These nucleic acid molecules include that of sequence SEQ ID NO: 4 or of SEQ ID NO: 5.

The gene SCF or *KIT ligand* (KITLG, FPH2, KL-1, Kitl, MGF, SCF, SF, SHEP7, c-Kit ligand; familial progressive hyperpigmentation 2; kit ligand; mast cell growth factor; steel factor; stem cell factor) is contained in chromosome 12 (12q22). SCF is the main ligand for the tyrosine kinase receptor c-kit (KIT). Its bindings support proliferation, differentiation and survival in cells expressing KIT, both in normal cells and in tumour cells, including pancreatic cancer cells (Yasuda et al., 2006. Mol. Cancer 5, 46) through activation of pathways (PI3K)/Akt; MAPK and STAT (Yasuda et al., 2007. Dig. Dis. Sci. 52, 2292-2300). Previous analyses have described the high levels of stem cell factor (SCF) in colorectal cancer sera and DACP (Mroczko et al., 2005. Clin. Toxicol. Lab. Med. 43, 146-150; Mroczko et al., 2004. Clin. Toxicol. Lab. Med. 42, 256-260; Mroczko et al., 2005. Int.J. Colorectal Dis. 22,33-38)..

In this invention, SCF is also defined by a nucleotide or polynucleotide sequence, forming the coding sequence of the protein included in SEQ ID NO: 13 or of SEO ID NO: 14, and that would comprise several variants from:

a) nucleic acid molecules coding a polypeptide that comprises the amino acid sequence of SEQ ID NO: 13 or of SEQ ID NO: 14,

b) nucleic acid molecules where the complementary hybrid chain with the polynucleotide sequence of a),

c) nucleic acid molecules where the sequence differs from a) and/or b) due to degeneration of the gene code,

d) nucleic acid molecules coding a polypeptide that comprises the amino acid sequence with an identity of at least 80%, 90%, 95%, 98% or 99% with SEQ ID NO: 13 or of SEQ ID NO: 14, and wherein the polypeptide coded by said nucleic acids has the activity and the structural characteristics of the SCF protein. These nucleic acid molecules include that of sequence SEQ ID NO: 6 or of SEQ ID NO: 7.

As DACP shows multiple genetic and epigenetic disorders and implicates several signalling pathways, a combination of CA 19-9 with several biomarkers could represent a new approach to establish new diagnostic biomarkers. *FGF-10, CXCL11, OSM, GPNMB* and *SCF* have been selected by the authors of this invention for an early diagnosis of the disease.

In another preferred embodiment, the detection of the amount of expression of any of genes *FGF-10, CXCL11, OSM, GPNMB* and *SCF* is performed by an immunoassay.

In another preferred embodiment, the immunoassay is an Enzyme-Linked ImmunoSorbent Assay (ELISA). The ELISA is based on the assumption that an immunoreagent (antigen or antibody) can be immobilised in a solid support, then placing this system in contact with a fluid phase containing the complementary reagent that can be bound to a marker compound. There are different types of ELISA: Direct ELISA, indirect ELISA, or sandwich ELISA:

Another aspect of the invention relates to an antibody to treat an individual suffering cancer of the pancreas (or alternatively to the use of an antibody for the preparation of a medicine for the treatment of cancer of the pancreas), that can be identified by a method of the invention, wherein the antibody is selected from anti-FGF-10, anti-CXCL11, anti-OSM, anti-GPNM and anti-SCF, or any of its combinations. Preferably a combination of all the above antibodies is used.

Another aspect of the invention relates to a pharmaceutical composition comprising an agent modulating at least one of the genes that are selected from *FGF-10, CXCL11, OSM; GPNMB* and SCF, to treat an individual suffering cancer of the pancreas (or alternatively to the use of a pharmaceutical composition comprising an agent modulating at least one of the genes that are selected from *FGF-10, CXCL11, OSM; GPNMB* and SCF in the preparation of a medicine for the treatment of cancer of the pancreas), that can be identified by a method of the invention. Preferably, the composition comprises one or more agents modulating all genes *FGF-10, CXCL11, OSM; GPNMB* and *SCF* simultaneously. Even more preferably, the pharmaceutical composition also comprises another active ingredient. In another preferred embodiment, the modulatory agent is an antibody that is selected from anti-FGF-10, anti- CXCL11, anti-OSM, anti- GPNM and anti-SCF, or any of its combinations.

Another aspect of this invention relates to a kit or device, hereinafter first kit of the invention, that comprises the elements necessary to measure the amount of products of expression of genes that are selected from *FGF-10, CXCL11, OSM, GPNMB* and *SCF* or any of its combinations.

In a preferred embodiment, the first kit of this invention comprises the antibodies that are selected from the list consisting of antibodies: anti-FGF-10, anti- CXCL11, anti-OSM, anti-GPNM and anti-SCF, or any of its combinations. Preferably, the kit of the invention comprises simultaneously at least one antibody of each type: anti-FGF-10, anti-CXCL11, anti-OSM, anti-GPNM and anti-SCF.

In another preferred embodiment, the first kit of the invention comprises secondary antibodies or positive and/or negative controls. The kit can also include, with no type of limitation, buffers, protein extraction solutions, agents for preventing contamination, protein degradation inhibitors, etc.

On the other hand, this kit can include all supports and containers necessary for set up and optimisation. Preferably, this kit comprises also the instructions to perform the methods of the invention.

Another aspect of the invention relates to a solid support, or protein chip, that comprises at least one of the antibodies anti-FGF-10/, anti- CXCL11, anti-OSM, anti- GPNM and anti-SCF, or any of its combinations, to perform any of the methods of the invention.

Another aspect of the invention relates to a solid support, or DNA chip, that comprises oligonucleotides or single-channel microarrays designed from a known sequence or a mRNA of at least one of genes *FGF-10, CXCL11, OSM, GPNM and SCF.* Preferably, it comprises oligonucleotides that can detect the mRNA of all genes *FGF-10,* EG-VEGF, IL-29, NRG1-beta1 and PD-ECGF.

Therefore, for instance, the oligonucleotides sequences are built in the chip surface by elongation of a chain in growth with a single nucleotide using photolithography. Therefore, oligonucleotides are anchored in edge 3' by a method of selective activation of nucleotides, protected by a photolabile reagent, by selective incidence of light through a photomask. The photomask can be physical or virtual.

Therefore, oligonucleotide probes can be from 10 to 100 nucleotides, more preferably, from 20 to 70 nucleotides, and even more preferably, from 24 to 30 nucleotides. For measuring gene expression, preferably about 40 oligonucleotides per gene are used.

Synthesis in situ on a solid support (for instance, glass) could be performed by ink-jet technology, which requires longer probes. Supports could be, amongst others, filter or membranes of NC or nylon (charged), silicon or glass slides for microscopes covered with aminosilanes, polylysine, aldehydes or epoxy. The probe is each of the chip samples. The target is the sample to be analysed. Messenger RNA, total RNA, CRP fragment, etc.

<u>Treatment response biomarkers</u>

On the other hand, the authors of this invention have analysed 507 proteins of the family of cytokines in the serum of individuals suffering DACP and that have been treated with the combination gemcitabine + erlotinib. A number of markers have been found which allow the prognosis of individuals with DACP. Therefore, this invention provides a method for obtaining useful data that prognosticate the response to treatment with gemcitabine + erlotinib of individuals with DACP, even before they are subject to said treatment.

Therefore, another aspect of the invention relates to the use of genes *CD80, EG-VEGF, IL-29, NRG1-beta1* and *PD-ECGF,* to predict or prognosticate the response of an individual to treatment with a nucleoside analogue and an epidermal growth factor receptor inhibitor, wherein the individual suffers cancer of the pancreas.

In a preferred embodiment, the nucleoside analogue is gemcitabine. In another preferred embodiment, the epidermal growth factor receptor inhibitor is erlotinib.

In another preferred embodiment, the cancer is ductal adenocarcinoma of the pancreas (DACP).

Another aspect of the invention relates to a method for obtaining useful data, hereinafter third method of the invention, to predict or prognosticate the response to treatment of cancer of the pancreas, that comprises:

a) Obtaining an isolated sample of serum from the individual.
b) measuring the product of expression of genes that are selected from the list consisting of: *CD80, EG-VEGF, IL-29, NRG1-beta1* and *PD-ECGF.*

In another preferred embodiment, this method also comprises:

c) finding a prognostic index value (PI) with the values of cytokines of step b):

$$\boldsymbol{IP_{PDAC}} = 4.351 \times \mathbf{B7\text{-}1/CD80} + 0.003 \times \mathbf{EG\text{-}VEGF\ /\ PK1} + 0.081 \times \mathbf{IL\text{-}29} + 0.020 \times \mathbf{NGR1\text{-}beta1/HRG1\text{-}beta1} + 0.264 \times \mathbf{PD\text{-}ECGF}$$

Where, the individuals with PI>17 show a poor prognosis of the disease, as survival is shorter than five months.

In another preferred embodiment, individuals with PI<17 show a better prognosis of the disease, as the survival is longer than five months.

In a preferred embodiment de this method, in the treatment the nucleoside analogue is gemcitabine. In another preferred embodiment, in the treatment the epidermal growth factor receptor inhibitor is erlotinib.
In another preferred embodiment, the cancer is ductal adenocarcinoma of the pancreas (DACP).
In another preferred embodiment of this aspect of the invention, the biological sample (a) is a blood sample.
Steps (b) and/or (c) described can be completely or partially automated, for instance, by means of a sensor robotic equipment for the detection of the amount in step (b) or the computerised comparison in step (c).
Preferably, the isolated biological sample of an individual of step (a) is a blood sample (serum).
The comparison described in section (c) of the method of this invention can be performed manually or computer-assisted.
The adequate reference amounts can be measured by the method of this invention from a reference sample that can be analysed, for instance, simultaneously or consecutively, together with the test biological sample.
The characteristics of the cytokines that are object of evaluation are described below:

CD80 (also known as B7-1): the B7 system is one of the most important secondary signalling mechanism and it is essential to maintain the delicate balance between the immune power and autoimmunity suppression. B7-1 (CD80) and B7-2 (CD86) are ligands in antigen-presenting cells and they are all responsible for c-stimulating signalling because the regulate growth, maturation and tolerance of the T cell (Seliger et al., 2012. Cancer Immunol. Immunother. 61, 1327-1341). After binding of its receptors the T cells are activated and survival is promoted (Chen et al., 1994. J. Exp. Med. 179, 523-532). On the other hand they can also deliver co-inhibitory signals in the inhibitor receptors and block the response of the T cells. An inadequate co-stimulation could contribute to the progressive growth of tumours. The combination of B7-1 and of B7-H1 has been already proposed as a prognostic factor for DACP. Although the role of B7-1 appears to be anti-tumoral, there is a new global view, where it is believed that the aberrant or imbalanced expression of members of the family B7 could contribute to escape of immune control (Wang et al., 2012. PLoS One 7, e45491; Wang et al., 2010. World J. Surg. 34, 1059-1065).

In this invention, CD80 is also defined by a nucleotide or polynucleotide sequence, forming the coding sequence of the protein included in SEQ ID NO: 24, and that would comprise several variants from:

a) nucleic acid molecules coding a polypeptide that comprises the amino acid sequence of SEQ ID NO: 24,

b) nucleic acid molecules where the complementary hybrid chain with the polynucleotide sequence of a),

c) nucleic acid molecules where the sequence differs from a) and/or b) due to degeneration of the gene code,

d) nucleic acid molecules coding a polypeptide that comprises the amino acid sequence with an identity of at least 80%, 90%, 95%, 98% or 99% with SEQ ID NO: 24, and wherein the polypeptide coded by said nucleic acids has the activity and the structural characteristics of the CD80 protein. These nucleic acid molecules include that of sequence SEQ ID NO: 15.

EG-VEGF (also known as PK1): this molecule was described in principle as an example of a type of specific mitogen to regulate proliferation and differentiation of vascular endothelium on a specific-tissue manner. Although this protein does not show any structural homology to the VEDF family, it has in common different regulating functions related to proliferation and migration (LeCouter et al., 2001. Nature 412, 877-884). It has been also described that EG-VEGF is related to cancer of the ovary (Balu et al., 2012. Rom. J. Morphol. Embryol. 53, 479-483), colorectal (Nagano et al., 2007 J. Surg. Oncol. 96, 605-610), prostate (Pasquali et al., 2006. Endocrinology 147, 4245-4251), hepatocellular (Li et al., 2006. J. Exp. Clin. Cancer Res. 25, 403-409), pancreas (Jiang et al., 2009. Pancreatology 9, 165-172) and neuroblastoma (Ngan et al., 2007. Clin. Cancer Res. 13, 868-875). As well as a factor for angiogenesis of the placenta (Brouillet, S., 2012. Trends Endocrinol. Metab. 23, 501-508).
In this invention, EG-VEGF is also defined by a nucleotide or polynucleotide sequence, forming the coding sequence of the protein included in SEQ ID NO: 25, and that would comprise several variants from:

a) nucleic acid molecules coding a polypeptide that comprises the amino acid sequence of SEQ ID NO: 25,

b) nucleic acid molecules with the complementary chain hybrid with the polynucleotide sequence of a),

c) nucleic acid molecules where the sequence differs from a) and/or b) due to degeneration of the gene code,

d) nucleic acid molecules coding a polypeptide that comprises the amino acid sequence with an identity of at least 80%, 90%, 95%, 98% or 99% with SEQ ID NO: 25, and wherein the polypeptide coded by said nucleic acids has the activity and the structural characteristics of the EG-VEGF protein. These nucleic acid molecules include that of sequence SEQ ID NO: 16.

IL-29: it is also named as IFN-λ1 and belongs to type III of the family IFN. It has been described to induce biological activities similar to those of type I of the same family. Although both can induce the anti-proliferative response in many types of cells, IFN-λ I appears to be more limited. Signalling produced by IFN-λ I triggers activation of the routes STAT1, STAT2, STAT3 and STAT5, that are the main 3 cascades of kinase proteins activated by mitogen (MAPK) and the phosphorylation of protein kinase B (Akt) through the route phosphatidylinositol-3-kinase (PI3K; Kotenko et al., 2011. Curr. Opin. Immunol. 23, 583-590; Maher et al., 2008. Cancer Biol. Ther. 7, 1109-1115; Donnelly et al., 2010. J. Interferon Cytokine Res. 30, 555-564). However, the ability of IFN-λ I to trigger these alternative routes, might be specified for altered cells or cancer cells. The conclusion results from other studies suggesting that the induction of multiple myeloma human cell growth occurs by activation of MAPK (Novak et al., 2008. Leukemia 22, 2240-2246). The specific role of IL-29 in the response of the host and the immune vigilance has not been defined yet in the context of cancer in general and in the context of DACP in particular.

In this invention, IL-29 is also defined by a nucleotide or polynucleotide sequence, forming the coding sequence of the protein included in SEQ ID NO: 26, and that would comprise several variants from:

a) nucleic acid molecules coding a polypeptide that comprises the amino acid sequence of SEQ ID NO: 26,

b) nucleic acid molecules with the complementary chain hybrid with the polynucleotide sequence of a),

c) nucleic acid molecules where the sequence differs from a) and/or b) due to degeneration of the gene code,

d) nucleic acid molecules coding a polypeptide that comprises the amino acid sequence with an identity of at least 80%, 90%, 95%, 98% or 99% with SEQ ID NO: 26, and wherein the polypeptide coded by said nucleic acids has the activity and the structural characteristics of the IL-29 protein. These nucleic acid molecules include that of sequence SEQ ID NO: 17.

NRG1-beta1: neuregulin-1 or heregulin-1 is a ligand of an extracellular protein aimed at binding to members of the family of receptors ErbB, ErbB3 and ErbB4. After the interaction with its receptors several biological events are stimulated, including the induction and progression of some epithelial cancers. Proteins NRG1/HRG1 also play an essential role in the nervous system, the heart and the breast, and are involved in the development of some diseases, including schizophrenia and cancer of the breast (Falls et al., 2003. Exp. Cell. Res. 284, 14-30; Hayes et al., 2008. J. Mammary Gland. Biol. Neoplasia 13, 205-214). Regulation of the angiogenic factor VEGF by NRG1/HRG1 (Yen et al., 2000. Oncogene 19, 3460-3469) has been also described. Its proliferative effects are probably achieved through the combined action with multiple pathways, including PI3K, MAPK and p38MAPK pathways (Stove et al., 2004. Clin. Exp. Metástasis 21, 665-684) that have been specifically described in DACP cells. DACP patients with a worse survival rate had a high expression of HRG-β mRNA. ErbB3 plays an essential role in pancreatic carcinogenesis as it promotes proliferation of cancer cells both *in vivo* and *in vitro.* It has been recently seen how fibroblasts associated with cancer develop the ligand NRG1/HRG1, activating DACP cells by signalling mediated by ErbB3/AKT and increasing carcinogenesis. This could be related to the insufficient effect of erlotinib (EGFR inhibitor) when it is combined with gemcitabine in the treatment of patients with DACP (Liles et al., 2011. Br. J. Cancer 105, 523-533).

In this invention, NRG1/HRG1 is also defined by a nucleotide or polynucleotide sequence, forming the coding sequence of the protein included in SEQ ID NO: 27, and that would comprise several variants from:

a) nucleic acid molecules coding a polypeptide that comprises the amino acid sequence of SEQ ID NO: 27,

b) nucleic acid molecules with the complementary chain hybrid with the polynucleotide sequence of a),

c) nucleic acid molecules where the sequence differs from a) and/or b) due to the degeneration of the genetic code,

d) nucleic acid molecules coding a polypeptide that comprises the amino acid sequence with an identity of at least 80%, 90%, 95%, 98% or 99% with SEQ ID NO: 27, and wherein the polypeptide coded by said nucleic acids has the activity and the structural characteristics of the NRG1/HRG1 protein. These nucleic acid molecules include that of sequence SEQ ID NO: 18.

PD-ECGF: also known as thymidine phosphorylase. Its activity and expression in carcinomas of the oesophagus, stomach, lung, pancreas and colorectal is significantly greater than in non-malignant adjacent tissues and may play a major role in the proliferation of these solid tumours. PD-ECGF is expressed both in tumour cells and in stromal cells associated to tumour. In the analyses of regression of carcinoma of the bladder, colorectal, gastric, renal and pancreas, PD-ECGF has been marked as a prognostic factor for poor results.

In this invention, PD-ECGF is also defined by a nucleotide or polynucleotide sequence, forming the coding sequence of the protein included in SEQ ID NO: 28, SEQ ID NO: 29, SEQ ID NO: 30, SEQ ID NO: 31, SEQ ID NO: 32, and that would comprise several variants from:

a) nucleic acid molecules coding a polypeptide that comprises the amino acid sequence of SEQ ID NO: 28, SEQ ID NO: 29, SEQ ID NO: 30, SEQ ID NO: 31, SEQ ID NO: 32

b) nucleic acid molecules with a complementary chain hybrid with the polynucleotide sequence of a),

c) nucleic acid molecules where the sequence differs from a) and/or b) due to the degeneration of the genetic code,

d) nucleic acid molecules coding a polypeptide that comprises the amino acid sequence with an identity of at least 80%, 90%, 95%, 98% or 99% with SEQ ID NO: 28, SEQ ID NO: 29, SEQ ID NO: 30, SEQ ID NO: 31, SEQ ID NO: 32, and wherein the polypeptide coded by said nucleic acids has the activity and the structural characteristics of the PD-ECGF protein. These nucleic acid molecules include that of sequence SEQ ID NO: 19, SEQ ID NO: 20, SEQ ID NO: 21, SEQ ID NO: 22, SEQ ID NO: 23.

In another preferred embodiment, the detection of the amount of expression of any of genes *CD80, EG-VEGF, IL-29, NRG1-beta1* and *PD-ECGF* is performed by an immunoassay.

In another preferred embodiment, the immunoassay is an Enzyme-Linked ImmunoSorbent Assay (ELISA). The ELISA is based on the assumption that an immunoreagent (antigen or antibody) can be immobilised in a solid support, then placing this system in contact with a fluid phase containing the complementary reagent that can be bound to a marker compound. There are different types of ELISA: Direct ELISA, indirect ELISA, or sandwich ELISA:

Another aspect of the invention relates to an antibody to treat an individual that suffers cancer of the pancreas, that can be identified by a method of the invention, wherein the antibody is selected from anti-CD80, anti-EG-VEGF, anti-IL-29, anti-NRG1-beta1 and anti-PD-ECGF, or any of its combinations. Preferably a combination of all the above antibodies is used.

Another aspect of the invention relates to a pharmaceutical composition that comprises a modulatory agent of at least one of the genes that are selected from *CD80, EG-VEGF, IL-29*, *NRG1-beta1* and *PD-ECGF,* to treat an individual that suffers cancer of the pancreas (or alternatively to the use of a pharmaceutical composition that comprises a modulatory agent of at least one of the genes that are selected from *CD80, EG-VEGF, IL-29, NRG1-beta1* and *PD-ECGF* in the preparation of a medicine for the treatment of cancer of the pancreas), that can be identified by a method of the invention. Preferably, the composition comprises one or more modulatory agents of all genes *CD80, EG-VEGF, IL-29, NRG1-beta1* and *PD-ECGF*, simultaneously. More preferably, the cancer of the pancreas is ductal adenocarcinoma of the pancreas. Even more preferably, the pharmaceutical composition also comprises another active ingredient. In another preferred embodiment, the modulatory agent is an antibody that is selected from anti-CD80, anti-EG-VEGF, anti-IL-29, anti-NRG1-beta1 and anti-PD-ECGF, or any of its combinations.

Another aspect of this invention relates to a kit or device, hereinafter second kit of the invention, that comprises the elements necessary to measure the amount of products of expression of genes that are selected from *CD80, EG-VEGF, IL-29, NRG1-beta1* and *PD-ECGF* or any of its combinations.

Even more preferably, the second kit of this invention comprises the antibodies that are selected from the list consisting of antibodies: anti-CD80, anti-EG-VEGF, anti-IL-29, anti-NRG1-beta1 and anti-PD-ECGF, or any of its combinations. Preferably, the second kit of the invention comprises simultaneously at least one antibody of each type: anti-CD80, anti-EG-VEGF, anti-IL29, anti-NRG1-beta1 and anti-PD-ECGF.

In another preferred embodiment, the second kit of the invention comprises secondary antibodies or positive and/or negative controls. The kit can also include, with no type of limitation, buffers, protein extraction solutions, agents for preventing contamination, protein degradation inhibitors, etc.

On the other hand, this kit can include all holders and containers necessary for set up and optimisation. Preferably, this kit comprises also the instructions to perform the methods of the invention.

In a preferred embodiment, the second kit of the invention comprises the assignment of a prognostic index value and classification of individuals, so that: if the individuals show PI>17 they have a poor prognosis of the disease, while if the individuals show PI<17 they have a better prognosis of the disease. Preferably, the individuals with PI≤17 show a survival longer than 5 months. In another preferred embodiment, the individuals with PI>17 show a survival shorter than 5 months.

Another aspect of the invention relates to a solid support, or protein chip, that comprises at least one of the antibodies anti-CD80, anti-EG-VEGF, anti-IL-29, anti-NRG1-beta1 and anti-PD-ECGF, or any of its combinations or any of its combinations, to perform any of the methods of the invention.

Another aspect of the invention relates to a solid support, or DNA chip, that comprises oligonucleotides or single-channel microarrays designed from a known sequence or a mRNA of at least one of genes *CD80, EG-VEGF, IL-29, NRG1-beta1* and *PD-ECGF.* Preferably, it comprises oligonucleotides that can detect the mRNA of all genes *CD80, EG-VEGF, IL-29*, *NRG1-beta1* and *PD-ECGF.*

Therefore, for instance, the oligonucleotides sequences are built in the chip surface by elongation of a chain in growth with a single nucleotide using photolithography. Therefore, oligonucleotides are anchored in edge 3' by a method of selective activation of nucleotides, protected by a photolabile reagent, by selective incidence of light through a photomask. The photomask can be physical or virtual.

Therefore, oligonucleotide probes can be from 10 to 100 nucleotides, more preferably, from 20 to 70 nucleotides, and even more preferably, from 24 to 30 nucleotides. For measuring gene expression, preferably about 40 oligonucleotides per gene are used.

Synthesis in situ on a solid support (for instance, glass) could be performed by ink-jet technology, which requires longer probes. Supports could be, amongst others, filters or membranes of NC or nylon (charged), silicon or glass slides for microscopes covered with aminosilanes, polylysine, aldehydes or epoxy. The probe is each of the chip samples. The target is the sample to be analysed. Messenger RNA, total RNA, CRP fragment, etc.

<u>Implementation of the methods of the invention</u>

Another aspect of the invention relates to a computer readable storage medium that comprises software instructions that can lead a computer to perform the steps of the method according to any of the methods of the invention.

Another aspect of the invention relates to a transmissible signal that comprises software instructions that lead a computer to perform steps of any of the methods of the invention.

**BRIEF DESCRIPTION OF THE FIGURES**

Figure 1 (A) shows the disease-specific Kaplan-Meier survival curves for the whole study population. The Kaplan-Meier survival curve is defined as the probability of surviving in a defined time period. Each time period is the time between two non-simultaneous terminal events. There were no censored survival data, as no information was lost on the survival time of any individual. (B-H) these graphs represent the Kaplan-Meier survival curves of each of the individual biomarkers, labelled as significant prognostic markers: (B), clinical response; (C) age; (D), BDNF; (E), *HVEM/TNFRSF14*; (F), *IL-24*; (G), *IL-29*; (H), leptin receptor; (I) *LRP-6* and (J), *ROBO4.* For dichotomisation the most significant cut-off values in terms of survival were used. The p values for the *log-rank te*st are shown for each variable.

**Figure 2.** Cox regression model. Prognostic curves observed (shown by dots with square, triangle and diamond shape) and planned (shown as solid line) for patients with DACP according to (A): univariate model or (B): multivariate odds ratio model. With the Cox regression it is intended to detect any relationship between the risk that a given event studied (death) and one or more independent variables are detected. (B) The three curves corresponding to the models generated with 3,4 and 5 cytokines are represented. As expected, the prognosis curves were adjusted to a logarithmic distribution. The coefficient of determination $R^2$ is illustrative of the goodness of fit model. As coefficient, these models provide useful predictions, the most accurate multivariate model being that of 5 cytokines, reaching 92.6%. Therefore, our PI model approaches 93% of the survival variation.

**Figure 3.** PI Kaplan-Meier survival curves. (A) shows the survival graph for PI derived from the univariate model, covering 2 cytokines. 1.5 as chosen as cut-off value to divide the cohort of patients of short survival time (<5 months) and long survival time (>5 months). (B) shows the survival graph for PI derived from the multivariate model, covering the 5 cytokines. 17 was chosen as cut-off value to divide the cohort of patients of short survival time (<5 months) or long survival time (>5 months). The p values for the log-rank tests are shown for both comparisons.

**Figure 4** The analysis of ROC curves for each of the five serum cytokines evidenced that they were differentially expressed among patients with DACP. The ROC curves summarised the precision of the cytokines in the prediction of patients with DACP. The area under the curve (ABC) is the mean sensitivity of the biomarker. A biomarker with non-predictive values would have an AUC of 0.5, while a biomarker with a perfect capacity to predict the disease, would have an AUC of 1. **A** shows the AUC values, cut-off value, sensitivity and specificity for FGF-10; **B** shows the AUC values, cut-off value, sensitivity and specificity for I-CXCL11; **C** shows the AUC values, cut-off value, sensitivity and specificity for OSM; **D** shows the AUC values, cut-off value, sensitivity and specificity for GPNMB; **E** shows the AUC values, cut-off value, sensitivity and specificity for SCF; **F** shows the AUC values, cut-off value, sensitivity and specificity for five combined cytokines. The cut-off values (intensity signal values) selected were those with both the highest sensitivity and specificity.

## EXAMPLES OF THE INVENTION

The following examples and drawings are provided by way of illustration, and it is not intended that they limit this invention.

All the data and statistical analysis was performed using the software IBM SPSS statistic 20 or with statistical language R. The quality of the analysis was increased using the package "ArrayQualityMetrics" in R to remove any possible atypical value.

Early diagnosis biomarkers

## MATERIALS AND METHODS

Thirty-nine patients participated in the study. Three different groups were established:

1) Twelve healthy patients, as control group.

2) Fourteen patients with DACP that had received no treatment (called pre-treated).

3) Thirteen patients with DACP under 2 weeks of treatment with the combination therapy gemcitabine + erlotinib (called post-treated)

All the patients were diagnosed with DACP at the Hospital Virgen de las Nieves (Granada, Spain) between 2008 and 2011. All patient information, including gender, age, degree of disease, and symptoms, was recorded. The mean age of the patients was 66 years (range 41-79 years) and male-female ratio 50:50. The clinical classification of the patients with adenocarcinoma of the pancreas was: state III (28%) and state IV (72%; Table 1).

**Table 1.** Clinical-pathological characteristics of the study population (n=14). Information gathered from all DACP patients participating in the study.

| ***Table 1. Clinical-pathological characteristics of the study population (n=14)*** | |
|---|---|
| ***Age at diagnosis, years (mean ± SD)*** | 66 ± 10.5 |
| ***Gender*** | M: 50%<br>F: 50% |
| ***Disease stage*** | III (28%)<br>IV (72%) |
| ***Type of chemotherapy*** | Gemcitabine + Erlotinib |
| ***Clinical response*** | PR (14.29%)<br>SD (21.43%)<br>PD (64.29%) |
| ***Survival time, months (*****mean** ***± SD)*** | 12.6 ± 12,6 |
| ***CEA levels [μg/l] (mean ± SD)*** | 2219 ± 5017<br>*Healthy: 0-37* |
| ***CA level 19-9 [U/l] (mean ± SD)*** | 899 ± 3185<br>*Healthy: 0-5* |

(continued)

| *Table 1. Clinical-pathological characteristics of the study population (n=14)* |
|---|
| *PR: partial response; SD: stable disease; PD: progressive disease; SD: standard deviation.* |

**Source of the samples.**

The blood samples were collected after obtaining approval from the relevant ethics committees and consents signed by the patients. A total of 39 serum samples were collected from 2009 to 2011, using the standard procedures of the Oncology Department of the Hospital Virgen de las Nieves (Granada, Spain). The blood samples were obtained from patients diagnosed with DACP at the start of the study and 2 weeks after the start of therapy (gemcitabine + erlotinib) and also in healthy individuals. The serum was obtained after blood centrifugation at 1500 rpm for 10 minutes at 4°C. Aliquots of the samples were taken and stored at -80°C.

**Cytokine antibody assay**

The proteins soluble in serum from patients with DACP were measured using a biotin label-based antibody array (*Human Antibody L-series 507 Array (RayBiotech, Norcross, GA, USA*), according to the recommended protocols. In short, the samples were biotinised. The antibodies were immobilised in given sites in glass slide. Incubation of the arrays with biological samples consisted of cytokines binding to their relevant antibodies. The signals are viewed using conjugated streptavidin-HRP and colourimetry. The final intensities were measured as the original intensities except for the background. The data were normalised to the positive control for each individual result

**Statistical analysis**

As the cytokines in all groups did not show a normal distribution, the Mann-Whitney test was used to highlight the differences between groups ($p<0.05$). In addition the fold change (FC) was calculated. The fold change values of cytokines were tested to indicate the relative expression values. Neither FC ≤ 1.5 nor FC ≥ 1.5 in the intensity signal between the groups were considered relevant. The area under the curve (AUC) of the receptor operative characteristic (ROC) of each marker was calculated to evaluate its diagnostic significance representing the rate of true positive values (sensitivity) vs the rate of false positive values (1-specificity). In addition, a marker combination index was generated and the ROC curve was also drawn for this combination. The cut-off values were selected to improve both sensitivity and specificity. In addition, box plots were represented that show the modulation of the expression of common cytokines modified significantly between post-treated vs pre-treated and between pre-treated and control.

**RESULTS**

**Analysis of serum diagnosis biomarkers in patients with DACP and healthy (control).**

For the purpose of finding markers helping to the early detection of DACP, an extensive screening was performed, with an antibody array for 507 human cytokines to identify those cytokines expressed differentially in health individuals and those suffering DACP. The clinical-pathological characteristics of the patients with DACP are summarised in Table 1. As shown in Table 2, there are five over-expressed cytokines in patients with DACP not treated when compared to healthy volunteers ($p<0.05$). The cytokines FGF-10, CXCL11, OSM, GPNMB and SCF could represent an altered serum profile in patients with DACP.

**Table** 2. The 5 cytokines significantly over-expressed. The differences were obtained by the Mann-Whitney test ($p<0.05$). The relative expression levels are given by the corresponding FC. Each FC≥1.5 or ≤1/1.5 in the intensity of signal between groups was considered relevant.

| ***Table 2**: Cytokines significantly over-expressed in patients with DACP.* | | | |
|---|---|---|---|
| ***ID*** | ***P-Value*** | ***Log FC*** | ***FC*** |
| ***FGF-10*** | 0.040 | 1.10 | 2.15 |
| ***CXCL11*** | 0.046 | 0.93 | 1.91 |
| ***OSM*** | 0.040 | 0.96 | 1.95 |

(continued)

| ***Table 2*: *Cytokines significantly over-expressed in patients with DACP.*** | | | |
|---|---|---|---|
| ***ID*** | ***P-Value*** | ***Log FC*** | ***FC*** |
| ***GPNMB*** | 0.019 | 1.36 | 2.56 |
| ***SCF*** | 0.022 | 1.51 | 2.85 |

**Analysis of sensitivity and specificity of serum biomarkers for early diagnosis of DACP.**

To establish whether these 5 cytokines could be used as markers for early detection of DACP, the diagnostic value (the ability to distinguish between health and disease) of each biomarker, alone and in combination, was evaluated. The sensitivity and specificity for each biomarker were analysed using the method of the area under the curve of the operative characteristics of the receptor (ROC). The curves are based on cytokine levels in healthy individuals and individuals before and after treatment. Each candidate biomarker was investigated independently (Figure 4A-E). All possible new markers had AUC values from 0.74 to 0.78 to distinguish healthy from DACP patients. The areas under the curve ROC for cytokines FGF-10, CXCL11, OSM, GPNMB and SCF were 0.744, 0.737, 0.744, 0.776 and 0.772 respectively. Osteoactivin stood out for developing the highest sensitivity for prediction of DACP. Then, to establish if these 5 cytokines overall could be used as group of biomarkers for the detection of DACP, a combination of these 5 markers was analysed to obtain an integrated ROC curve. The combined group of these cytokines improved significantly the ability of all biomarkers separately alone to distinguish patients with cancer of the pancreas of healthy controls, the AUC value improved to 0.841 (Figure 4F).

To establish the sensitivity and specificity of each biomarker, the cut-off points providing the best waived points between the higher sensitivity and the higher specificity (Figure 4). All had a specificity of 75% and a sensitivity with a range within 69-77%. The group of the 5 cytokines evidence the best yield to detect DACP in serum samples, also with a sensitivity of 77% but a specificity of 83%, higher than separately.

<u>Treatment response biomarkers</u>

All study patients were diagnosed with DACP at the Hospital Virgen de las Nieves (Granada, Spain) between 2008 and 2011. All patient information was recorded: gender, age, degree of disease, and symptoms. The mean age of the patients was 66 years (range 41-79 years) and male-female ratio (50:50). The clinical classification of the patients with adenocarcinoma of the pancreas was: state III (28%) and state IV (72%; Table 3). The overall survival time of the patients with DACP was 12.6 months and were treated with combination therapy with + erlotinib. The blood samples were collected once approval was obtained from the relevant ethics committees and consent from the donors. The serum samples were collected between 2009 and 2011, using standard procedures at the oncology department of the Hospital Virgen de las Nieves. In addition, the blood samples were obtained from patients diagnosed with DACP at the baseline conditions in patients after 2 weeks after the start of therapy (gemcitabine + erlotinib) and also in healthy individuals. The serum was obtained after blood centrifugation at 1500 rpm for 10 minutes at 4°C. The samples were aliquoted and stored at a temperature of-80°C.

**Table** 3. Clinical-pathological characteristics of the study population (n=14) Information compiled from all DACP patients participating in the study

| *Clinical-pathological characteristics of the study population (n=14)* | |
|---|---|
| *Age at diagnosis, years (mean ± SD)* | 66 ± 10.5 |
| *Gender* | M: 50%<br>F: 50% |
| *Disease stage* | III (28%)<br>IV (72%) |
| *Type of chemotherapy* | Gemcitabine + Erlotinib |
| *Clinical response* | PR (14.29%)<br>SD (21.43%)<br>PD (64.29%) |

(continued)

| *Clinical-pathological characteristics of the study population (n=14)* | |
|---|---|
| *Survival time, months (mean ± SD)* | 12.6 ± 12.6 |
| *CEA levels [μg/l] (mean ± SD)* | 2219 ± 5017<br>*Healthy: 0-37* |
| *CA level 19-9 [U/l] (mean ± SD)* | 899 ± 3185<br>*Healthy: 0-5* |
| *PR: partial response; SD: stable disease; PD: progressive disease; SD: standard deviation.* | |

**Cytokine antibody assay**

A human biotin-based antibody array (Human Antibody L-series 507 array (RayBiotech, Norcross, GA, USA)) was used, and according to the protocols recommended to measure proteins soluble in sera of patients with DACP. All samples were biotinised. The antibodies were immobilised at specific sites of the glass slide. The array membranes were incubated with the biological samples to give rise to binding of cytokines with their corresponding antibodies. The signals were viewed using conjugated streptavidin-HRP and colorimetry. The intensities of the final points were calculated as the original intensity, subtracting from the bottom. The data were normalised to the positive controls in the individual device.

**Statistical analysis**

The mean survival after administration of gemcitabine and erlotinib was calculated in months from the start of treatment to death. For an analysis of overall survival (S), the Kaplan-Meier was used a method to estimate the probability of survival in a given time, using the percentage of patients surviving that time. The log rank test *was used* to establish the survival differences between groups. The Kaplan-Meier survival curves for individual markers were obtained after dichotomisation. The cut-off values for each marker were those developing the most significant survival between the two groups. To establish the most significant variables contributing to OS, univariate and multivariate analyses were performed with the Cox regression model of odds ratio to establish associations between serum cytokines and cancer-related mortality. First, the association between mortality and cytokine levels was analysed, considered one factor every time. Then the Cox odds ratio multivariate model was applied, using a stepwise conditional algorithm, based on the probability rate.

The global model adjustment was considered to be significant according to the Chi-squared test. ($p<0.05$). In addition, the Wald index was shown to measure the weight of each variable in the global model, both in univariate and multivariate. The levels of 507 cytokines were entered in the models, as continuous parameters, and the results were expressed as a hazard ratio or relative risk ratio. By the analysis of this variables, the prognostic index (PI) was obtained, which considers the regression coefficients derived from the Cox model of all significant factors. The differences were considered significant when $p<0.05$.

The Cox regression model for the study of DACP patient survival was defined by the following equation:

$$Ecuación\ 1:\ h(t) = h_0(t)e^{\sum_{j=1}^{N}\beta_{ij}x_{ij}}$$

Where h (t) is the hazard ratio at time t, $h_o$ (t) is the baseline hazard ratio and the exponent, which does not depend on time, is known as prognostic index (PI). The prognostic index for a specific patient is defined as:

$$Equation\ 2:\ PI_i = \sum_{j=1}^{N}\beta_{ij}x_{ij}$$

Where $\beta_{ij}$ defines the coefficient planned for the Cox regression model for a particular patient *i* and cytokine *j*; $x_{ij}$ determines the expression level measured for the patient *i* and cytokine *j*; and N represents the number of cytokines included in the model.

## RESULTS

### **Analysis** of **survival of patients with DACP**

The clinical characteristics of the DACP patients are summarised in Table 1. For the whole of the study population, the OS rates were 46.15% at 6 months, 23.08% at 12 months and 7.69% at 24 months. The mean duration of the follow-up for the study group was 12 months (range 1-40 months) and during this time 100% of the patients with DACP died due to the disease. The probabilities of survival were calculated using the Kaplan-Meier method. The survival curve for the entire patient cohort is shown in Figure 1A.

### Univariate analysis between serum cytokines and survival

First, a univariate approach was used in the study to identify the measurable diagnostic factors that were relevant and independent and that could be associated with a high risk of death for DACP. Serum levels of cytokines and the clinical-pathological parameters such as age, gender, state and clinical response were analysed. The clinical-pathological parameters, age and clinical response (progressive or non-progressive disease) had a low weight in the prognosis according to the univariate analysis ($p$=0.030 and $p$=0.013, respectively). With regard to cytokines, the univariate analysis of the expression levels of *BDNF* ($p$=0.034, HR 1.005, 95% CI (1.000-1.009)); *HVEM/TNFRSF14* ($p$=0.039, HR 0,924, 95% CI (0.858-0,996)); *IL-24* ($p$=0.023 HR 1.041, 95% CI (1.006-1.078)); *IL-29* ($p$=0.021, HR 1.012, 95% CI (1.002-1.023)); *Leptin R* ($p$=0.018, HR 1.008, 95% CI (1.001-1.015)); *LRP-6* ($p$=0.022 HR 1.027, 95% CI (1.004-1.051)) and *ROBO4* ($p$=0.045 HR 1.002, 95% CI (1.000-1.004)) had a significant influence in the prognosis. The results of the univariate analysis of each cytokine, and of the prognostic factors independently, and its beta-coefficients, hazard ratio (HR) represents the factor for which the risk changes for each unit that increases the expression of cytokines), 95% CI (upper and lower limits of the confidence interval with a significant level of 0.05) and p values, are shown in Table 4.

**Table 4.** Prognostic factors according to the univariate analysis Variables significantly associated with survival of patients with DACP in the univariate analysis. The following are shown: The betaβ)-coefficients, hazard ratio, 95% CI and $p$ values for the variables selected.

| ***Table 4*: *Prognostic factors according to the univariate analysis*** | | | | | |
|---|---|---|---|---|---|
| ***Variable*** | ***Overall survival*** | | | | |
| | *β* | ***HR*** | ***95% CI*** | | ***p-value*** |
| ***BDNF*** | 0.005 | 1.005 | 1.000 | 1.009 | 0.034 |
| ***HVEM / TNFRSF14*** | -0.079 | 0.924 | 0.858 | 0.996 | 0.038 |
| ***IL-24*** | 0.040 | 1.041 | 1.006 | 1.078 | 0.023 |
| ***IL-29*** | 0.012 | 1.012 | 1.002 | 1.023 | 0.021 |
| ***Leptin R*** | 0.008 | 1.008 | 1.001 | 1.015 | 0.018 |
| ***LRP-6*** | 0.027 | 1.027 | 1.004 | 1.051 | 0.022 |
| ***ROBO4*** | 0.002 | 1.002 | 1.000 | 1.004 | 0.045 |
| ***Age.*** | 0.086 | 1.089 | 1.008 | 1.177 | 0.030 |
| ***Clinical response*** | 2.064 | 8.706 | 1.057 | 71.692 | 0.013 |
| ***Cytokines*** | ***Fit of the general model (p= 0.0023)*** | | | | |
| | β | ***HR*** | ***95% CI*** | | ***p-value*** |
| ***Il-24 (1)*** | 0.042 | 1.042 | 1.003 | 1.023 | 0.026 |
| ***Il-29 (2)*** | 0.014 | 1.014 | 1.005 | 1.081 | 0.017 |
| *β: coefficient according to the Cox regression model for a given patient and cytokine; HR: Random ratio (represents the factor whereby the hazard ratio changes for each unit of increase of cytokine expression); 95% CI: upper and lower limit of the confidence interval with a significance level of 0.05.* | | | | | |

The positive beta-coefficients for an explanatory variable represent a high risk and, therefore, the prognosis is worse. On the contrary, a negative regression coefficient involves a better prognosis for patients with higher values of this variable. The hazard ratio is a descriptive measure used to compare the survival times of the two different groups of patients. The hazard ratio indicates the change in the risk of death if the variable increases by one unit (an unit of expression for cytokines, one year for the age variable and disease progression in clinical response). 95% CI is the confidence interval for the hazard ratio. The p values, in the top of the table, show the significance of each individual variable explaining the survival. The model is shown stepwise in the bottom of the table, using only significant cytokines according to the univariate analysis. The p values indicated show the significance of cytokines in the complete model.

For the purpose of establishing the survival differences of these markers individually in patients with DACP, Kaplan-Meier survival curves were generated using the cut-off points, providing the most significant discrimination in terms of survival between groups. Figure 1 (B-J) shows the Kaplan-Meier survival group of individual markers that show significant differences in the prognosis.

Multivariate analysis between serum cytokines and survival

Although statistically significant variables of the univariate analysis are often included in the multivariate analysis, some variables that are not significant in the univariate analysis could appear as significant in the multivariate analysis. Furthermore, in addition to the statistically significant variables related to the prognosis in the univariate analysis, those without impact were also included. The best combination of the cytokines selected according to the Multivariate Cox hazard ratio is shown in Table 5.

**Table 5.** Prognostic factors according to the multivariate analysis: The cytokines significantly associated with survival of patients with DACP in the multivariate analysis: The following are shown: coefficients-beta (β), hazard ratio (HR). 95% CI and p values for cytokines selected. The last column of *p* values represents the significance of cytokines in the complete model.

| ***Table 5*: *Prognostic factors according to the multivariate analysis*** | | | | | | |
|---|---|---|---|---|---|---|
| ***Cytokines*** | ***Overall survival*** | | | | | ***Adjustment of general model*** |
| | *β* | ***HR*** | ***95% CI*** | | ***p-value*** | ***p-value*** |
| ***IL-29*** | 0.081 | 1.084 | 1.010 | 1.164 | 0.026 | 0.004212 |
| ***B7-1/CD80*** | 4.351 | 77.574 | 1.138 | 5289.453 | 0.043 | 0.002494 |
| ***PD-ECGF*** | 0.264 | 1.302 | 0.944 | 1.797 | 0.108 | 0.001350 |
| ***EG-VEGF/PK1*** | 0.003 | 1.003 | 1.000 | 1.005 | 0.049 | 0.000134 |
| ***NRG1-beta1 / HRG1-beta1*** | 0.020 | 1.020 | 0.994 | 1.047 | 0.129 | 0.000286 |
| *β: coefficient according to the Cox regression model for a given patient and cytokine; HR: Random ratio (represents the factor whereby the risk changes for each unit of increase of cytokine expression); 95% CI: upper and lower limit of the confidence interval with a significance level of 0.05.* | | | | | | |

None of the clinical-pathological parameters demonstrated a significant trend to the reduction of overall survival ($p$>0.05) and were not considered in the global model. Referring to cytokines and according to the multivariate analysis, the expression levels of *CD80* ($p$=0.043, HR 77,574, 95% CI (1.138-5289.4)); *EG-VEGF* ($p$=0.049, HR 1.003, 95% CI (1.000-1.005)) and *IL-29* ($p$=0.026, HR 1.084, 95% CI (1.010-1.164)), had a significant influence in the prognosis. The significant influence of *IL-29* in survival was seen in the univariate analysis and was confirmed with the multivariate analysis. The beta(β)-coefficients, hazard ratio (HR), 95% CI and p values for cytokines selected are shown in Table 3. Although *NRG1-beta1* (($p$=0.129), HR 1.020, 95% CI 0.994-1.797)) did not show a significant influence in the prognosis as independent factor.

**Prognosis indices of cytokines in patients with DACP:**

An adequate combination of biomarkers could provide us with the most accurate information on the prognosis; therefore, the most accurate set of variables was searched for using the conditional regression protocol based on the odds ratio.

To illustrate the inter-related effect in OS of the 7 markers remarked by the univariate analysis, the Cox odds ratio was used for selecting variables related jointly with survival. As a result of this analysis, a model resulted that contained only IL-24 ($p$=0.026, HR 1.042, 95% CI, (1.003-1.023)) and IL-29 (p=0.017, HR 1,014, 95% CI (1.005-1.081)). The global adjustment fit was shown to be significant for the statistical Chi-squared test (p=0.0023). For the purpose of establishing a prognostic index to establish overall survival of the patients with DACP, these beta-coefficients of cytokines were entered in the following equation and the following prognostic index model was generated:

$$\boldsymbol{PI}_{\textbf{univariate}} = 0.042 \times \textbf{IL-24} + 0.014 \times \textbf{IL-29}$$

The univariate PI represents the multivariate model derived from the combination of significant markers in the univariate analysis. With regard to the cytokines obtained by multivariate analysis, a second statistically significant survival model (*p*=0.0003) was built, and the following PI model was generated:

$$\boldsymbol{PI}_{\textbf{multivariate}} = 4.351 \times \textbf{B7-1/CD80} + 0.003 \times \textbf{EG-VEGF / PK1} + 0.081 \times \textbf{IL-29} + 0.020 \times \textbf{NGR1-beta1/HRG1-beta1} + 0.264 \times \textbf{PD-ECGF}$$

The multivariate PI represents the multivariate model derived from the best of all possible combinations using the 507 cytokines of the multivariate analysis.

It was evaluated by regression analysis if the PI could generate an accurate survival model for this cohort of patients. The $R^2$ was used as evidence of goodness of fit. The results of the univariate PI and multivariate PI proposed were calculated, classified and related to OS. As expected, both survival models evidenced a logarithmic trend when represented vs time. Figure 2 represents the results of the PI observed and the planned log-adjustments for these models. According to the multivariate model, the global model with 5 cytokines was statistically significant, though the regression analyses were also evaluated for the models containing 3 and 4 cytokines. The $R^2$ values obtained were 0.664, 0.727, 0.906 and 0.926 for univariate PI model and for the multivariate PI models with 3, 4 and 5, respectively. All models gave satisfactory results, but the multivariate model covering *CD80, EG-VEGF, IL-29, NRG1-beta1* and *PD-ECGF,* stood out from the rest.

The prognosis index for the multivariate model with these 5 cytokines ranged from 0 to 40 in our cohort. The patients were categorised in two groups, according to their prognosis indices: unfavourable prognosis (PI>17) and favourable prognosis (PI<17). The survival curves were then compared between these two prognostic groups (Figure 3 A). The proposed groups that are shown to distinguish the overall survival time are: low (<5 months) and high (>5 months). Overall survival in these groups was statistically very significant (*p*<0.00056). The prognostic index for univariate model was also represented, and its range was from 0 to 5. According to this PI, the patients were categorised again in 2 groups: unfavourable prognosis (PI>1.5) and favourable prognosis (PI<1.5). In addition, the survival curves were compared between these 2 prognostic groups (Figure 3 B), and a significant correlation was obtained with the overall survival, as low survival (< 5 months) and high survival (> 5 months). The overall survival in these groups was less, but still significant (*p* < 0.004) compared to the multivariate PI.

**Clauses**

In summary, the aspects of this invention are described in the following numbered clauses:

1.- The simultaneous use of genes: *CD80, EG-VEGF, IL-29, NRG1-beta1* and *PD-ECGF,* to predict or prognosticate the response of an individual to the treatment comprising a nucleoside analogue and a epidermal growth factor receptor inhibitor, wherein the individual suffers cancer of the pancreas.

2.- The use according to the above clause, wherein the individual suffers ductal adenocarcinoma of the pancreas (DACP).

3.- The use, according to any of the above clauses, wherein the treatment comprises the nucleoside analogue gemcitabine and the growth factor receptor inhibitor erlotinib.

4.- A method for obtaining useful data, to predict or prognosticate the response of an individual to the treatment that comprises a nucleoside analogue and an epidermal growth factor receptor inhibitor, wherein the individual suffers cancer of the pancreas, that comprises:

a) obtaining an isolated biological sample from the individual.

b) measuring the amount of product of expression of genes: *CD80, EG-VEGF, IL-29, NRG1-beta1* and *PD-*

*ECGF.*

5.- The method according to the above clause, that also comprises:

c) finding a prognostic index (PI) value with the cytokine values of step b) applying the formula:

$$IP_{PDAC} = 4.351 \times \textbf{B7-1/CD80} + 0.003 \times \textbf{EG-VEGF / PK1} + 0.081 \times \textbf{IL-29} + 0.020 \times \textbf{NGR1-beta1/HRG1-beta1} + 0.264 \times \textbf{PD-ECGFc})$$

6.- A method to predict or prognosticate the response of an individual to the treatment comprising a nucleoside analogue and an epidermal growth factor receptor inhibitor, wherein the individual suffers cancer of the pancreas, comprising steps (a) - (c) according to any of clauses 4 - 5, and also comprises classifying individuals with PI>17 in the group of individuals with a poor prognosis of the disease and preferably a survival shorter than 5 months.

7.- A method to predict or prognosticate the response of an individual the treatment comprising a nucleoside analogue and an epidermal growth factor receptor inhibitor, wherein the individual suffers cancer of the pancreas, comprising the steps (a) - (c) according to any of clauses 4 - 5, and also comprises classifying individuals with PI≤17 in the group of individuals with a better prognosis of the disease, and more preferably a survival over 5 months.

8.- The method according to any of clauses 4-7, wherein the nucleoside analogue is gemcitabine.

9.- The method according to any of clauses 4-8, wherein the epidermal growth factor receptor inhibitor is erlotinib.

10.- The method according to any of clauses 4-9, wherein the individual suffers ductal adenocarcinoma of the pancreas (DACP).

11.- The method according to any of clauses 4-10, wherein the biological sample is a blood sample (serum).

12.- The method according to any of clauses 4-11, where steps (b) and/or (c) the methods described above can be completely or partially automated.

13.- The method according to any of clauses 4-12, wherein the measurement is performed by immunoassay.

14.- The method according to any of clauses 4-13, wherein the immunoassay is an ELISA.

15.- The use of antibody in the preparation of a medicine to treat an individual that suffers cancer of the pancreas, that can be identified a method according to any of clauses 4-14, wherein the antibody is selected from anti-CD80, anti-EG-VEGF, anti-IL29, anti-NRG1-beta1 and anti-PD-ECGF, or any of its combinations.

16.- A pharmaceutical composition that comprises a modulatory agent of at least one of genes that are selected from *CD80, EG-VEGF, IL-29, NRG1-beta1* and *PD-ECGF,* to treat an individual that suffers cancer of the pancreas, that can be identified by the method according to any of clauses 4-14.

17.- A kit or device, that comprises the elements necessary to measure the amount of expression of genes that are selected from *CD80, EG-VEGF, IL-29, NRG1-beta1* and *PD-ECGF*.

18.- The kit or device, according to the above clause, that comprises the antibodies anti-CD80, anti-EG-VEGF, anti-IL29, anti-NRG1-beta1 and anti-PD-ECGF.

19.- The kit or device according to clauses 17-18, that also comprises the assignment of a prognostic index value and classification of individuals, according to clauses 5-6.

20.- A solid support, or protein chip, that comprises at least one of the antibodies anti-CD80, anti-EG-VEGF, anti-IL29, anti-NRG1-beta1 and anti-PD-ECGF, or any of its combinations, to perform a method according to any of clauses 4-14.

21.- A solid support, or DNA chip, that comprises oligonucleotides or single-channel microarrays designed from a known sequence or a mRNA of at least one of genes *CD80, EG-VEGF, IL-29, NRG1-beta1* and *PD-ECGF,* or any of its combinations, to perform a method according to any of clauses 4-14.

22.- A computer readable storage medium that comprises software instructions that can lead a computer to perform the steps of the method according to any of clauses 4-14.

23.- A transmissible signal that comprises software instructions that can lead a computer to perform steps of the method according to any of clauses 4-14.

## Claims

1. Simultaneous use of genes *FGF-10, CXCL11, OSM, GPNMB* and SCF, for the diagnosis of cancer of the pancreas.

2. The use, according to the above claim, wherein cancer of the pancreas is ductal adenocarcinoma of the pancreas (DACP).

3. A method for obtaining useful date for the diagnosis of cancer of the pancreas, that comprises:

    a) obtaining an isolated sample from the individual.
    b) measuring the amount of expression product of genes *FGF-10, CXCL11, OSM, GPNMB and SCF.*

4. The method according to the above claim, that also comprises:

    c) comparing the amounts obtained in step (b) with a reference amount.

5. The method according to any of claims 3-4, where steps (b) and/or (c) can be completely or partially automated.

6. A diagnostic method for cancer of the pancreas that comprises steps (a)-(c) according to any of claims 3-5, and also comprises assigning the individual of step (a) to the group of individuals suffering cancer of the pancreas when they show an amount of expression products of genes *FGF-10, CXCL11, OSM, GPNMB and SCF,* higher than the reference amount.

7. The method according to any of claims 3-6, where cancer of the pancreas is ductal adenocarcinoma of the pancreas (DACP).

8. The method according to any of claims 3-7, wherein the biological sample is a blood sample, and preferably serum.

9. The method according to any of claims 3-8, wherein the measurement is performed by immunoassay.

10. The use of an antibody in the preparation of a medicine to treat an individual that suffers cancer of the pancreas, identifiable by a method according to any of claims 3-9, wherein the antibody is selected from anti-FGF-10, anti-CXCL11, anti-OSM, anti- GPNM and anti-SCF.

11. A pharmaceutical composition that comprises a modulatory agent of at least one of genes that are selected from *FGF-10, CXCL11, OSM; GPNMB* and *SCF*, in the preparation of a medicine for the treatment of an individual that suffers cancer of the pancreas, identifiable by a method according to any of claims 3-9.

12. A kit or device, that comprises the elements necessary to measure the amount of expression of genes that are selected from *FGF-10, CXCL11, OSM, GPNMB* and *SCF*.

13. The kit or device, according to claim 12, that comprises the antibodies anti-FGF-10, anti-CXCL11, anti-OSM, anti-GPNM and anti-SCF.

14. A solid support, or protein chip, that comprises at least one of the antibodies anti-FGF-10/, anti- CXCL11, anti-OSM, anti- GPNM and anti-SCF, or any of its combinations, to perform a method according to any of claims 3-9.

15. A solid support, or DNA chip, that comprises oligonucleotides or single-channel microarrays designed from a known sequence or a mRNA of at least one of genes *FGF-10, CXCL11, OSM, GPNM and SCF,* or any of its combinations, to perform a method according to any of claims 3-9.

Fig. 1

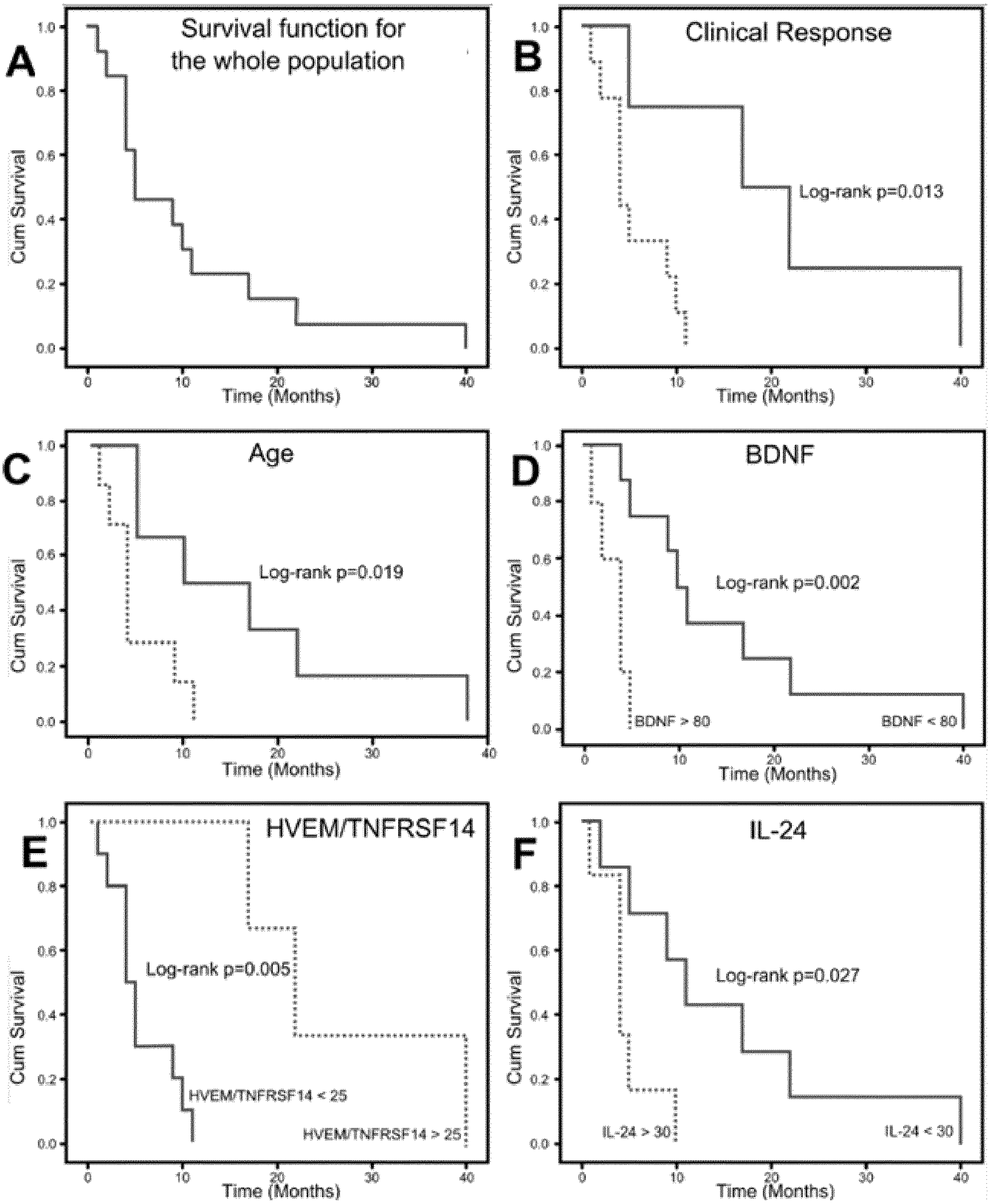
A
Survival function for
the whole population
Cum Survival
Time (Months)
B
Clinical Response
Log-rank p=0.013
Cum Survival
Time (Months)
C
Age
Log-rank p=0.019
Cum Survival
Time (Months)
D
BDNF
Log-rank p=0.002
BDNF > 80
BDNF < 80
Cum Survival
Time (Months)
E
HVEM/TNFRSF14
Log-rank p=0.005
HVEM/TNFRSF14 < 25
HVEM/TNFRSF14 > 25
Cum Survival
Time (Months)
F
IL-24
Log-rank p=0.027
IL-24 > 30
IL-24 < 30
Cum Survival
Time (Months)

**Fig. 1 cont.**

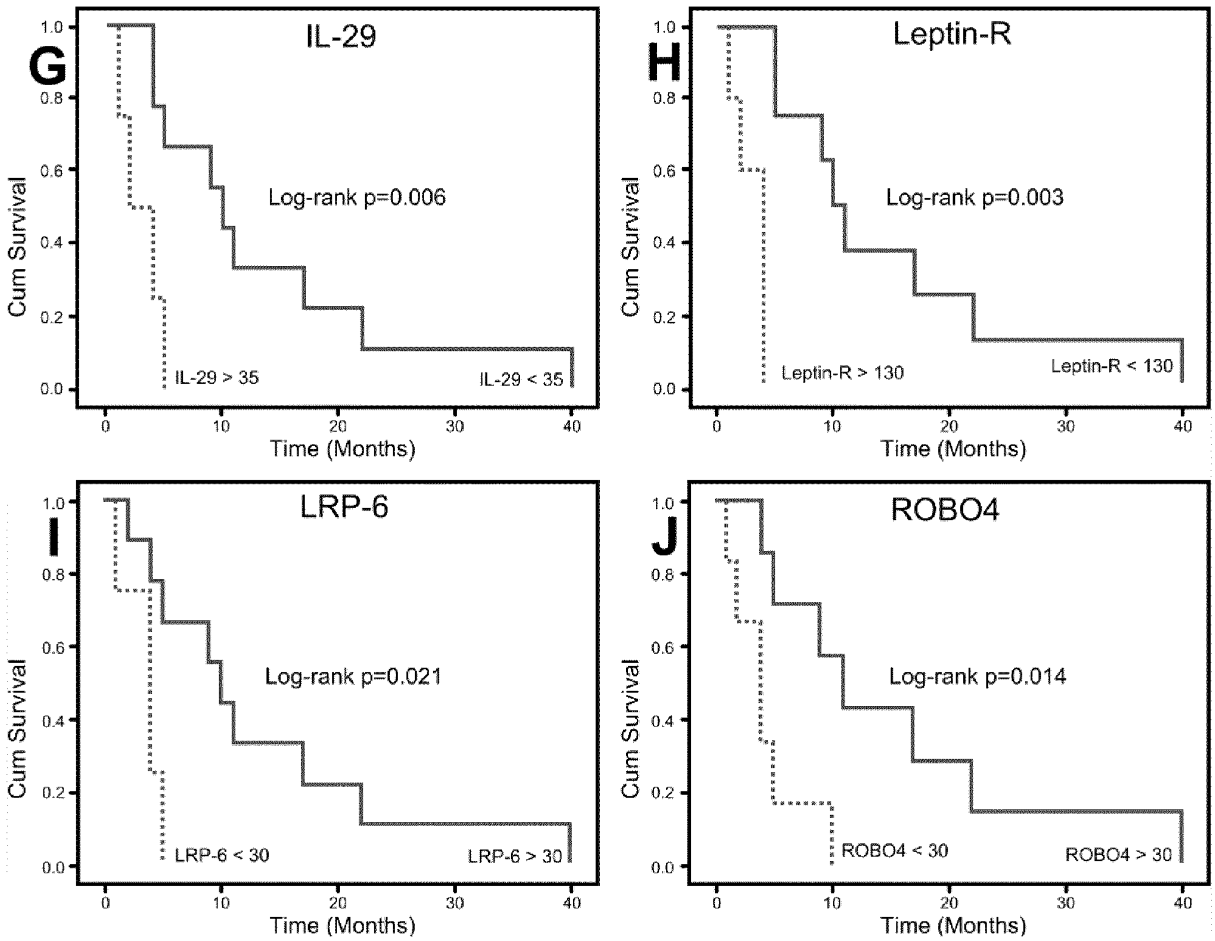
G
IL-29
Log-rank p=0.006
IL-29 > 35
IL-29 < 35
Cum Survival
Time (Months)
H
Leptin-R
Log-rank p=0.003
Leptin-R > 130
Leptin-R < 130
I
LRP-6
Log-rank p=0.021
LRP-6 < 30
LRP-6 > 30
J
ROBO4
Log-rank p=0.014
ROBO4 < 30
ROBO4 > 30

Fig. 2

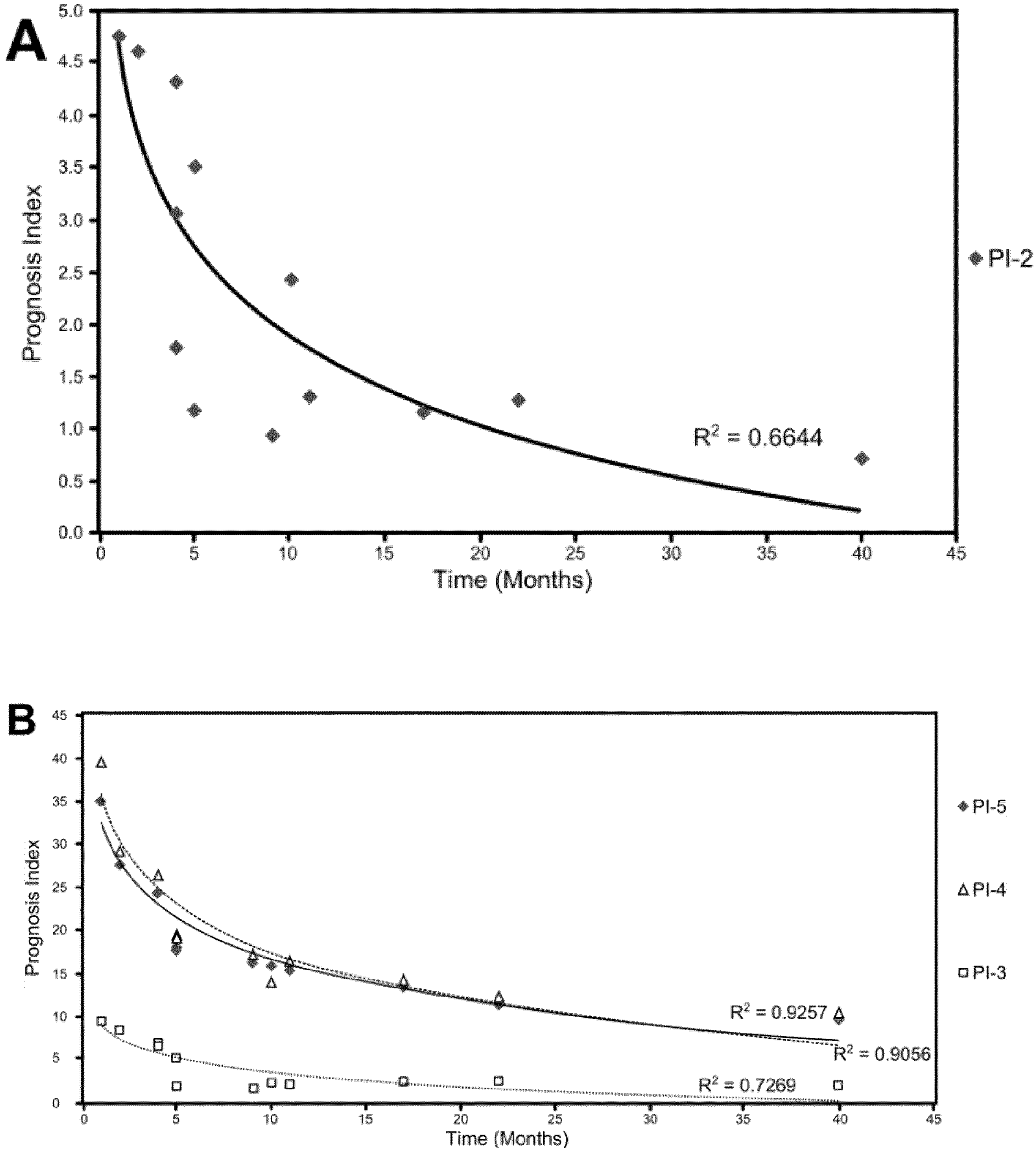
A
5.0
4.5
4.0
3.5
3.0
2.5
2.0
1.5
1.0
0.5
0.0
Prognosis Index
0
5
10
15
20
25
30
35
40
45
Time (Months)
$R^2$ = 0.6644
PI-2
B
45
40
35
30
25
20
15
10
5
0
Prognosis Index
Time (Months)
PI-5
PI-4
PI-3
$R^2$ = 0.9257
$R^2$ = 0.9056
$R^2$ = 0.7269

Fig. 3

A

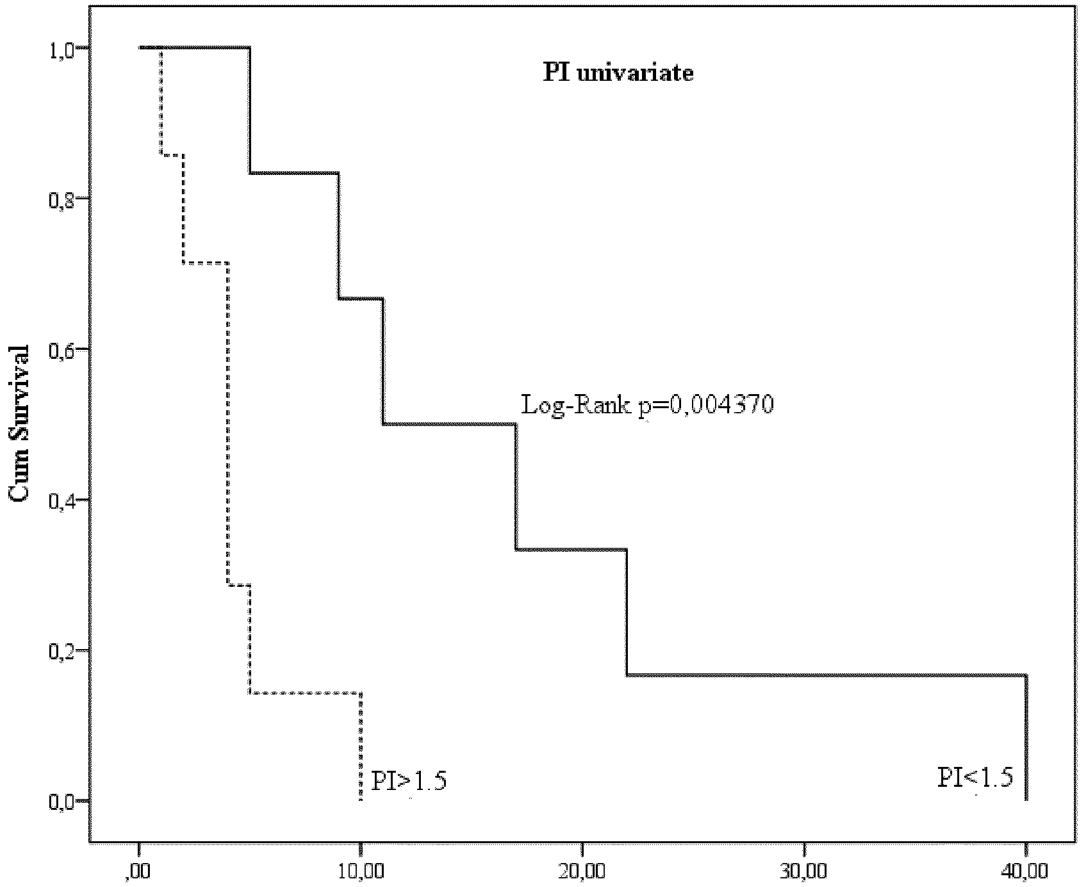
PI univariate
Cum Survival
1,0
0,8
0,6
0,4
0,2
0,0
Log-Rank p=0,004370
PI>1.5
PI<1.5
,00
10,00
20,00
30,00
40,00

**Fig. 3 cont.**

B

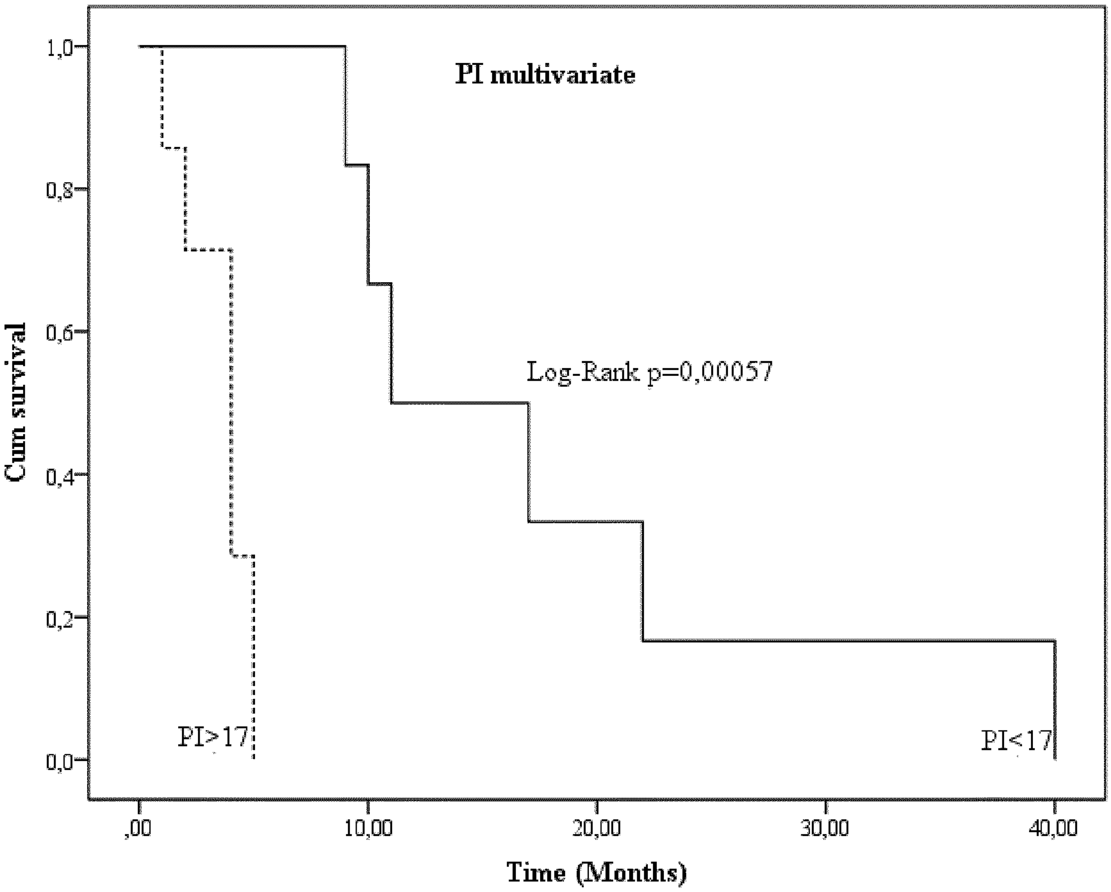
PI multivariate
Log-Rank p=0,00057
Cum survival
1,0
0,8
0,6
0,4
0,2
0,0
PI>17
PI<17
,00
10,00
20,00
30,00
40,00
Time (Months)

Fig. 4

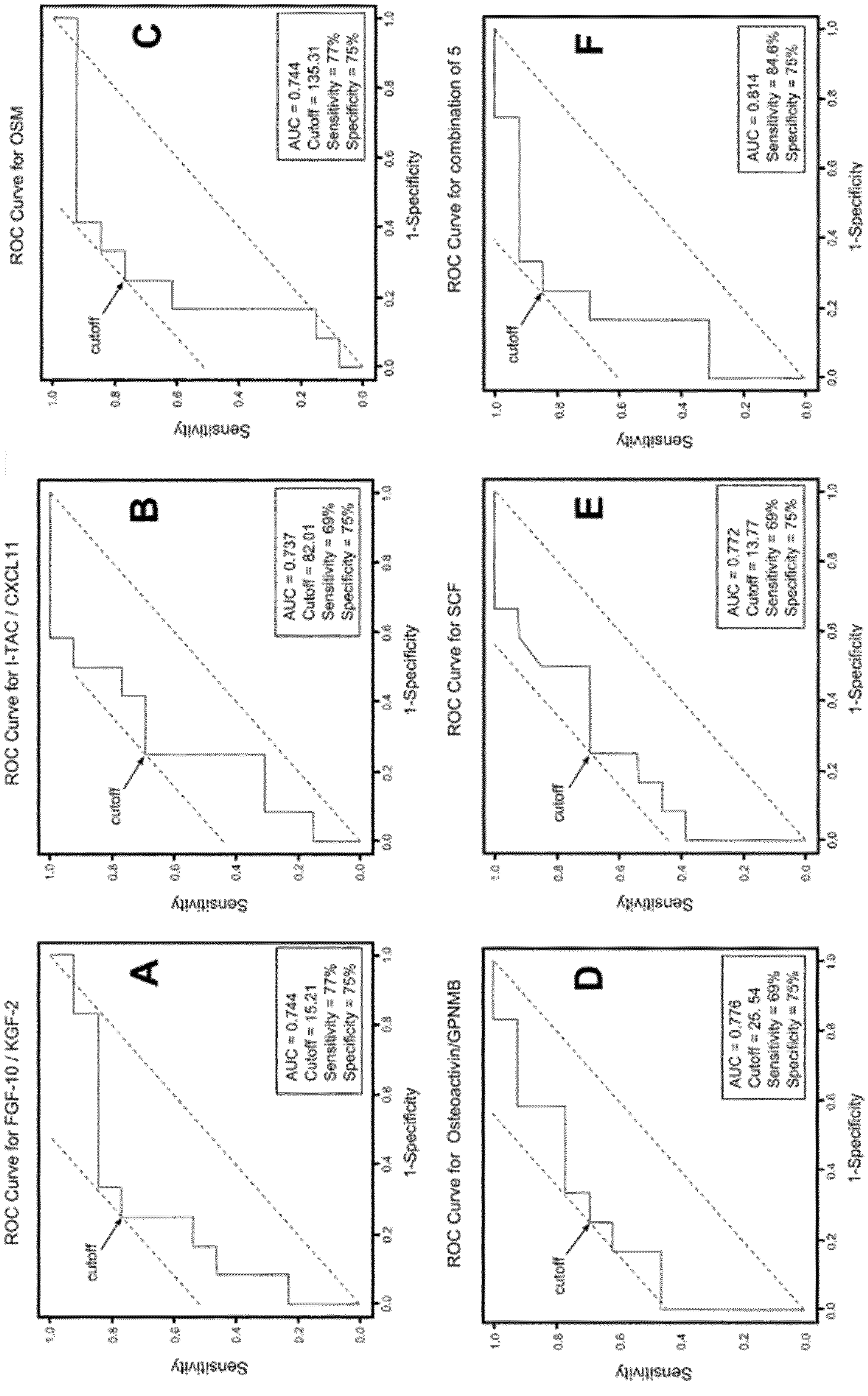
ROC Curve for FGF-10 / KGF-2
A
cutoff
AUC = 0.744
Cutoff = 15.21
Sensitivity = 77%
Specificity = 75%
ROC Curve for I-TAC / CXCL11
B
cutoff
AUC = 0.737
Cutoff = 82.01
Sensitivity = 69%
Specificity = 75%
ROC Curve for OSM
C
cutoff
AUC = 0.744
Cutoff = 135.31
Sensitivity = 77%
Specificity = 75%
ROC Curve for Osteoactivin/GPNMB
D
cutoff
AUC = 0.776
Cutoff = 25. 54
Sensitivity = 69%
Specificity = 75%
ROC Curve for SCF
E
cutoff
AUC = 0.772
Cutoff = 13.77
Sensitivity = 69%
Specificity = 75%
ROC Curve for combination of 5
F
cutoff
AUC = 0.814
Sensitivity = 84.6%
Specificity = 75%
Sensitivity
1-Specificity
0.0
0.2
0.4
0.6
0.8
1.0

## INTERNATIONAL SEARCH REPORT

International application No.
PCT/ES2014/070495

A. CLASSIFICATION OF SUBJECT MATTER

***G01N33/574*** (2006.01)
***G01N33/68*** (2006.01)
According to International Patent Classification (IPC) or to both national classification and IPC

B. FIELDS SEARCHED

Minimum documentation searched (classification system followed by classification symbols)
G01N

Documentation searched other than minimum documentation to the extent that such documents are included in the fields searched

Electronic data base consulted during the international search (name of data base and, where practicable, search terms used)

INVENES, EPODOC, WPI, NPL, BIOSIS, MEDLINE, EMBASE, USPTO PATENT DATABASE, PUBMED, GOOGLE PATENTS, GOOGLE SCHOLAR.

C. DOCUMENTS CONSIDERED TO BE RELEVANT

| Category* | Citation of document, with indication, where appropriate, of the relevant passages | Relevant to claim No. |
|---|---|---|
| A | MURRAY KORC. Pancreatic cancer-associated stroma production. The American Journal of Surgery. 2007. Vol. 194, pages: S84-S86, the whole document | 1-10, 13-15 |
| A | MARKUS WAGNER. et al. Suppression of Fibroblast Growth Factor Receptor Signaling Inhibits Pancreatic Cancer Growth In Vitro and In Vivo. Gastroenterology. 1998. Vol. 114, pages: 798-807, the whole document. | 1-10, 13-15 |
| A | WEIXIA ZHONG. et al. CXCL12/CXCR4 axis plays pivotal roles in the organ-specific metastasis of pancreatic adenocarcinoma: A clinical study. Experimental and Therapeutic Medicine. 2012. Vol. 4, pages: 363-369, the whole document. | 1-10, 13-15 |

☒ Further documents are listed in the continuation of Box C. ☒ See patent family annex.

* Special categories of cited documents:
"A" document defining the general state of the art which is not considered to be of particular relevance.
"E" earlier document but published on or after the international filing date
"L" document which may throw doubts on priority claim(s) or which is cited to establish the publication date of another citation or other special reason (as specified)
"O" document referring to an oral disclosure use, exhibition, or other means.
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other documents , such combination being obvious to a person skilled in the art
"&" document member of the same patent family

| Date of the actual completion of the international search | Date of mailing of the international search report |
|---|---|
| 15/10/2014 | **(20/10/2014)** |
| Name and mailing address of the ISA/<br><br>OFICINA ESPAÑOLA DE PATENTES Y MARCAS<br>Paseo de la Castellana, 75 - 28071 Madrid (España)<br>Facsimile No.: 91 349 53 04 | Authorized officer<br>M. García Grávalos<br><br>Telephone No. 91 3493404 |

Form PCT/ISA/210 (second sheet) (July 2009)

## INTERNATIONAL SEARCH REPORT

International application No.
PCT/ES2014/070495

C (continuation). DOCUMENTS CONSIDERED TO BE RELEVANT

| Category * | Citation of documents, with indication, where appropriate, of the relevant passages | Relevant to claim No. |
|---|---|---|
| A | SLATER EP. et al. LCN2 and TIMP1 as Potential Serum Markers for the Early Detection of Familial Pancreatic Cancer. Translational Oncology. April 2013. Vol. 6(2), pages: 99-103,the whole document. | 1-10, 12-15 |
| A | ES 2403781 A1 (SERVICIO ANDALUZ DE SALUD Y UNIVERSIDAD DE GRANADA) 21.05.2013, page 8, line 20 – page 11, line 24; claims 1-10. | 1-10, 13-15 |

Form PCT/ISA/210 (continuation of second sheet) (July 2009)

**SUPPLEMENTARY INTERNATIONAL SEARCH REPORT**

International application No. PCT/ES2014/070495

**Box No. II Observations where certain claims were found unsearchable (Continuation of item 2 of first sheet)**

This supplementary international search report has not been established in respect of certain claims under Article 17(2)(a) and Rule 45*bis*.5(c) and (d) for the following reasons:

1. ☒ Claims Nos.: Number 11 and 12
because they relate to subject matter not required to be searched by this Authority, namely:

Claims 11 and 12 have not been searched when this international search report was prepared, since they lack clarity, technical features and support by the description, and therefore fail to comply with the requirements of PCT Article 6.

2. ☐ Claims Nos.:
because they relate to parts of the international application that do not comply with the prescribed requirements to such an extent that no meaningful supplementary international search can be carried out, specifically:

3. ☐ Claims Nos.:
because they are dependent claims and are not drafted in accordance with the second and third sentences of Rule 6.4(a).

4. ☐ Claims Nos.:
because they were not the subject of the international search (Rule 45*bis*.5(d)).

**Box No. III Observations concerning unity of invention (Continuation of item 3 of first sheet)**

1. ☐ This Authority specified for supplementary search agrees with the conclusions of the International Searching Authority regarding the issue of unity of invention (see Forms PCT/ISA/210 and 237 dated ____________ ) and refers the applicant to these documents for further details.

2. ☐ At the request of the applicant, this supplementary international search report is limited to the invention specified by the applicant under Rule 45*bis*.1(d) and those parts of the international application which relate to that invention (Rule 45*bis*.5(b)).

3. This Authority specified for supplementary search:

(i) considers that there are ____________ *(number)* inventions claimed in the international application covered by the claims indicated below/on an extra sheet:

(ii) therefore finds that **the international application does not comply with the requirement of unity of invention** (Rules 13.1, 13.2 and 13.3) for the reasons indicated below/on an extra sheet:

(iii) draws the attention of the applicant to the possibility of requesting, within **one month** from the date of mailing of this report, a review of this opinion.

☐ Where the applicant requests the Authority to review this opinion, the applicant is hereby invited, within **one month** from the date of mailing of this report, to pay a review fee (Rule 45*bis*.6(c)) in the amount of ____________ *(currency/amount)*

4. ☐ This supplementary international search report therefore covers only those parts of the international application which relate to the invention first mentioned in the claims ("main invention"). Consequently, this supplementary international search report covers only the following claims: ____________

5. ☐ As all searchable claims could be searched without unreasonable additional effort, this supplementary international search report covers all claimed inventions.

Form PCT/SISA/501 (continuation of first sheet (2)) (January 2009)

## INTERNATIONAL SEARCH REPORT

Information on patent family members

International application No.
PCT/ES2014/070495

| Patent document cited in the search report | Publication date | Patent family member(s) | Publication date |
|---|---|---|---|
| ES2403781 A1 | 21.05.2013 | WO2012059617 A1 | 10.05.2012 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SIEGEL et al.** *C.A. Cancer J. Clin.,* 2012, vol. 62, 10-29
- **HIDALGO.** *N. Engl. J. Med.,* 2010, vol. 362, 1605-1617
- **SAKAR et al.** *Toxicol. Appl. Pharmacol.,* 2007, vol. 224, 326-336
- **AGARWAL et al.** *Pancreas,* 2008, vol. 36, 15-20
- **CHAN et al.** *J. Proteomics,* 2012
- **DUFFY et al.** *Ann. Oncl.,* 2010, vol. 21, 441-447
- **MANTOVANI et al.** *Nature,* 2008, vol. 454, 436-444
- **GERMANO et al.** *Cytokine,* 2008, vol. 43, 374-379
- **SHIELDS et al.** *Biochem J.,* 2012, vol. 441, 541-542
- **NESSE et al.** *Gut.,* 2011, vol. 60, 861-868
- **LUO et al.** *Biochim. Biophys Acta.,* 2012, vol. 1826, 170-178
- **BREMAN et al.** *Nat. Rev. Cancer,* 2010, vol. 10, 605-617
- **HIDALGO.** *Pancreatic cancer. N. Engl. J. Med.,* 2010, vol. 362, 1605-1617
- **COSTELLO et al.** *Nat. Rev. Gastroenterol Hepatol.,* 2012, vol. 9, 435-444
- **FONG et al.** *Cancer J.,* 2012, vol. 18, 530-538
- **JAMIESON et al.** *Clin Cancer Res,* 2011, vol. 17, 3316-333
- **GARCEA et al.** *Eur. J. Cancer,* 2005, vol. 41, 2213-2236
- **LUO et al.** *Hum. Pathol.,* 2013, vol. 44, 69-76
- **MANN et al.** *PLoS One,* 2012, vol. 7, e51119
- **DALLOL et al.** *Cancer Epidemiol. Biomarkers. Prev.,* 2012, vol. 21, 2069-2075
- **SCHETTER et al.** *Carcinogenesis,* 2010, vol. 31, 37-49
- **BHUSHAN et al.** *Development,* 2001, vol. 128, 5109-5117
- **NOMURA et al.** *Br. J. Cancer,* 2008, vol. 99, 305-313
- **MIEKUS et al.** *Folia Histochem. Cytobiol.,* 2010, vol. 48, 104-111
- **LO et al.** *Am. J. Pathol.,* 2010, vol. 176, 2435-2446
- **FURUYA et al.** *Gynecol. Oncol.,* 2011, vol. 122, 648-655
- **KLEE et al.** *Clin. Toxicol.,* 2012, vol. 58, 599-609
- **ZARLING et al.** *Proc. Nat. Acad. Sci. USA.,* 1986, vol. 83, 9739-43
- **FOSSEY et al.** *BMC Cancer,* 2011, vol. 11, 125
- **LI et al.** *Int. J. Mol. Med.,* 2011, vol. 28, 101-108
- **WINDER et al.** *J. Pathol.,* 2011, vol. 225, 448-462
- **TIFFEN et al.** *Mol. Endocrinol.,* 2008, vol. 22, 2677-2688
- **CORCORAN et al.** *Cancer Res.,* 2011, vol. 71, 5020-5029
- **WETERMAN et al.** *Int. J. Cancer,* 1995, vol. 60, 73-81
- **ROSE et al.** *PLos One,* 2010, vol. 5, 12093
- **TOMIHARI et al.** *Cancer Res,* 2010, vol. 70, 5778-5787
- **TSE et al.** *Clin. Cancer Res.,* 2006, vol. 12, 1373-1382
- **WILLIAMS et al.** *Melanoma res.,* 2010, vol. 20, 184-190
- **KUAN et al.** *Clin. Cancer Res.,* 2006, vol. 12, 1970-1982
- **ONAGA et al.** *J. Hepatol.,* 2003, vol. 39, 779-785
- **ROSE et al.** *Clin. Cancer Res.,* 2010, vol. 16, 2147-2156
- **TANAKA et al.** *Sci. Rep.,* 2012, vol. 2, 573
- **YASUDA et al.** *Mol. Cancer,* 2006, vol. 5, 46
- **YASUDA et al.** *Dig. Dis. Sci.,* 2007, vol. 52, 2292-2300
- **MROCZKO et al.** *Clin. Toxicol. Lab. Med.,* 2005, vol. 43, 146-150
- **MROCZKO et al.** *Clin. Toxicol. Lab. Med.,* 2004, vol. 42, 256-260
- **MROCZKO et al.** *Int.J. Colorectal Dis.,* 2005, vol. 22, 33-38
- **SELIGER et al.** *Cancer Immunol. Immunother.,* 2012, vol. 61, 1327-1341
- **CHEN et al.** *J. Exp. Med.,* 1994, vol. 179, 523-532
- **WANG et al.** *PLoS One,* 2012, vol. 7, e45491
- **WANG et al.** *World J. Surg.,* 2010, vol. 34, 1059-1065
- **LECOUTER et al.** *Nature,* 2001, vol. 412, 877-884
- **BALU et al.** *Rom. J. Morphol. Embryol.,* 2012, vol. 53, 479-483

- **NAGANO et al.** *J. Surg. Oncol.,* 2007, vol. 96, 605-610
- **PASQUALI et al.** *Endocrinology,* 2006, vol. 147, 4245-4251
- **LI et al.** *J. Exp. Clin. Cancer Res.,* 2006, vol. 25, 403-409
- **JIANG et al.** *Pancreatology,* 2009, vol. 9, 165-172
- **NGAN et al.** *Clin. Cancer Res.,* 2007, vol. 13, 868-875
- **BROUILLET, S.** *Trends Endocrinol. Metab.,* 2012, vol. 23, 501-508
- **KOTENKO et al.** *Curr. Opin. Immunol.,* 2011, vol. 23, 583-590
- **MAHER et al.** *Cancer Biol. Ther.,* 2008, vol. 7, 1109-1115
- **DONNELLY et al.** *J. Interferon Cytokine Res.,* 2010, vol. 30, 555-564
- **NOVAK et al.** *Leukemia,* 2008, vol. 22, 2240-2246
- **FALLS et al.** *Exp. Cell. Res.,* 2003, vol. 284, 14-30
- **HAYES et al.** *J. Mammary Gland. Biol. Neoplasia,* 2008, vol. 13, 205-214
- **YEN et al.** *Oncogene,* 2000, vol. 19, 3460-3469
- **STOVE et al.** *Clin. Exp. Metástasis,* 2004, vol. 21, 665-684
- **LILES et al.** *Br. J. Cancer,* 2011, vol. 105, 523-533

SEQUENCE LISTING

<110> UNIVERSIDAD DE GRANADA
SERVICIO ANDALUZ DE SALUD
UNIVERSIDAD DE JAÉN

<120> BIOMARKERS FOR DIAGNOSIS AND RESPONSE TO TREATMENT IN CANCER OF THE PANCREAS

<130> IPR-470_1-PCT

<160> 32

<170> PatentIn version 3.5

<210> 1
<211> 627
<212> DNA
<213> Homo sapiens

<400> 1
atgtggaaat ggatactgac acattgtgcc tcagcctttc cccacctgcc cggctgctgc 60

tgctgctgct ttttgttgct gttcttggtg tcttccgtcc ctgtcacctg ccaagccctt 120

ggtcaggaca tggtgtcacc agaggccacc aactcttctt cctcctcctt ctcctctcct 180

tccagcgcgg gaaggcatgt gcggagctac aatcaccttc aaggagatgt ccgctggaga 240

aagctattct ctttcaccaa gtactttctc aagattgaga agaacgggaa ggtcagcggg 300

accaagaagg agaactgccc gtacagcatc ctggagataa catcagtaga aatcggagtt 360

gttgccgtca aagccattaa cagcaactat tacttagcca tgaacaagaa ggggaaactc 420

tatggctcaa aagaatttaa caatgactgt aagctgaagg agaggataga ggaaaatgga 480

tacaatacct atgcatcatt taactggcag cataatggga ggcaaatgta tgtggcattg 540

aatggaaaag gagctccaag gagaggacag aaaacacgaa ggaaaaacac ctctgctcac 600

tttcttccaa tggtggtaca ctcatag 627

<210> 2
<211> 1610
<212> DNA
<213> Homo sapiens

<400> 2
agagaacaaa acagaaactc ttggaagcag gaaaggtgca tgactcaaag agggaaattc 60

ctgtgccata aaaggattgc tggtgtataa aatgctctat atatgccaat tatcaatttc 120

ctttcatgtt cagcatttct actccttcca agaagagcag caaagctgaa gtagcagcag 180

cagcaccagc agcaacagca aaaaacaaac atgagtgtga agggcatggc tatagccttg 240

gctgtgatat tgtgtgctac agttgttcaa ggcttcccca tgttcaaaag aggacgctgt 300

ctttgcatag gccctggggt aaaagcagtg aaagtggcag atattgagaa agcctccata 360

atgtacccaa gtaacaactg tgacaaaata gaagtgatta ttaccctgaa agaaaataaa 420

```
ggacaacgat gcctaaatcc caaatcgaag caagcaaggc ttataatcaa aaaagttgaa      480

agaaagaatt tttaaaaata tcaaaacata tgaagtcctg gaaaagagca tctgaaaaac      540

ctagaacaag tttaactgtg actactgaaa tgacaagaat tctacagtag gaaactgaga      600

cttttctatg gttttgtgac tttcaacttt tgtacagtta tgtgaaggat gaaaggtggg      660

tgaaaggacc aaaaacagaa atacagtctt cctgaatgaa tgacaatcag aattccactg      720

cccaaaggag tccaacaatt aaatggattt ctaggaaaag ctaccttaag aaaggctggt      780

taccatcgga gtttacaaag tgctttcacg ttcttacttg ttgcattata cattcatgca      840

tttctaggct agagaacctt ctagatttga tgcttacaac tattctgttg tgactatgag      900

aacatttctg tctctagaag tcatctgtct gtattgatct ttatgctata ttactatctg      960

tggttacggt ggagacattg acattattac tggagtcaag cccttataag tcaaaagcat     1020

ctatgtgtcg taaaacattc ctcaaacatt ttttcatgca aatacacact tctttcccca     1080

aacatcatgt agcacatcaa tatgtaggga gacattctta tgcatcattt ggtttgtttt     1140

ataaccaatt cattaaatgt aattcataaa atgtactatg aaaaaaatta tacgctatgg     1200

gatactggca aaagtgcaca tatttcataa ccaaattagt agcaccagtc ttaatttgat     1260

gtttttcaac ttttattcat tgagatgttt tgaagcaatt aggatatgtg tgtttactgt     1320

actttttgtt ttgatccgtt tgtataaatg atagcaatat cttggacaca tctgaaatac     1380

aaaatgtttt tgtctaccaa agaaaaatgt tgaaaaataa gcaaatgtat acctagcaat     1440

cacttttact ttttgtaatt ctgtctctta gaaaaataca taatctaatc aatttctttg     1500

ttcatgccta tatactgtaa aatttaggta tactcaagac tagtttaaag aatcaaagtc     1560

atttttttct ctaataaact accacaacct ttcttttttta aaaaaaaaaa                1610
```

<210> 3
<211> 1869
<212> DNA
<213> Homo sapiens

<400> 3

```
gtcacccccca gcgggcgcgg gccggagcac gggcacccag catgggggta ctgctcacac       60

agaggacgct gctcagtctg gtccttgcac tcctgtttcc aagcatggcg agcatggcgg      120

ctataggcag ctgctcgaaa gagtaccgcg tgctccttgg ccagctccag aagcagacag      180

atctcatgca ggacaccagc agactcctgg acccctatat acgtatccaa ggcctggatg      240

ttcctaaact gagagagcac tgcagggagc gccccggggc cttccccagt gaggagaccc      300

tgaggggggct gggcaggcgg ggcttcctgc agaccctcaa tgccacactg ggctgcgtcc      360

tgcacagact ggccgactta gagcagcgcc tccccaaggc ccaggatttg gagaggtctg      420

ggctgaacat cgaggacttg gagaagctgc agatggcgag gccgaacatc ctcgggctca      480
```

ggaacaacat ctactgcatg gcccagctgc tggacaactc agacacggct gagcccacga 540

aggctggccg gggggcctct cagccgccca cccccacccc tgcctcggat gcttttcagc 600

gcaagctgga gggctgcagg ttcctgcatg gctaccatcg cttcatgcac tcagtggggc 660

gggtcttcag caagtggggg gagagcccga accggagccg gagacacagc cccccaccagg 720

ccctgaggaa gggggtgcgc aggaccagac cctccaggaa aggcaagaga ctcatgacca 780

ggggacagct gccccggtag cctcgagagc acccccttgcc ggtgaaggat gcggcaggtg 840

ctctgtggat gagaggaacc atcgcaggat gacagctccc gggtccccaa acctgttccc 900

ctctgctact agccactgag aagtgcactt taagaggtgg gagctgggca gacccctcta 960

cctcctccag gctgggagac agagtcaggc tgttgcgctc ccacctcagc cccaagttcc 1020

ccaggcccag tggggtggcc gggcgggcca cgcgggaccg actttccatt gattcagggg 1080

tctgatgaca caggctgact catggccggg ctgactgccc ccctgccttg ctccccgagg 1140

cctgccggtc cttccctctc atgacttgca gggccgttgc ccccagactt cctcctttcc 1200

gtgtttctga aggggaggtc acagcctgag ctggcctcct atgcctcatc atgtcccaaa 1260

ccagacacct ggatgtctgg gtgacctcac tttaggcagc tgtaacagcg gcagggtgtc 1320

ccaggagccc tgatccgggg gtccagggaa tggagctcag gtcccaggcc agccccgaag 1380

tcgccacgtg gcctggggca ggtcacttta cctctgtgga cctgttttct ctttgtgaag 1440

ctagggagtt agaggctgta caaggccccc actgcctgtc ggttgcttgg attccctgac 1500

gtaaggtgga tattaaaaat ctgtaaatca ggacaggtgg tgcaaatggc gctgggaggt 1560

gtacacggag gtctctgtaa aagcagaccc acctcccagc gccgggaagc ccgtcttggg 1620

tcctcgctgc tggctgctcc ccctggtggt ggatcctgga attttctcac gcaggagcca 1680

ttgctctcct agagggggtc tcagaaactg cgaggccagt tccttggagg gacatgacta 1740

atttatcgat ttttatcaat ttttatcagt tttatattta taagccttat ttatgatgta 1800

tatttaatgt taatattgtg caaacttata tttaaaactt gcctggtttc taaaaaaaaa 1860

aaaaaaaaa 1869

<210> 4
<211> 2775
<212> DNA
<213> Homo sapiens

<400> 4
tgccgcttaa taccatcaca tgatcctccc cgaggccctg tatttaatta aaatagagag 60

ggaggcacca cagatgccag aagaacactg ttgctcttgg tggacgggcc cagaggaatt 120

cagagttaaa ccttgagtgc ctgcgtccgt gagaattcag catggaatgt ctctactatt 180

tcctgggatt tctgctcctg gctgcaagat tgccacttga tgccgccaaa cgatttcatg 240

atgtgctggg caatgaaaga ccttctgctt acatgaggga gcacaatcaa ttaaatggct 300

ggtcttctga tgaaaatgac tggaatgaaa aactctaccc agtgtggaag cggggagaca 360

tgaggtggaa aaactcctgg aagggaggcc gtgtgcaggc ggtcctgacc agtgactcac 420

cagccctcgt gggctcaaat ataacatttg cggtgaacct gatattccct agatgccaaa 480

aggaagatgc caatggcaac atagtctatg agaagaactg cagaaatgag gctggtttat 540

ctgctgatcc gtatgtttac aactggacag catggtcaga ggacagtgac ggggaaaatg 600

gcaccggcca aagccatcat aacgtcttcc ctgatgggaa accttttcct caccaccccg 660

gatggagaag atggaatttc atctacgtct tccacacact tggtcagtat ttccagaaat 720

tgggacgatg ttcagtgaga gtttctgtga acacagccaa tgtgacactt gggcctcaac 780

tcatggaagt gactgtctac agaagacatg gacgggcata tgttcccatc gcacaagtga 840

aagatgtgta cgtggtaaca gatcagattc ctgtgtttgt gactatgttc cagaagaacg 900

atcgaaattc atccgacgaa accttcctca aagatctccc cattatgttt gatgtcctga 960

ttcatgatcc tagccacttc ctcaattatt ctaccattaa ctacaagtgg agcttcgggg 1020

ataatactgg cctgtttgtt tccaccaatc atactgtgaa tcacacgtat gtgctcaatg 1080

gaaccttcag ccttaacctc actgtgaaag ctgcagcacc aggaccttgt ccgccaccgc 1140

caccaccacc cagaccttca aaacccaccc cttctttagc aactactcta aaatcttatg 1200

attcaaacac cccaggacct gctggtgaca accccctgga gctgagtagg attcctgatg 1260

aaaactgcca gattaacaga tatggccact ttcaagccac catcacaatt gtagagggaa 1320

tcttagaggt taacatcatc cagatgacag acgtcctgat gccggtgcca tggcctgaaa 1380

gctccctaat agactttgtc gtgacctgcc aagggagcat tcccacggag gtctgtacca 1440

tcatttctga ccccacctgc gagatcaccc agaacacagt ctgcagccct gtggatgtgg 1500

atgagatgtg tctgctgact gtgagacgaa ccttcaatgg gtctgggacg tactgtgtga 1560

acctcaccct gggggatgac acaagcctgg ctctcacgag caccctgatt tctgttcctg 1620

acagagaccc agcctcgcct ttaaggatgg caaacagtgc cctgatctcc gttggctgct 1680

tggccatatt tgtcactgtg atctccctct tggtgtacaa aaaacacaag gaatacaacc 1740

caatagaaaa tagtcctggg aatgtggtca gaagcaaagg cctgagtgtc tttctcaacc 1800

gtgcaaaagc cgtgttcttc ccgggaaacc aggaaaagga tccgctactc aaaaaccaag 1860

aatttaaagg agtttcttaa atttcgacct tgtttctgaa gctcactttt cagtgccatt 1920

gatgtgagat gtgctggagt ggctattaac cttttttttcc taaagattat tgttaaatag 1980

atattgtggt ttggggaagt tgaatttttt ataggttaaa tgtcatttta gagatgggga 2040

gagggattat actgcaggca gcttcagcca tgttgtgaaa ctgataaaag caacttagca 2100

aggcttcttt tcattatttt ttatgtttca cttataaagt cttaggtaac tagtaggata 2160

gaaacactgt gtcccgagag taaggagaga agctactatt gattagagcc taacccaggt 2220

taactgcaag aagaggcggg atactttcag ctttccatgt aactgtatgc ataaagccaa 2280

tgtagtccag tttctaagat catgttccaa gctaactgaa tcccacttca atacacactc 2340

atgaactcct gatggaacaa taacaggccc aagcctgtgg tatgatgtgc acacttgcta 2400

gactcagaaa aaatactact ctcataaatg ggtgggagta ttttggtgac aacctacttt 2460

gcttggctga gtgaaggaat gatattcata tattcattta ttccatggac atttagttag 2520

tgctttttat ataccaggca tgatgctgag tgacactctt gtgtatattt ccaaattttt 2580

gtacagtcgc tgcacatatt tgaaatcata tattaagact ttccaaagat gaggtccctg 2640

gtttttcatg gcaacttgat cagtaaggat ttcacctctg tttgtaacta aaaccatcta 2700

ctatatgtta gacatgacat tctttttctc tccttcctga aaaataaagt gtgggaagag 2760

acaagaaaaa aaaaa 2775

<210> 5
<211> 2739
<212> DNA
<213> Homo sapiens

<400> 5
tgccgcttaa taccatcaca tgatcctccc cgaggccctg tatttaatta aaatagagag 60

ggaggcacca cagatgccag aagaacactg ttgctcttgg tggacgggcc cagaggaatt 120

cagagttaaa ccttgagtgc ctgcgtccgt gagaattcag catggaatgt ctctactatt 180

tcctgggatt tctgctcctg gctgcaagat tgccacttga tgccgccaaa cgatttcatg 240

atgtgctggg caatgaaaga ccttctgctt acatgaggga gcacaatcaa ttaaatggct 300

ggtcttctga tgaaaatgac tggaatgaaa aactctaccc agtgtggaag cggggagaca 360

tgaggtggaa aaactcctgg aagggaggcc gtgtgcaggc ggtcctgacc agtgactcac 420

cagccctcgt gggctcaaat ataacatttg cggtgaacct gatattccct agatgccaaa 480

aggaagatgc caatggcaac atagtctatg agaagaactg cagaaatgag gctggtttat 540

ctgctgatcc gtatgtttac aactggacag catggtcaga ggacagtgac ggggaaaatg 600

gcaccggcca aagccatcat aacgtcttcc ctgatgggaa accttttcct caccaccccg 660

gatggagaag atggaatttc atctacgtct tccacacact tggtcagtat ttccagaaat 720

tgggacgatg ttcagtgaga gtttctgtga acacagccaa tgtgacactt gggcctcaac 780

tcatggaagt gactgtctac agaagacatg gacgggcata tgttcccatc gcacaagtga 840

aagatgtgta cgtggtaaca gatcagattc ctgtgtttgt gactatgttc cagaagaacg 900

atcgaaattc atccgacgaa accttcctca aagatctccc cattatgttt gatgtcctga 960

ttcatgatcc tagccacttc ctcaattatt ctaccattaa ctacaagtgg agcttcgggg 1020

ataatactgg cctgtttgtt tccaccaatc atactgtgaa tcacacgtat gtgctcaatg 1080

gaaccttcag ccttaacctc actgtgaaag ctgcagcacc aggaccttgt ccgccaccgc 1140

caccaccacc cagaccttca aaacccaccc cttctttagg acctgctggt gacaaccccc 1200

tggagctgag taggattcct gatgaaaact gccagattaa cagatatggc cactttcaag 1260

ccaccatcac aattgtagag ggaatcttag aggttaacat catccagatg acagacgtcc 1320

tgatgccggt gccatggcct gaaagctccc taatagactt tgtcgtgacc tgccaaggga 1380

gcattcccac ggaggtctgt accatcattt ctgaccccac ctgcgagatc acccagaaca 1440

cagtctgcag ccctgtggat gtggatgaga tgtgtctgct gactgtgaga cgaaccttca 1500

atgggtctgg gacgtactgt gtgaacctca ccctggggga tgacacaagc ctggctctca 1560

cgagcaccct gatttctgtt cctgacagag acccagcctc gcctttaagg atggcaaaca 1620

gtgccctgat ctccgttggc tgcttggcca tatttgtcac tgtgatctcc ctcttggtgt 1680

acaaaaaaca caaggaatac aacccaatag aaaatagtcc tgggaatgtg gtcagaagca 1740

aaggcctgag tgtctttctc aaccgtgcaa aagccgtgtt cttcccggga aaccaggaaa 1800

aggatccgct actcaaaaac caagaattta aaggagtttc ttaaatttcg accttgtttc 1860

tgaagctcac ttttcagtgc cattgatgtg agatgtgctg gagtggctat taaccttttt 1920

ttcctaaaga ttattgttaa atagatattg tggtttgggg aagttgaatt ttttataggt 1980

taaatgtcat tttagagatg gggagaggga ttatactgca ggcagcttca gccatgttgt 2040

gaaactgata aaagcaactt agcaaggctt cttttcatta ttttttatgt ttcacttata 2100

aagtcttagg taactagtag gatagaaaca ctgtgtcccg agagtaagga gagaagctac 2160

tattgattag agcctaaccc aggttaactg caagaagagg cgggatactt tcagctttcc 2220

atgtaactgt atgcataaag ccaatgtagt ccagtttcta agatcatgtt ccaagctaac 2280

tgaatcccac ttcaatacac actcatgaac tcctgatgga acaataacag gcccaagcct 2340

gtggtatgat gtgcacactt gctagactca gaaaaaatac tactctcata aatgggtggg 2400

agtattttgg tgacaaccta ctttgcttgg ctgagtgaag gaatgatatt catatattca 2460

tttattccat ggacatttag ttagtgcttt ttatatacca ggcatgatgc tgagtgacac 2520

tcttgtgtat atttccaaat ttttgtacag tcgctgcaca tatttgaaat catatattaa 2580

gactttccaa agatgaggtc cctggttttt catggcaact tgatcagtaa ggatttcacc 2640

tctgtttgta actaaaacca tctactatat gttagacatg acattctttt tctctccttc 2700

ctgaaaaata aagtgtggga agagacaaga aaaaaaaa 2739

<210> 6
<211> 5376

<212> DNA
<213> Homo sapiens

<400> 6

```
gggcttcgct cgccgcctcg cgccgagact agaagcgctg cgggaagcag ggacagtgga      60
gagggcgctg cgctcgggct acccaatgcg tggactatct gccgccgctg ttcgtgcaat     120
atgctggagc tccagaacag ctaaacggag tcgccacacc actgtttgtg ctggatcgca     180
gcgctgcctt tccttatgaa gaagacacaa acttggattc tcacttgcat ttatcttcag     240
ctgctcctat ttaatcctct cgtcaaaact gaagggatct gcaggaatcg tgtgactaat     300
aatgtaaaag acgtcactaa attggtggca aatcttccaa aagactacat gataaccctc     360
aaatatgtcc ccgggatgga tgttttgcca agtcattgtt ggataagcga gatggtagta     420
caattgtcag acagcttgac tgatcttctg gacaagtttt caaatatttc tgaaggcttg     480
agtaattatt ccatcataga caaacttgtg aatatagtgg atgaccttgt ggagtgcgtg     540
aaagaaaact catctaagga tctaaaaaaa tcattcaaga gcccagaacc caggctcttt     600
actcctgaag aattctttag aatttttaat agatccattg atgccttcaa ggactttgta     660
gtggcatctg aaactagtga ttgtgtggtt tcttcaacat taagtcctga gaaagggaag     720
gccaaaaatc cccctggaga ctccagccta cactgggcag ccatggcatt gccagcattg     780
ttttctctta taattggctt tgcttttgga gccttatact ggaagaagag acagccaagt     840
cttacaaggg cagttgaaaa tatacaaatt aatgaagagg ataatgagat aagtatgttg     900
caagagaaag agagagagtt tcaagaagtg taattgtggc ttgtatcaac actgttactt     960
tcgtacattg gctggtaaca gttcatgttt gcttcataaa tgaagcagct ttaaacaaat    1020
tcatattctg tctggagtga cagaccacat ctttatctgt tcttgctacc catgacttta    1080
tatggatgat tcagaaattg gaacagaatg ttttactgtg aaactggcac tgaattaatc    1140
atctataaag aagaacttgc atggagcagg actctatttt aaggactgcg ggacttgggt    1200
ctcatttaga acttgcagct gatgttggaa gagaaagcac gtgtctcaga ctgcatgtac    1260
catttgcatg gctccagaaa tgtctaaatg ctgaaaaaac acctagcttt attcttcaga    1320
tacaaactgc agcctgtagt tatcctggtc tctgcaagta gatttcagct tggatagtga    1380
gggtaacaat tttctcaaa gggatctgga aaaaatgttt aaaactcagt agtgtcagcc     1440
actgtacagt gtagaaagca gtgggaactg tgattggatt tggcaacatg tcagctttat    1500
agttgccgat tagtgatatg ggtctgattt cgatctcttc ctgatgtaaa ccatgctcac    1560
ccatatccca ctatacaaat gcaaatggtt gcctggttcc atttatgcaa gggagccagt    1620
actgaattat gccttggcag aggggagact ccaaaagagt catcgcagga agaagttaag    1680
aacactgaac atcagaacag tctgccaaga aggacattgg catcctggga aagtccgcct    1740
tttcccttga ccactatagg gtgtataaat cgtgtttgca aaatgtgtta tgatgtgttt    1800
```

```
atattctaaa actattacag agctatgtaa agggacttag gagaaaatgc tgaatgtaag     1860
atggtcccat ttcaatttcc accatgggag agcctaaaaa taaattatga catttagtat     1920
ctaaggttag aaaaccacgc ccacatgcta atatgggtgt tgaaaactag gttacttata     1980
atgcaaggaa tcaggaaact ttagttattt atagtataat caccattatc tgtttaaagg     2040
atccatttag ttaaaatcgg gcactctata ttcattaagg tttatgaatt aaaaagaaag     2100
ctttatgtag ttatgcatgt cagtttgcta tttaaaatgt gtgacagtgt ttgtcatatt     2160
aagagtgaat ttggcaggaa ttcccaagat ggacattgtg cttttaaact agaacttgta     2220
agacattatg tgaatatccc ttgccaattt tttttataat aagaaaacat ctgactaaag     2280
tcaaagaatg atttcttatg gtttattttg atgaaagttc ttttaacatg tcttgaatgt     2340
acacataaag gaatccaaag ctttccattc taacttaatc tttgtgataa cattattgcc     2400
atgttctaca accgtaagat gacagttttc aatgtagtga cacaaaaggg catgaaaaac     2460
taactgctag ctttcctttc atttcaaaag tccaagaatt tctagtatat ttggatttta     2520
gcttctgttc aaagcaaatc cagatgcaac tccagtaagt ggcctttgct ctttttttgta     2580
ccaaagagcc cagatgattc ctacagtccc tttcttctct aacatgctgt ggttccttaa     2640
atatgagtaa tttctctaag atataaccca ggtgctttga gaagctgcat taaggtgttc     2700
aggccctcag atatcacatg gtacacttga ttagtaataa aaccagagat caatttaaat     2760
tgctgatagg tcctgtctca gtgtgtggca ttgactgttt tcaggaaaat agatacagat     2820
taatatgagt tatgcgtgta ggttgtgtat agattgagaa gatagatact tctcaatcta     2880
gtagtttgat ttatttaacc aatggtttca gtttgcttga gcatatgaaa atcctgctta     2940
atgtgcttaa gagtataata aatgtgtact tttgtcctca aacctagtag ctgggtttta     3000
acactcatgg acatggtctt aatcaatgga gttaaataaa caaattcagc aagttattaa     3060
atctgacatg gtaggagagg ggagatgtgt cctgcttatt aaatgtgttg gtccattgaa     3120
agttacatgg attgccaatt tttaaaacac taaagttgaa taaaatgcat gaacaataga     3180
aaaatgctga acattatttt ggatgctagc tgcttggaca ttaactgtgt tatttctgct     3240
ttgagatgaa aatatatatt tatctttgct tattttatcc cagatgtgtt ctgaatatcc     3300
ttcttcataa atcatggaaa actcactgct gagatagtaa accatgaaat cgccttttca     3360
gttggtgcca tgtatctgac agttccatct tggaaggttt caaaattacc ttttaaaatg     3420
atctcagaag tctgtagatt ctcaatgata ctgaaagctt tgcacctctt tggtagaaac     3480
caggtctatt tagaaaatgg ctttatgata aatgttgcct cctgagtgat aatgaagtgt     3540
tcctggatat tgtattgtaa tttaatgtgc ttaccacact gccacatttt aatgagtcag     3600
agaaaaatta atttttcttc aatacaataa tagaacaagt agcctattct cttaaaaagt     3660
```

atgtgaaaag aaaattatga aaaaatatgc atacctaatg aagtattggt tttagtaaga 3720

attaaataca tttcattgag ctttaaagta ctttggagaa actttggggc acgttttcct 3780

actctaattc aactaaagtt ataaataaag agaaaaactc attcagaaat catggatttt 3840

aaaaatattt tactgcagcc aagttttcat ttcaaaatgt aatttcagtt tggagctttt 3900

aggcattatg tatatttaaa aaatatattc ttcaaaaatg cattttggca tggtgggatg 3960

gatgttgcaa aagatatccg gagcctccag tctgtcatta actgatatgg taaatcacct 4020

ctcttctttg ggtctcaatt ttttatttat ctatatggta aactcagaga tcactcctta 4080

ggggtgagtc ctattgcaat atgaccgaca aagaagacaa aatagcattg aaactaaccc 4140

atacaaaata tccaactctg gattctgtga ataagtatct tgaccataaa aagtcattgc 4200

tgttcttgtt tctaatgtaa atagtgtcca ttagtaaaag tgaaattcag tcttaagtag 4260

ggtgaattgg atcaccattt acacaagaga tggctttttc ctttgcttga ataaacattt 4320

tggatcacct ccaaagaatg aaaaccagta gtacgtttta gtcatattag tcaggatgag 4380

aaactataag atgtgtgtaa catttggaaa tgcaccaaag tgagcgttta aatcttctca 4440

ttttattgaa aactaagagc agaaaatgta aaatgctcat gaaggttttg aatgccaaaa 4500

gatattttag aatcaattta taaaggggta attcattaat tacactttaa aattggaaag 4560

tgggataaga aatctaaagt aaaccagctt atctttgaaa caatattatt ttgaaattgg 4620

ctttaaaata aaaccattca gattgaaatt ctaattagct catttgtgga gtttgatcac 4680

acaattcata atgttgctgc tttccattaa ctagtcttga aatgcctttg tttgtaaaaa 4740

taaaataatg gtactttcat tttataacaa ggtgtttttt tcaagaaata atccatgcta 4800

aaatggatat ttgtgatcct gaaatgttta ctaagcattg taaatttatt tataactgcc 4860

atctccaact acatccttat gatgttttta acaataaaat taaaacaact gttaaactaa 4920

aaaccacacc gttttccagt acttgatctc tgagctacaa tactcactaa atataatttt 4980

ccaatcaaaa tattctattc tatattctaa gggttaatat gtgattatag tgtccacttg 5040

ccaccatttt tttaaatcaa tggacttgaa aagtattaat ttagatggat gcgcagatat 5100

accctcagtt cagtcataga ttggagtttg catataataa tgtaaatgta tgtcgacact 5160

attctaaata gttctattat gactgaaatt taattaaata aaaaaggttg taaaatgtga 5220

tgtgtatgtg tatatactgt atgtgtactt tttaaaatag gtgtatgtcc caaccctttt 5280

ttatacaggt ttgaatttaa aattacatga tatatacata tactttattg ttctaaataa 5340

agaattttat gcactctcaa aaaaaaaaaa aaaaaa 5376

<210> 7
<211> 5460
<212> DNA
<213> Homo sapiens

<400> 7

```
gggcttcgct cgccgcctcg cgccgagact agaagcgctg cgggaagcag ggacagtgga      60
gagggcgctg cgctcgggct acccaatgcg tggactatct gccgccgctg ttcgtgcaat     120
atgctggagc tccagaacag ctaaacggag tcgccacacc actgtttgtg ctggatcgca     180
gcgctgcctt tccttatgaa gaagacacaa acttggattc tcacttgcat ttatcttcag     240
ctgctcctat ttaatcctct cgtcaaaact gaagggatct gcaggaatcg tgtgactaat     300
aatgtaaaag acgtcactaa attggtggca aatcttccaa aagactacat gataaccctc     360
aaatatgtcc ccgggatgga tgttttgcca agtcattgtt ggataagcga gatggtagta     420
caattgtcag acagcttgac tgatcttctg gacaagtttt caaatatttc tgaaggcttg     480
agtaattatt ccatcataga caaacttgtg aatatagtgg atgaccttgt ggagtgcgtg     540
aaagaaaact catctaagga tctaaaaaaa tcattcaaga gcccagaacc caggctcttt     600
actcctgaag aattctttag aatttttaat agatccattg atgccttcaa ggactttgta     660
gtggcatctg aaactagtga ttgtgtggtt tcttcaacat taagtcctga gaaagattcc     720
agagtcagtg tcacaaaacc atttatgtta ccccctgttg cagccagctc ccttaggaat     780
gacagcagta gcagtaatag gaaggccaaa aatccccctg gagactccag cctacactgg     840
gcagccatgg cattgccagc attgttttct cttataattg gctttgcttt tggagcctta     900
tactggaaga agagacagcc aagtcttaca agggcagttg aaaatataca aattaatgaa     960
gaggataatg agataagtat gttgcaagag aaagagagag agtttcaaga agtgtaattg    1020
tggcttgtat caacactgtt actttcgtac attggctggt aacagttcat gtttgcttca    1080
taaatgaagc agctttaaac aaattcatat tctgtctgga gtgacagacc acatctttat    1140
ctgttcttgc tacccatgac tttatatgga tgattcagaa attggaacag aatgttttac    1200
tgtgaaactg gcactgaatt aatcatctat aaagaagaac ttgcatggag caggactcta    1260
ttttaaggac tgcgggactt gggtctcatt tagaacttgc agctgatgtt ggaagagaaa    1320
gcacgtgtct cagactgcat gtaccatttg catggctcca gaaatgtcta aatgctgaaa    1380
aaacacctag ctttattctt cagatacaaa ctgcagcctg tagttatcct ggtctctgca    1440
agtagatttc agcttggata gtgagggtaa caatttttct caaagggatc tggaaaaaat    1500
gtttaaaact cagtagtgtc agccactgta cagtgtagaa agcagtggga actgtgattg    1560
gatttggcaa catgtcagct ttatagttgc cgattagtga tatgggtctg atttcgatct    1620
cttcctgatg taaaccatgc tcacccatat cccactatac aaatgcaaat ggttgcctgg    1680
ttccatttat gcaagggagc cagtactgaa ttatgccttg gcagagggga gactccaaaa    1740
gagtcatcgc aggaagaagt taagaacact gaacatcaga acagtctgcc aagaaggaca    1800
ttggcatcct gggaaagtcc gccttttccc ttgaccacta tagggtgtat aaatcgtgtt    1860
```

tgcaaaatgt gttatgatgt gtttatattc taaaactatt acagagctat gtaaagggac 1920
ttaggagaaa atgctgaatg taagatggtc ccatttcaat ttccaccatg ggagagccta 1980
aaaataaatt atgacattta gtatctaagg ttagaaaacc acgcccacat gctaatatgg 2040
gtgttgaaaa ctaggttact tataatgcaa ggaatcagga aactttagtt atttatagta 2100
taatcaccat tatctgttta aaggatccat ttagttaaaa tcgggcactc tatattcatt 2160
aaggtttatg aattaaaaag aaagctttat gtagttatgc atgtcagttt gctatttaaa 2220
atgtgtgaca gtgtttgtca tattaagagt gaatttggca ggaattccca agatggacat 2280
tgtgctttta aactagaact tgtaagacat tatgtgaata tcccttgcca attttttta 2340
taataagaaa acatctgact aaagtcaaag aatgatttct tatggtttat tttgatgaaa 2400
gttcttttaa catgtcttga atgtacacat aaaggaatcc aaagctttcc attctaactt 2460
aatctttgtg ataacattat tgccatgttc tacaaccgta agatgacagt tttcaatgta 2520
gtgacacaaa agggcatgaa aaactaactg ctagctttcc tttcatttca aaagtccaag 2580
aatttctagt atatttggat tttagcttct gttcaaagca aatccagatg caactccagt 2640
aagtggcctt tgctcttttt tgtaccaaag agcccagatg attcctacag tccctttctt 2700
ctctaacatg ctgtggttcc ttaaatatga gtaatttctc taagatataa cccaggtgct 2760
ttgagaagct gcattaaggt gttcaggccc tcagatatca catggtacac ttgattagta 2820
ataaaaccag agatcaattt aaattgctga taggtcctgt ctcagtgtgt ggcattgact 2880
gttttcagga aaatagatac agattaatat gagttatgcg tgtaggttgt gtatagattg 2940
agaagataga tacttctcaa tctagtagtt tgatttattt aaccaatggt ttcagtttgc 3000
ttgagcatat gaaaatcctg cttaatgtgc ttaagagtat aataaatgtg tacttttgtc 3060
ctcaaaccta gtagctgggt tttaacactc atggacatgg tcttaatcaa tggagttaaa 3120
taaacaaatt cagcaagtta ttaaatctga catggtagga gaggggagat gtgtcctgct 3180
tattaaatgt gttggtccat tgaaagttac atggattgcc aatttttaaa acactaaagt 3240
tgaataaaat gcatgaacaa tagaaaaatg ctgaacatta ttttggatgc tagctgcttg 3300
gacattaact gtgttatttc tgctttgaga tgaaaatata tatttatctt tgcttatttt 3360
atcccagatg tgttctgaat atccttcttc ataaatcatg gaaaactcac tgctgagata 3420
gtaaaccatg aaatcgcctt ttcagttggt gccatgtatc tgacagttcc atcttggaag 3480
gtttcaaaat taccttttaa aatgatctca gaagtctgta gattctcaat gatactgaaa 3540
gctttgcacc tctttggtag aaaccaggtc tatttagaaa atggctttat gataaatgtt 3600
gcctcctgag tgataatgaa gtgttcctgg atattgtatt gtaatttaat gtgcttacca 3660
cactgccaca ttttaatgag tcagagaaaa attaattttt cttcaataca ataatagaac 3720

```
aagtagccta ttctcttaaa aagtatgtga aaagaaaatt atgaaaaaat atgcatacct     3780
aatgaagtat tggttttagt aagaattaaa tacatttcat tgagctttaa agtactttgg     3840
agaaactttg gggcacgttt tcctactcta attcaactaa agttataaat aaagagaaaa     3900
actcattcag aaatcatgga ttttaaaaat attttactgc agccaagttt tcatttcaaa     3960
atgtaatttc agtttggagc ttttaggcat tatgtatatt taaaaaatat attcttcaaa     4020
aatgcatttt ggcatggtgg gatggatgtt gcaaaagata tccggagcct ccagtctgtc     4080
attaactgat atggtaaatc acctctcttc tttgggtctc aatttttat ttatctatat      4140
ggtaaactca gagatcactc cttaggggtg agtcctattg caatatgacc gacaaagaag     4200
acaaaatagc attgaaacta acccatacaa aatatccaac tctggattct gtgaataagt     4260
atcttgacca taaaaagtca ttgctgttct tgtttctaat gtaaatagtg tccattagta     4320
aaagtgaaat tcagtcttaa gtagggtgaa ttggatcacc atttacacaa gagatggctt     4380
tttcctttgc ttgaataaac attttggatc acctccaaag aatgaaaacc agtagtacgt     4440
tttagtcata ttagtcagga tgagaaacta taagatgtgt gtaacatttg gaaatgcacc     4500
aaagtgagcg tttaaatctt ctcattttat tgaaaactaa gagcagaaaa tgtaaaatgc     4560
tcatgaaggt tttgaatgcc aaaagatatt ttagaatcaa tttataaagg ggtaattcat     4620
taattacact ttaaaattgg aaagtgggat aagaaatcta aagtaaacca gcttatcttt     4680
gaaacaatat tattttgaaa ttggctttaa aataaaacca ttcagattga aattctaatt     4740
agctcatttg tggagtttga tcacacaatt cataatgttg ctgctttcca ttaactagtc     4800
ttgaaatgcc tttgtttgta aaaataaaat aatggtactt tcattttata acaaggtgtt     4860
tttttcaaga aataatccat gctaaaatgg atatttgtga tcctgaaatg tttactaagc     4920
attgtaaatt tatttataac tgccatctcc aactacatcc ttatgatgtt tttaacaata     4980
aaattaaaac aactgttaaa ctaaaaacca caccgttttc cagtacttga tctctgagct     5040
acaatactca ctaaatataa ttttccaatc aaaatattct attctatatt ctaagggtta     5100
atatgtgatt atagtgtcca cttgccacca ttttttaaa tcaatggact tgaaaagtat      5160
taatttagat ggatgcgcag atataccctc agttcagtca tagattggag tttgcatata     5220
ataatgtaaa tgtatgtcga cactattcta aatagttcta ttatgactga aatttaatta     5280
aataaaaaag gttgtaaaat gtgatgtgta tgtgtatata ctgtatgtgt actttttaaa     5340
ataggtgtat gtcccaaccc ttttttatac aggtttgaat ttaaaattac atgatatata     5400
catatacttt attgttctaa ataaagaatt ttatgcactc tcaaaaaaaa aaaaaaaaaa     5460
```

<210> 8
<211> 208
<212> PRT
<213> Homo sapiens

<400> 8

Met Trp Lys Trp Ile Leu Thr His Cys Ala Ser Ala Phe Pro His Leu
1 5 10 15

Pro Gly Cys Cys Cys Cys Cys Phe Leu Leu Leu Phe Leu Val Ser Ser
20 25 30

Val Pro Val Thr Cys Gln Ala Leu Gly Gln Asp Met Val Ser Pro Glu
35 40 45

Ala Thr Asn Ser Ser Ser Ser Ser Phe Ser Ser Pro Ser Ser Ala Gly
50 55 60

Arg His Val Arg Ser Tyr Asn His Leu Gln Gly Asp Val Arg Trp Arg
65 70 75 80

Lys Leu Phe Ser Phe Thr Lys Tyr Phe Leu Lys Ile Glu Lys Asn Gly
85 90 95

Lys Val Ser Gly Thr Lys Lys Glu Asn Cys Pro Tyr Ser Ile Leu Glu
100 105 110

Ile Thr Ser Val Glu Ile Gly Val Val Ala Val Lys Ala Ile Asn Ser
115 120 125

Asn Tyr Tyr Leu Ala Met Asn Lys Lys Gly Lys Leu Tyr Gly Ser Lys
130 135 140

Glu Phe Asn Asn Asp Cys Lys Leu Lys Glu Arg Ile Glu Glu Asn Gly
145 150 155 160

Tyr Asn Thr Tyr Ala Ser Phe Asn Trp Gln His Asn Gly Arg Gln Met
165 170 175

Tyr Val Ala Leu Asn Gly Lys Gly Ala Pro Arg Arg Gly Gln Lys Thr
180 185 190

Arg Arg Lys Asn Thr Ser Ala His Phe Leu Pro Met Val Val His Ser
195 200 205

<210> 9
<211> 94
<212> PRT
<213> Homo sapiens

<400> 9

Met Ser Val Lys Gly Met Ala Ile Ala Leu Ala Val Ile Leu Cys Ala

```
1               5                   10                  15

Thr Val Val Gln Gly Phe Pro Met Phe Lys Arg Gly Arg Cys Leu Cys
            20                  25                  30

Ile Gly Pro Gly Val Lys Ala Val Lys Val Ala Asp Ile Glu Lys Ala
        35                  40                  45

Ser Ile Met Tyr Pro Ser Asn Asn Cys Asp Lys Ile Glu Val Ile Ile
    50                  55                  60

Thr Leu Lys Glu Asn Lys Gly Gln Arg Cys Leu Asn Pro Lys Ser Lys
65                  70                  75                  80

Gln Ala Arg Leu Ile Ile Lys Lys Val Glu Arg Lys Asn Phe
                85                  90

<210>  10
<211>  252
<212>  PRT
<213>  Homo sapiens

<400>  10

Met Gly Val Leu Leu Thr Gln Arg Thr Leu Leu Ser Leu Val Leu Ala
1               5                   10                  15

Leu Leu Phe Pro Ser Met Ala Ser Met Ala Ala Ile Gly Ser Cys Ser
            20                  25                  30

Lys Glu Tyr Arg Val Leu Leu Gly Gln Leu Gln Lys Gln Thr Asp Leu
        35                  40                  45

Met Gln Asp Thr Ser Arg Leu Leu Asp Pro Tyr Ile Arg Ile Gln Gly
    50                  55                  60

Leu Asp Val Pro Lys Leu Arg Glu His Cys Arg Glu Arg Pro Gly Ala
65                  70                  75                  80

Phe Pro Ser Glu Glu Thr Leu Arg Gly Leu Gly Arg Arg Gly Phe Leu
                85                  90                  95

Gln Thr Leu Asn Ala Thr Leu Gly Cys Val Leu His Arg Leu Ala Asp
            100                 105                 110

Leu Glu Gln Arg Leu Pro Lys Ala Gln Asp Leu Glu Arg Ser Gly Leu
        115                 120                 125

Asn Ile Glu Asp Leu Glu Lys Leu Gln Met Ala Arg Pro Asn Ile Leu
```

    130                 135                 140

Gly Leu Arg Asn Asn Ile Tyr Cys Met Ala Gln Leu Leu Asp Asn Ser
145                 150                 155                 160

Asp Thr Ala Glu Pro Thr Lys Ala Gly Arg Gly Ala Ser Gln Pro Pro
                165                 170                 175

Thr Pro Thr Pro Ala Ser Asp Ala Phe Gln Arg Lys Leu Glu Gly Cys
            180                 185                 190

Arg Phe Leu His Gly Tyr His Arg Phe Met His Ser Val Gly Arg Val
        195                 200                 205

Phe Ser Lys Trp Gly Glu Ser Pro Asn Arg Ser Arg Arg His Ser Pro
    210                 215                 220

His Gln Ala Leu Arg Lys Gly Val Arg Arg Thr Arg Pro Ser Arg Lys
225                 230                 235                 240

Gly Lys Arg Leu Met Thr Arg Gly Gln Leu Pro Arg
                245                 250

<210> 11
<211> 572
<212> PRT
<213> Homo sapiens

<400> 11

Met Glu Cys Leu Tyr Tyr Phe Leu Gly Phe Leu Leu Leu Ala Ala Arg
1               5                   10                  15

Leu Pro Leu Asp Ala Ala Lys Arg Phe His Asp Val Leu Gly Asn Glu
            20                  25                  30

Arg Pro Ser Ala Tyr Met Arg Glu His Asn Gln Leu Asn Gly Trp Ser
        35                  40                  45

Ser Asp Glu Asn Asp Trp Asn Glu Lys Leu Tyr Pro Val Trp Lys Arg
    50                  55                  60

Gly Asp Met Arg Trp Lys Asn Ser Trp Lys Gly Gly Arg Val Gln Ala
65                  70                  75                  80

Val Leu Thr Ser Asp Ser Pro Ala Leu Val Gly Ser Asn Ile Thr Phe
                85                  90                  95

Ala Val Asn Leu Ile Phe Pro Arg Cys Gln Lys Glu Asp Ala Asn Gly

```
            100                 105                 110

Asn Ile Val Tyr Glu Lys Asn Cys Arg Asn Glu Ala Gly Leu Ser Ala
        115                 120                 125

Asp Pro Tyr Val Tyr Asn Trp Thr Ala Trp Ser Glu Asp Ser Asp Gly
    130                 135                 140

Glu Asn Gly Thr Gly Gln Ser His His Asn Val Phe Pro Asp Gly Lys
145                 150                 155                 160

Pro Phe Pro His His Pro Gly Trp Arg Arg Trp Asn Phe Ile Tyr Val
                165                 170                 175

Phe His Thr Leu Gly Gln Tyr Phe Gln Lys Leu Gly Arg Cys Ser Val
            180                 185                 190

Arg Val Ser Val Asn Thr Ala Asn Val Thr Leu Gly Pro Gln Leu Met
        195                 200                 205

Glu Val Thr Val Tyr Arg Arg His Gly Arg Ala Tyr Val Pro Ile Ala
    210                 215                 220

Gln Val Lys Asp Val Tyr Val Val Thr Asp Gln Ile Pro Val Phe Val
225                 230                 235                 240

Thr Met Phe Gln Lys Asn Asp Arg Asn Ser Ser Asp Glu Thr Phe Leu
                245                 250                 255

Lys Asp Leu Pro Ile Met Phe Asp Val Leu Ile His Asp Pro Ser His
            260                 265                 270

Phe Leu Asn Tyr Ser Thr Ile Asn Tyr Lys Trp Ser Phe Gly Asp Asn
        275                 280                 285

Thr Gly Leu Phe Val Ser Thr Asn His Thr Val Asn His Thr Tyr Val
    290                 295                 300

Leu Asn Gly Thr Phe Ser Leu Asn Leu Thr Val Lys Ala Ala Ala Pro
305                 310                 315                 320

Gly Pro Cys Pro Pro Pro Pro Pro Pro Pro Arg Pro Ser Lys Pro Thr
                325                 330                 335

Pro Ser Leu Ala Thr Thr Leu Lys Ser Tyr Asp Ser Asn Thr Pro Gly
            340                 345                 350
```

```
Pro Ala Gly Asp Asn Pro Leu Glu Leu Ser Arg Ile Pro Asp Glu Asn
        355                 360                 365

Cys Gln Ile Asn Arg Tyr Gly His Phe Gln Ala Thr Ile Thr Ile Val
    370                 375                 380

Glu Gly Ile Leu Glu Val Asn Ile Ile Gln Met Thr Asp Val Leu Met
385                 390                 395                 400

Pro Val Pro Trp Pro Glu Ser Ser Leu Ile Asp Phe Val Val Thr Cys
                405                 410                 415

Gln Gly Ser Ile Pro Thr Glu Val Cys Thr Ile Ile Ser Asp Pro Thr
            420                 425                 430

Cys Glu Ile Thr Gln Asn Thr Val Cys Ser Pro Val Asp Val Asp Glu
        435                 440                 445

Met Cys Leu Leu Thr Val Arg Arg Thr Phe Asn Gly Ser Gly Thr Tyr
    450                 455                 460

Cys Val Asn Leu Thr Leu Gly Asp Asp Thr Ser Leu Ala Leu Thr Ser
465                 470                 475                 480

Thr Leu Ile Ser Val Pro Asp Arg Asp Pro Ala Ser Pro Leu Arg Met
                485                 490                 495

Ala Asn Ser Ala Leu Ile Ser Val Gly Cys Leu Ala Ile Phe Val Thr
            500                 505                 510

Val Ile Ser Leu Leu Val Tyr Lys Lys His Lys Glu Tyr Asn Pro Ile
        515                 520                 525

Glu Asn Ser Pro Gly Asn Val Val Arg Ser Lys Gly Leu Ser Val Phe
    530                 535                 540

Leu Asn Arg Ala Lys Ala Val Phe Phe Pro Gly Asn Gln Glu Lys Asp
545                 550                 555                 560

Pro Leu Leu Lys Asn Gln Glu Phe Lys Gly Val Ser
                565                 570


<210>  12
<211>  560
<212>  PRT
<213>  Homo sapiens

<400>  12
```

```
Met Glu Cys Leu Tyr Tyr Phe Leu Gly Phe Leu Leu Leu Ala Ala Arg
1               5                   10                  15

Leu Pro Leu Asp Ala Ala Lys Arg Phe His Asp Val Leu Gly Asn Glu
            20                  25                  30

Arg Pro Ser Ala Tyr Met Arg Glu His Asn Gln Leu Asn Gly Trp Ser
        35                  40                  45

Ser Asp Glu Asn Asp Trp Asn Glu Lys Leu Tyr Pro Val Trp Lys Arg
    50                  55                  60

Gly Asp Met Arg Trp Lys Asn Ser Trp Lys Gly Gly Arg Val Gln Ala
65                  70                  75                  80

Val Leu Thr Ser Asp Ser Pro Ala Leu Val Gly Ser Asn Ile Thr Phe
                85                  90                  95

Ala Val Asn Leu Ile Phe Pro Arg Cys Gln Lys Glu Asp Ala Asn Gly
            100                 105                 110

Asn Ile Val Tyr Glu Lys Asn Cys Arg Asn Glu Ala Gly Leu Ser Ala
        115                 120                 125

Asp Pro Tyr Val Tyr Asn Trp Thr Ala Trp Ser Glu Asp Ser Asp Gly
    130                 135                 140

Glu Asn Gly Thr Gly Gln Ser His His Asn Val Phe Pro Asp Gly Lys
145                 150                 155                 160

Pro Phe Pro His His Pro Gly Trp Arg Arg Trp Asn Phe Ile Tyr Val
                165                 170                 175

Phe His Thr Leu Gly Gln Tyr Phe Gln Lys Leu Gly Arg Cys Ser Val
            180                 185                 190

Arg Val Ser Val Asn Thr Ala Asn Val Thr Leu Gly Pro Gln Leu Met
        195                 200                 205

Glu Val Thr Val Tyr Arg Arg His Gly Arg Ala Tyr Val Pro Ile Ala
    210                 215                 220

Gln Val Lys Asp Val Tyr Val Val Thr Asp Gln Ile Pro Val Phe Val
225                 230                 235                 240

Thr Met Phe Gln Lys Asn Asp Arg Asn Ser Ser Asp Glu Thr Phe Leu
                245                 250                 255
```

```
Lys Asp Leu Pro Ile Met Phe Asp Val Leu Ile His Asp Pro Ser His
            260                 265                 270

Phe Leu Asn Tyr Ser Thr Ile Asn Tyr Lys Trp Ser Phe Gly Asp Asn
        275                 280                 285

Thr Gly Leu Phe Val Ser Thr Asn His Thr Val Asn His Thr Tyr Val
    290                 295                 300

Leu Asn Gly Thr Phe Ser Leu Asn Leu Thr Val Lys Ala Ala Ala Pro
305                 310                 315                 320

Gly Pro Cys Pro Pro Pro Pro Pro Pro Pro Arg Pro Ser Lys Pro Thr
                325                 330                 335

Pro Ser Leu Gly Pro Ala Gly Asp Asn Pro Leu Glu Leu Ser Arg Ile
            340                 345                 350

Pro Asp Glu Asn Cys Gln Ile Asn Arg Tyr Gly His Phe Gln Ala Thr
        355                 360                 365

Ile Thr Ile Val Glu Gly Ile Leu Glu Val Asn Ile Ile Gln Met Thr
    370                 375                 380

Asp Val Leu Met Pro Val Pro Trp Pro Glu Ser Ser Leu Ile Asp Phe
385                 390                 395                 400

Val Val Thr Cys Gln Gly Ser Ile Pro Thr Glu Val Cys Thr Ile Ile
                405                 410                 415

Ser Asp Pro Thr Cys Glu Ile Thr Gln Asn Thr Val Cys Ser Pro Val
            420                 425                 430

Asp Val Asp Glu Met Cys Leu Leu Thr Val Arg Arg Thr Phe Asn Gly
        435                 440                 445

Ser Gly Thr Tyr Cys Val Asn Leu Thr Leu Gly Asp Asp Thr Ser Leu
    450                 455                 460

Ala Leu Thr Ser Thr Leu Ile Ser Val Pro Asp Arg Asp Pro Ala Ser
465                 470                 475                 480

Pro Leu Arg Met Ala Asn Ser Ala Leu Ile Ser Val Gly Cys Leu Ala
                485                 490                 495

Ile Phe Val Thr Val Ile Ser Leu Leu Val Tyr Lys Lys His Lys Glu
            500                 505                 510
```

Tyr Asn Pro Ile Glu Asn Ser Pro Gly Asn Val Val Arg Ser Lys Gly
515 520 525

Leu Ser Val Phe Leu Asn Arg Ala Lys Ala Val Phe Phe Pro Gly Asn
530 535 540

Gln Glu Lys Asp Pro Leu Leu Lys Asn Gln Glu Phe Lys Gly Val Ser
545 550 555 560

<210> 13
<211> 245
<212> PRT
<213> Homo sapiens

<400> 13

Met Lys Lys Thr Gln Thr Trp Ile Leu Thr Cys Ile Tyr Leu Gln Leu
1 5 10 15

Leu Leu Phe Asn Pro Leu Val Lys Thr Glu Gly Ile Cys Arg Asn Arg
20 25 30

Val Thr Asn Asn Val Lys Asp Val Thr Lys Leu Val Ala Asn Leu Pro
35 40 45

Lys Asp Tyr Met Ile Thr Leu Lys Tyr Val Pro Gly Met Asp Val Leu
50 55 60

Pro Ser His Cys Trp Ile Ser Glu Met Val Val Gln Leu Ser Asp Ser
65 70 75 80

Leu Thr Asp Leu Leu Asp Lys Phe Ser Asn Ile Ser Glu Gly Leu Ser
85 90 95

Asn Tyr Ser Ile Ile Asp Lys Leu Val Asn Ile Val Asp Asp Leu Val
100 105 110

Glu Cys Val Lys Glu Asn Ser Ser Lys Asp Leu Lys Lys Ser Phe Lys
115 120 125

Ser Pro Glu Pro Arg Leu Phe Thr Pro Glu Glu Phe Phe Arg Ile Phe
130 135 140

Asn Arg Ser Ile Asp Ala Phe Lys Asp Phe Val Val Ala Ser Glu Thr
145 150 155 160

Ser Asp Cys Val Val Ser Ser Thr Leu Ser Pro Glu Lys Gly Lys Ala
165 170 175

```
Lys Asn Pro Pro Gly Asp Ser Ser Leu His Trp Ala Ala Met Ala Leu
            180                 185                 190

Pro Ala Leu Phe Ser Leu Ile Ile Gly Phe Ala Phe Gly Ala Leu Tyr
        195                 200                 205

Trp Lys Lys Arg Gln Pro Ser Leu Thr Arg Ala Val Glu Asn Ile Gln
    210                 215                 220

Ile Asn Glu Glu Asp Asn Glu Ile Ser Met Leu Gln Glu Lys Glu Arg
225                 230                 235                 240

Glu Phe Gln Glu Val
                245


<210>  14
<211>  273
<212>  PRT
<213>  Homo sapiens

<400>  14

Met Lys Lys Thr Gln Thr Trp Ile Leu Thr Cys Ile Tyr Leu Gln Leu
1               5                   10                  15

Leu Leu Phe Asn Pro Leu Val Lys Thr Glu Gly Ile Cys Arg Asn Arg
            20                  25                  30

Val Thr Asn Asn Val Lys Asp Val Thr Lys Leu Val Ala Asn Leu Pro
        35                  40                  45

Lys Asp Tyr Met Ile Thr Leu Lys Tyr Val Pro Gly Met Asp Val Leu
    50                  55                  60

Pro Ser His Cys Trp Ile Ser Glu Met Val Val Gln Leu Ser Asp Ser
65                  70                  75                  80

Leu Thr Asp Leu Leu Asp Lys Phe Ser Asn Ile Ser Glu Gly Leu Ser
                85                  90                  95

Asn Tyr Ser Ile Ile Asp Lys Leu Val Asn Ile Val Asp Asp Leu Val
            100                 105                 110

Glu Cys Val Lys Glu Asn Ser Ser Lys Asp Leu Lys Lys Ser Phe Lys
        115                 120                 125

Ser Pro Glu Pro Arg Leu Phe Thr Pro Glu Glu Phe Phe Arg Ile Phe
    130                 135                 140
```

```
Asn Arg Ser Ile Asp Ala Phe Lys Asp Phe Val Val Ala Ser Glu Thr
145                 150                 155                 160

Ser Asp Cys Val Val Ser Ser Thr Leu Ser Pro Glu Lys Asp Ser Arg
                165                 170                 175

Val Ser Val Thr Lys Pro Phe Met Leu Pro Pro Val Ala Ala Ser Ser
            180                 185                 190

Leu Arg Asn Asp Ser Ser Ser Ser Asn Arg Lys Ala Lys Asn Pro Pro
        195                 200                 205

Gly Asp Ser Ser Leu His Trp Ala Ala Met Ala Leu Pro Ala Leu Phe
    210                 215                 220

Ser Leu Ile Ile Gly Phe Ala Phe Gly Ala Leu Tyr Trp Lys Lys Arg
225                 230                 235                 240

Gln Pro Ser Leu Thr Arg Ala Val Glu Asn Ile Gln Ile Asn Glu Glu
                245                 250                 255

Asp Asn Glu Ile Ser Met Leu Gln Glu Lys Glu Arg Glu Phe Gln Glu
            260                 265                 270

Val
```

<210> 15
<211> 2757
<212> DNA
<213> Homo sapiens

<400> 15

```
gacaagtact gagtgaactc aaaccctctg taaagtaaca gaagttagaa ggggaaatgt      60
cgcctctctg aagattaccc aaagaaaaag tgatttgtca ttgctttata gactgtaaga     120
agagaacatc tcagaagtgg agtcttaccc tgaaatcaaa ggatttaaag aaaaagtgga     180
atttttcttc agcaagctgt gaaactaaat ccacaacctt tggagaccca ggaacaccct     240
ccaatctctg tgtgttttgt aaacatcact ggagggtctt ctacgtgagc aattggattg     300
tcatcagccc tgcctgtttt gcacctggga agtgccctgg tcttacttgg gtccaaattg     360
ttggctttca cttttgaccc taagcatctg aagccatggg ccacacacgg aggcagggaa     420
catcaccatc caagtgtcca tacctcaatt tctttcagct cttggtgctg gctggtcttt     480
ctcacttctg ttcaggtgtt atccacgtga ccaaggaagt gaaagaagtg gcaacgctgt     540
cctgtggtca caatgtttct gttgaagagc tggcacaaac tcgcatctac tggcaaaagg     600
```

agaagaaaat ggtgctgact atgatgtctg gggacatgaa tatatggccc gagtacaaga 660
accggaccat ctttgatatc actaataacc tctccattgt gatcctggct ctgcgcccat 720
ctgacgaggg cacatacgag tgtgttgttc tgaagtatga aaaagacgct ttcaagcggg 780
aacacctggc tgaagtgacg ttatcagtca aagctgactt ccctacacct agtatatctg 840
actttgaaat tccaacttct aatattagaa ggataatttg ctcaacctct ggaggttttc 900
cagagcctca cctctcctgg ttggaaaatg gagaagaatt aaatgccatc aacacaacag 960
tttcccaaga tcctgaaact gagctctatg ctgttagcag caaactggat ttcaatatga 1020
caaccaacca cagcttcatg tgtctcatca agtatggaca tttaagagtg aatcagacct 1080
tcaactggaa tacaaccaag caagagcatt ttcctgataa cctgctccca tcctgggcca 1140
ttaccttaat ctcagtaaat ggaatttttg tgatatgctg cctgacctac tgctttgccc 1200
caagatgcag agagagaagg aggaatgaga gattgagaag ggaaagtgta cgccctgtat 1260
aacagtgtcc gcagaagcaa ggggctgaaa agatctgaag gtcccacctc catttgcaat 1320
tgacctcttc tgggaacttc ctcagatgga caagattacc ccaccttgcc ctttacgtat 1380
ctgctcttag gtgcttcttc acttcagttg ctttgcagga agtgtctaga ggaatatggt 1440
gggcacagaa gtagctctgg tgaccttgat caaggtgttt tgaaatgcag aattcttgag 1500
ttctggaagg gactttagag aataccagtg ttattaatga caaaggcact gaggcccagg 1560
gaggtgaccc gaattataaa ggccagcgcc agaacccaga tttcctaact ctggtgctct 1620
ttccctttat cagtttgact gtggcctgtt aactggtata tacatatata tgtcaggcaa 1680
agtgctgctg gaagtagaat ttgtccaata acaggtcaac ttcagagact atctgatttc 1740
ctaatgtcag agtagaagat tttatgctgc tgtttacaaa agcccaatgt aatgcatagg 1800
aagtatggca tgaacatctt taggagacta atggaaatat tattggtgtt tacccagtat 1860
tccatttttt tcattgtgtt ctctattgct gctctctcac tcccccatga ggtacagcag 1920
aaaggagaac tatccaaaac taatttcctc tgacatgtaa gacgaatgat ttaggtacgt 1980
caaagcagta gtcaaggagg aaagggatag tccaaagact taactggttc atattggact 2040
gataatctct ttaaatggct ttatgctagt ttgacctcat ttgtaaaata tttatgagaa 2100
agttctcatt taaaatgaga tcgttgttta cagtgtatgt actaagcagt aagctatctt 2160
caaatgtcta aggtagtaac tttccatagg gcctccttag atccctaaga tggctttttc 2220
tccttggtat ttctgggtct ttctgacatc agcagagaac tggaaagaca tagccaactg 2280
ctgttcatgt tactcatgac tcctttctct aaaactgcct tccacaattc actagaccag 2340
aagtggacgc aacttaagct gggataatca cattatcatc tgaaaatctg gagttgaaca 2400
gcaaaagaag acaacatttc tcaaatgcac atctcatggc agctaagcca catggctggg 2460
atttaaagcc tttagagcca gcccatggct ttagctacct cactatgctg cttcacaaac 2520

cttgctcctg tgtaaaacta tattctcagt gtagggcaga gaggtctaac accaacataa 2580

ggtactagca gtgtttcccg tattgacagg aatacttaac tcaataattc ttttcttttc 2640

catttagtaa cagttgtgat gactatgttt ctattctaag taattcctgt attctacagc 2700

agatactttg tcagcaatac taagggaaga aacaaagttg aaccgtttct ttaataa 2757

<210> 16
<211> 1372
<212> DNA
<213> Homo sapiens

<400> 16
gaagcgagag gcatctaagc aggcagtgtt ttgccttcac cccaagtgac catgagaggt 60

gccacgcgag tctcaatcat gctcctccta gtaactgtgt ctgactgtgc tgtgatcaca 120

ggggcctgtg agcgggatgt ccagtgtggg gcaggcacct gctgtgccat cagcctgtgg 180

cttcgagggc tgcggatgtg caccccgctg gggcgggaag gcgaggagtg ccaccccggc 240

agccacaagg tccccttctt caggaaacgc aagcaccaca cctgtccttg cttgcccaac 300

ctgctgtgct ccaggttccc ggacggcagg taccgctgct ccatggactt gaagaacatc 360

aatttttagg cgcttgcctg gtctcaggat acccaccatc cttttcctga gcacagcctg 420

gatttttatt tctgccatga aacccagctc ccatgactct cccagtccct acactgacta 480

ccctgatctc tcttgtctag tacgcacata tgcacacagg cagacatacc tcccatcatg 540

acatggtccc caggctggcc tgaggatgtc acagcttgag gctgtggtgt gaaaggtggc 600

cagcctggtt ctcttccctg ctcaggctgc cagagaggtg gtaaatggca gaaaggacat 660

tccccctccc ctccccaggt gacctgctct ctttcctggg ccctgcccct ctccccacat 720

gtatccctcg gtctgaatta gacattcctg ggcacaggct cttgggtgca ttgctcagag 780

tcccaggtcc tggcctgacc ctcaggccct tcacgtgagg tctgtgagga ccaatttgtg 840

ggtagttcat cttccctcga ttggttaact ccttagtttc agaccacaga ctcaagattg 900

gctcttccca gagggcagca gacagtcacc ccaaggcagg tgtagggagc ccagggaggc 960

caatcagccc cctgaagact ctggtcccag tcagcctgtg gcttgtggcc tgtgacctgt 1020

gaccttctgc cagaattgtc atgcctctga ggccccctct taccacactt taccagttaa 1080

ccactgaagc ccccaattcc cacagctttt ccattaaaat gcaaatggtg gtggttcaat 1140

ctaatctgat attgacatat tagaaggcaa ttagggtgtt tccttaaaca actcctttcc 1200

aaggatcagc cctgagagca ggttggtgac tttgaggagg gcagtcctct gtccagattg 1260

gggtgggagc aagggacagg gagcagggca ggggctgaaa ggggcactga ttcagaccag 1320

ggaggcaact acacaccaac ctgctggctt tagaataaaa gcaccaactg aa 1372

<210> 17
<211> 856
<212> DNA
<213> Homo sapiens

<400> 17
aattaccttt tcactttaca cacatcatct tggattgccc attttgcgtg gctaaaaagc 60

agagccatgc cgctggggaa gcagttgcga tttagccatg gctgcagctt ggaccgtggt 120

gctggtgact ttggtgctag gcttggccgt ggcaggccct gtccccactt ccaagcccac 180

cacaactggg aagggctgcc acattggcag gttcaaatct ctgtcaccac aggagctagc 240

gagcttcaag aaggccaggg acgccttgga agagtcactc aagctgaaaa actggagttg 300

cagctctcct gtcttccccg ggaattggga cctgaggctt ctccaggtga gggagcgccc 360

tgtggccttg gaggctgagc tggccctgac gctgaaggtc ctggaggccg ctgctggccc 420

agccctggag gacgtcctag accagcccct tcacaccctg caccacatcc tctcccagct 480

ccaggcctgt atccagcctc agcccacagc agggcccagg ccccggggcc gcctccacca 540

ctggctgcac cggctccagg aggcccccaa aaaggagtcc gctggctgcc tggaggcatc 600

tgtcaccttc aacctcttcc gcctcctcac gcgagacctc aaatatgtgg ccgatgggaa 660

cctgtgtctg agaacgtcaa cccaccctga gtccacctga caccccacac cttatttatg 720

cgctgagccc tactccttcc ttaatttatt tcctctcacc ctttatttat gaagctgcag 780

ccctgactga gacatagggc tgagtttatt gttttacttt tatacattat gcacaaataa 840

acaacaagga attgga 856

<210> 18
<211> 2553
<212> DNA
<213> Homo sapiens

<400> 18
gcactcgggg cgacagagag ggaggaggcg cgcggggacg gggacgccca ggaggaccca 60

ctcgcgggtc ccgctccgct ccggcagcag catggggaaa ggacgcgcgg gccgagttgg 120

caccacagcc ttgcctcccc gattgaaaga gatgaaaagc caggaatcgg ctgcaggttc 180

caaactagtc cttcggtgtg aaaccagttc tgaatactcc tctctcagat tcaagtggtt 240

caagaatggg aatgaattga atcgaaaaaa caaaccacaa aatatcaaga tacaaaaaaa 300

gccagggaag tcagaacttc gcattaacaa agcatcactg gctgattctg gagagtatat 360

gtgcaaagtg atcagcaaat taggaaatga cagtgcctct gccaatatca ccatcgtgga 420

atcaaacgag atcatcactg gtatgccagc ctcaactgaa ggagcatatg tgtcttcagc 480

tacatctaca tccaccactg ggacaagcca tcttgtaaaa tgtgcggaga aggagaaaac 540

tttctgtgtg aatggagggg agtgcttcat ggtgaaagac ctttcaaacc cctcgagata 600

cttgtgcaag tgcccaaatg agtttactgg tgatcgctgc caaaactacg taatggccag 660

cttctacaag catcttggga ttgaatttat ggaggcggag gagctgtacc agaagagagt 720

gctgaccata accggcatct gcatcgccct ccttgtggtc ggcatcatgt gtgtggtggc 780

ctactgcaaa accaagaaac agcggaaaaa gctgcatgac cgtcttcggc agagccttcg 840

gtctgaacga aacaatatga tgaacattgc caatgggcct caccatccta acccaccccc 900

cgagaatgtc cagctggtga atcaatacgt atctaaaaac gtcatctcca gtgagcatat 960

tgttgagaga gaagcagaga catccttttc caccagtcac tatacttcca cagcccatca 1020

ctccactact gtcacccaga ctcctagcca cagctggagc aacggacaca ctgaaagcat 1080

cctttccgaa agccactctg taatcgtgat gtcatccgta gaaaacagta ggcacagcag 1140

cccaactggg ggcccaagag gacgtcttaa tggcacagga ggccctcgtg aatgtaacag 1200

cttcctcagg catgccagag aaacccctga ttcctaccga gactctcctc atagtgaaag 1260

gtatgtgtca gccatgacca ccccggctcg tatgtcacct gtagatttcc acacgccaag 1320

ctcccccaaa tcgccccctt cggaaatgtc tccacccgtg tccagcatga cggtgtccat 1380

gccttccatg gcggtcagcc ccttcatgga agaagagaga cctctacttc tcgtgacacc 1440

accaaggctg cgggagaaga agtttgacca tcaccctcag cagttcagct ccttccacca 1500

caaccccgcg catgacagta acagcctccc tgctagcccc ttgaggatag tggaggatga 1560

ggagtatgaa acgacccaag agtacgagcc agcccaagag cctgttaaga aactcgccaa 1620

tagccggcgg gccaaaagaa ccaagcccaa tggccacatt gctaacagat tggaagtgga 1680

cagcaacaca agctcccaga gcagtaactc agagagtgaa acagaagatg aaagagtagg 1740

tgaagatacg cctttcctgg gcatacagaa ccccctggca gccagtcttg aggcaacacc 1800

tgccttccgc ctggctgaca gcaggactaa cccagcaggc cgcttctcga cacaggaaga 1860

aatccaggcc aggctgtcta gtgtaattgc taaccaagac cctattgctg tataaaacct 1920

aaataaacac atagattcac ctgtaaaact ttattttata taataaagta ttccacctta 1980

aattaaacaa tttattttat tttagcagtt ctgcaaatag aaaacaggaa aaaaactttt 2040

ataaattaaa tatatgtatg taaaaatgtg ttatgtgcca tatgtagcaa ttttttacag 2100

tatttcaaaa cgagaaagat atcaatggtg cctttatgtt atgttatgtc gagagcaagt 2160

tttgtacagt tacagtgatt gcttttccac agtatttctg caaaacctct catagattca 2220

gtttttgctg gcttcttgtg cattgcatta tgatgttgac tggatgtatg atttgcaaga 2280

cttgcaactg tccctctgtt tgcttgtagt agcacccgat cagtatgtct tgtaatggca 2340

catccatcca gatatgcctc tcttgtgtat gaagttttct ttgctttcag aatatgaaat 2400

gagttgtgtc tactctgcca gccaaaggtt tgcctcattg ggctctgaga taatagtaga 2460

tccaacagca tgctactatt aaatacagca agaaactgca ttaagtaatg ttaaatatta 2520

ggaagaaagt aatactgtga tttaaaaaaa act 2553

<210> 19
<211> 1667
<212> DNA
<213> Homo sapiens

<400> 19
cgactgccga gctccgccct ccaggcggcc ccacccgcct gccgtcctgg ggcgccgccg 60

ccccgccgcc ggcagtggac cgctgtgcgc gaaccctgaa ccctacggtc ccgacccgcg 120

ggcgaggccg ggtacctggg ctgggatccg gagcaagcgg gcgagggcag cgccctaagc 180

aggcccggag cgatggcagc cttgatgacc ccgggaaccg gggccccacc cgcgcctggt 240

gacttctccg gggaagggag ccagggactt cccgaccctt cgccagagcc caagcagctc 300

ccggagctga tccgcatgaa gcgagacgga ggccgcctga gcgaagcgga catcaggggc 360

ttcgtggccg ctgtggtgaa tgggagcgcg cagggcgcac agatcggggc catgctgatg 420

gccatccgac ttcggggcat ggatctggag gagacctcgg tgctgaccca ggccctggct 480

cagtcgggac agcagctgga gtggccagag gcctggcgcc agcagcttgt ggacaagcat 540

tccacagggg gtgtgggtga caaggtcagc ctggtcctcg cacctgccct ggcggcatgt 600

ggctgcaagg tgccaatgat cagcggacgt ggtctggggc acacaggagg caccttggat 660

aagctggagt ctattcctgg attcaatgtc atccagagcc cagagcagat gcaagtgctg 720

ctggaccagg cgggctgctg tatcgtgggt cagagtgagc agctggttcc tgcggacgga 780

atcctatatg cagccagaga tgtgacagcc accgtggaca gcctgccact catcacagcc 840

tccattctca gtaagaaact cgtggagggg ctgtccgctc tggtggtgga cgttaagttc 900

ggaggggccg ccgtcttccc caaccaggag caggcccggg agctggcaaa gacgctggtt 960

ggcgtgggag ccagcctagg gcttcgggtc gcggcagcgc tgaccgccat ggacaagccc 1020

ctgggtcgct gcgtgggcca cgccctggag gtggaggagg cgctgctctg catggacggc 1080

gcaggcccgc cagacttaag ggacctggtc accacgctcg gggcgccct gctctggctc 1140

agcggacacg cggggactca ggcccagggc gctgcccggg tggccgcggc gctggacgac 1200

ggctcggccc ttggccgctt cgagcggatg ctggcggcgc agggcgtgga tcccggtctg 1260

gcccgagccc tgtgctcggg aagtcccgca gaacgccggc agctgctgcc tcgcgcccgg 1320

gagcaggagg agctgctggc gcccgcagat ggcaccgtgg agctggtccg ggcgctgccg 1380

ctggcgctgg tgctgcacga gctcggggcc gggcgcagcc gcgctgggga gccgctccgc 1440

ctgggggtgg gcgcagagct gctggtcgac gtgggtcaga ggctgcgccg tgggaccccc 1500

tggctccgcg tgcaccggga cggccccgcg ctcagcggcc cgcagagccg cgccctgcag 1560

gaggcgctcg tactctccga ccgcgcgcca ttcgccgccc cctcgccctt cgcagagctc 1620

gttctgccgc cgcagcaata aagctccttt gccgcgaaaa aaaaaaa 1667

<210> 20
<211> 1682
<212> DNA
<213> Homo sapiens

<400> 20
cgactgccga gctccgccct ccaggcggcc ccacccgcct gccgtcctgg ggcgccgccg 60

ccccgccgcc ggcagtggac cgctgtgcgc gaaccctgaa ccctacggtc ccgacccgcg 120

ggcgaggccg ggtacctggg ctgggatccg gagcaagcgg gcgagggcag cgccctaagc 180

aggcccggag cgatggcagc cttgatgacc ccgggaaccg gggccccacc cgcgcctggt 240

gacttctccg gggaagggag ccagggactt cccgaccctt cgccagagcc caagcagctc 300

ccggagctga tccgcatgaa gcgagacgga ggccgcctga gcgaagcgga catcaggggc 360

ttcgtggccg ctgtggtgaa tgggagcgcg cagggcgcac agatcggggc catgctgatg 420

gccatccgac ttcggggcat ggatctggag gagacctcgg tgctgaccca ggccctggct 480

cagtcgggac agcagctgga gtggccagag gcctggcgcc agcagcttgt ggacaagcat 540

tccacagggg gtgtgggtga caaggtcagc ctggtcctcg cacctgccct ggcggcatgt 600

ggctgcaagg tgccaatgat cagcggacgt ggtctggggc acacaggagg caccttggat 660

aagctggagt ctattcctgg attcaatgtc atccagagcc cagagcagat gcaagtgctg 720

ctggaccagg cgggctgctg tatcgtgggt cagagtgagc agctggttcc tgcggacgga 780

atcctatatg cagccagaga tgtgacagcc accgtggaca gcctgccact catcacagcc 840

tccattctca gtaagaaact cgtggagggg ctgtccgctc tggtggtgga cgttaagttc 900

ggaggggccg ccgtcttccc caaccaggag caggcccggg agctggcaaa gacgctggtt 960

ggcgtgggag ccagcctagg gcttcgggtc gcggcagcgc tgaccgccat ggacaagccc 1020

ctgggtcgct gcgtgggcca cgccctggag gtggaggagg cgctgctctg catggacggc 1080

gcaggcccgc cagacttaag ggacctggtc accacgctcg gggcgccct gctctggctc 1140

agcggacacg cggggactca ggcccagggc gctgcccggg tggccgcggc gctggacgac 1200

ggctcggccc ttggccgctt cgagcggatg ctggcggcgc agggcgtgga tcccggtctg 1260

gcccgagccc tgtgctcggg aagtcccgca gaacgccggc agctgctgcc tcgcgcccgg 1320

gagcaggagg agctgctggc gcccgcagat gcccctctcc ccgcaggcac cgtggagctg 1380

gtccgggcgc tgccgctggc gctggtgctg cacgagctcg ggccgggcg cagccgcgct 1440

ggggagccgc tccgcctggg ggtgggcgca gagctgctgg tcgacgtggg tcagaggctg 1500

cgccgtggga ccccctggct ccgcgtgcac cgggacggcc ccgcgctcag cggcccgcag 1560

agccgcgccc tgcaggaggc gctcgtactc tccgaccgcg cgccattcgc cgccccctcg 1620

cccttcgcag agctcgttct gccgccgcag caataaagct cctttgccgc gaaaaaaaaa 1680

aa 1682

<210> 21
<211> 1679
<212> DNA
<213> Homo sapiens

<400> 21
cgactgccga gctccgccct ccaggcggcc ccacccgcct gccgtcctgg ggcgccgccg 60

ccccgccgcc ggcagtggac cgctgtgcgc gaaccctgaa ccctacggtc ccgacccgcg 120

ggcgaggccg ggtacctggg ctgggatccg gagcaagcgg gcgagggcag cgccctaagc 180

aggcatcccc gcaggcccgg agcgatggca gccttgatga ccccgggaac cggggcccca 240

cccgcgcctg gtgacttctc cggggaaggg agccagggac ttcccgaccc ttcgccagag 300

cccaagcagc tcccggagct gatccgcatg aagcgagacg gaggccgcct gagcgaagcg 360

gacatcaggg gcttcgtggc cgctgtggtg aatgggagcg cgcagggcgc acagatcggg 420

gccatgctga tggccatccg acttcggggc atggatctgg aggagacctc ggtgctgacc 480

caggccctgg ctcagtcggg acagcagctg gagtggccag aggcctggcg ccagcagctt 540

gtggacaagc attccacagg gggtgtgggt gacaaggtca gcctggtcct cgcacctgcc 600

ctggcggcat gtggctgcaa ggtgccaatg atcagcggac gtggtctggg gcacacagga 660

ggcaccttgg ataagctgga gtctattcct ggattcaatg tcatccagag cccagagcag 720

atgcaagtgc tgctggacca ggcgggctgc tgtatcgtgg gtcagagtga gcagctggtt 780

cctgcggacg gaatcctata tgcagccaga gatgtgacag ccaccgtgga cagcctgcca 840

ctcatcacag cctccattct cagtaagaaa ctcgtggagg ggctgtccgc tctggtggtg 900

gacgttaagt tcggaggggc cgccgtcttc cccaaccagg agcaggcccg ggagctggca 960

aagacgctgg ttggcgtggg agccagccta gggcttcggg tcgcggcagc gctgaccgcc 1020

atggacaagc ccctgggtcg ctgcgtgggc cacgccctgg aggtggagga ggcgctgctc 1080

tgcatggacg gcgcaggccc gccagactta agggacctgg tcaccacgct cggggggcgcc 1140

ctgctctggc tcagcggaca cgcggggact caggcccagg gcgctgcccg ggtggccgcg 1200

gcgctggacg acggctcggc ccttggccgc ttcgagcgga tgctggcggc gcagggcgtg 1260

gatcccggtc tggcccgagc cctgtgctcg ggaagtcccg cagaacgccg gcagctgctg 1320

cctcgcgccc gggagcagga ggagctgctg gcgcccgcag atggcaccgt ggagctggtc 1380

cgggcgctgc cgctggcgct ggtgctgcac gagctcgggg ccgggcgcag ccgcgctggg 1440

gagccgctcc gcctgggggt gggcgcagag ctgctggtcg acgtgggtca gaggctgcgc 1500

cgtgggaccc cctggctccg cgtgcaccgg gacggccccg cgctcagcgg cccgcagagc 1560

cgcgccctgc aggaggcgct cgtactctcc gaccgcgcgc cattcgccgc cccctcgccc 1620

ttcgcagagc tcgttctgcc gccgcagcaa taaagctcct ttgccgcgaa aaaaaaaaa 1679

<210> 22
<211> 1718
<212> DNA
<213> Homo sapiens

<400> 22
cgactgccga gctccgccct ccaggcggcc ccacccgcct gccgtcctgg ggcgccgccg 60

ccccgccgcc ggcagtggac cgctgtgcgc gaaccctgaa ccctacggtc ccgacccgcg 120

ggcgaggccg ggtacctggg ctgggatccg gagcaagcgg gcgagggcag cgccctaagc 180

aggccgcaga ccccggacgt gtcaggcatc cccgcaggcc cggagcgatg gcagccttga 240

tgaccccggg aaccggggcc ccacccgcgc ctggtgactt ctccggggaa gggagccagg 300

gacttcccga cccttcgcca gagcccaagc agctcccgga gctgatccgc atgaagcgag 360

acggaggccg cctgagcgaa gcggacatca ggggcttcgt ggccgctgtg gtgaatggga 420

gcgcgcaggg cgcacagatc ggggccatgc tgatggccat ccgacttcgg ggcatggatc 480

tggaggagac ctcggtgctg acccaggccc tggctcagtc gggacagcag ctggagtggc 540

cagaggcctg gcgccagcag cttgtggaca agcattccac aggggggtgtg ggtgacaagg 600

tcagcctggt cctcgcacct gccctggcgg catgtggctg caaggtgcca atgatcagcg 660

gacgtggtct ggggcacaca ggaggcacct tggataagct ggagtctatt cctggattca 720

atgtcatcca gagcccagag cagatgcaag tgctgctgga ccaggcgggc tgctgtatcg 780

tgggtcagag tgagcagctg gttcctgcgg acggaatcct atatgcagcc agagatgtga 840

cagccaccgt ggacagcctg ccactcatca cagcctccat tctcagtaag aaactcgtgg 900

aggggctgtc cgctctggtg gtggacgtta agttcggagg ggccgccgtc ttccccaacc 960

aggagcaggc ccgggagctg gcaaagacgc tggttggcgt gggagccagc ctagggcttc 1020

gggtcgcggc agcgctgacc gccatggaca agcccctggg tcgctgcgtg ggccacgccc 1080

tggaggtgga ggaggcgctg ctctgcatgg acggcgcagg cccgccagac ttaagggacc 1140

tggtcaccac gctcgggggc gccctgctct ggctcagcgg acacgcgggg actcaggccc 1200

agggcgctgc ccgggtggcc gcggcgctgg acgacggctc ggcccttggc cgcttcgagc 1260

ggatgctggc ggcgcagggc gtggatcccg gtctggcccg agccctgtgc tcgggaagtc 1320

ccgcagaacg ccggcagctg ctgcctcgcg cccgggagca ggaggagctg ctggcgcccg 1380

cagatggcac cgtggagctg gtccgggcgc tgccgctggc gctggtgctg cacgagctcg 1440

gggccgggcg cagccgcgct ggggagccgc tccgcctggg ggtgggcgca gagctgctgg 1500

tcgacgtggg tcagaggctg cgccgtggga cccccctggct ccgcgtgcac cgggacggcc 1560

ccgcgctcag cggcccgcag agccgcgccc tgcaggaggc gctcgtactc tccgaccgcg 1620

cgccattcgc cgccccctcg cccttcgcag agctcgttct gccgccgcag caataaagct 1680

cctttgccgc gaaaaaaaaa aaaaaaaaaa aaaaaaaa 1718

<210> 23
<211> 1851
<212> DNA
<213> Homo sapiens

<400> 23
cgactgccga gctccgccct ccaggcggcc ccacccgcct gccgtcctgg ggcgccgccg 60

ccccgccgcc ggcagtggac cgctgtgcgc gaaccctgaa ccctacggtc ccgacccgcg 120

ggcgaggccg ggtacctggg ctgggatccg gagcaagcgg gcgagggcag cgccctaagc 180

aggtacgggc ggggctcaag tcgcgaggcg gggaagcggg aggcagacac ggacgagggc 240

gacacagaca cgggaccgag gggcggacac cggagagaca cgggaaaggg gtcgggacag 300

gagcacgtgg ctcagacacc gacgccggga ggccgcagac cccggacgtg tcaggcatcc 360

ccgcaggccc ggagcgatgg cagccttgat gaccccggga accggggccc cacccgcgcc 420

tggtgacttc tccggggaag ggagccaggg acttcccgac ccttcgccag agcccaagca 480

gctcccggag ctgatccgca tgaagcgaga cggaggccgc ctgagcgaag cggacatcag 540

gggcttcgtg gccgctgtgg tgaatgggag cgcgcagggc gcacagatcg gggccatgct 600

gatggccatc cgacttcggg gcatggatct ggaggagacc tcggtgctga cccaggccct 660

ggctcagtcg ggacagcagc tggagtggcc agaggcctgg cgccagcagc ttgtggacaa 720

gcattccaca gggggtgtgg gtgacaaggt cagcctggtc ctcgcacctg ccctggcggc 780

atgtggctgc aaggtgccaa tgatcagcgg acgtggtctg ggcacacag gaggcacctt 840

ggataagctg gagtctattc ctggattcaa tgtcatccag agcccagagc agatgcaagt 900

gctgctggac caggcgggct gctgtatcgt gggtcagagt gagcagctgg ttcctgcgga 960

cggaatccta tatgcagcca gagatgtgac agccaccgtg gacagcctgc cactcatcac 1020

agcctccatt ctcagtaaga aactcgtgga ggggctgtcc gctctggtgg tggacgttaa 1080

gttcggaggg gccgccgtct tccccaacca ggagcaggcc cgggagctgg caaagacgct 1140

ggttggcgtg ggagccagcc tagggcttcg ggtcgcggca gcgctgaccg ccatggacaa 1200

gcccctgggt cgctgcgtgg gccacgccct ggaggtggag gaggcgctgc tctgcatgga 1260

cggcgcaggc ccgccagact taagggacct ggtcaccacg ctcgggggcg ccctgctctg 1320

gctcagcgga cacgcgggga ctcaggccca gggcgctgcc cgggtggccg cggcgctgga 1380

cgacggctcg gcccttggcc gcttcgagcg gatgctggcg gcgcagggcg tggatcccgg 1440

tctggcccga gccctgtgct cgggaagtcc cgcagaacgc cggcagctgc tgcctcgcgc 1500

ccgggagcag gaggagctgc tggcgcccgc agatggcacc gtggagctgg tccgggcgct 1560

gccgctggcg ctggtgctgc acgagctcgg ggccgggcgc agccgcgctg gggagccgct 1620

ccgcctgggg gtgggcgcag agctgctggt cgacgtgggt cagaggctgc gccgtgggac 1680

cccctggctc cgcgtgcacc gggacggccc cgcgctcagc ggcccgcaga gccgcgccct 1740

gcaggaggcg ctcgtactct ccgaccgcgc gccattcgcc gccccctcgc ccttcgcaga 1800

gctcgttctg ccgccgcagc aataaagctc ctttgccgcg aaaaaaaaaa a 1851

<210> 24
<211> 288
<212> PRT
<213> Homo sapiens

<400> 24

Met Gly His Thr Arg Arg Gln Gly Thr Ser Pro Ser Lys Cys Pro Tyr
1 5 10 15

Leu Asn Phe Phe Gln Leu Leu Val Leu Ala Gly Leu Ser His Phe Cys
20 25 30

Ser Gly Val Ile His Val Thr Lys Glu Val Lys Glu Val Ala Thr Leu
35 40 45

Ser Cys Gly His Asn Val Ser Val Glu Glu Leu Ala Gln Thr Arg Ile
50 55 60

Tyr Trp Gln Lys Glu Lys Lys Met Val Leu Thr Met Met Ser Gly Asp
65 70 75 80

Met Asn Ile Trp Pro Glu Tyr Lys Asn Arg Thr Ile Phe Asp Ile Thr
85 90 95

Asn Asn Leu Ser Ile Val Ile Leu Ala Leu Arg Pro Ser Asp Glu Gly
100 105 110

Thr Tyr Glu Cys Val Val Leu Lys Tyr Glu Lys Asp Ala Phe Lys Arg
115 120 125

Glu His Leu Ala Glu Val Thr Leu Ser Val Lys Ala Asp Phe Pro Thr
130 135 140

Pro Ser Ile Ser Asp Phe Glu Ile Pro Thr Ser Asn Ile Arg Arg Ile
145 150 155 160

Ile Cys Ser Thr Ser Gly Gly Phe Pro Glu Pro His Leu Ser Trp Leu
165 170 175

```
Glu Asn Gly Glu Glu Leu Asn Ala Ile Asn Thr Thr Val Ser Gln Asp
            180                 185                 190

Pro Glu Thr Glu Leu Tyr Ala Val Ser Ser Lys Leu Asp Phe Asn Met
        195                 200                 205

Thr Thr Asn His Ser Phe Met Cys Leu Ile Lys Tyr Gly His Leu Arg
    210                 215                 220

Val Asn Gln Thr Phe Asn Trp Asn Thr Thr Lys Gln Glu His Phe Pro
225                 230                 235                 240

Asp Asn Leu Leu Pro Ser Trp Ala Ile Thr Leu Ile Ser Val Asn Gly
                245                 250                 255

Ile Phe Val Ile Cys Cys Leu Thr Tyr Cys Phe Ala Pro Arg Cys Arg
            260                 265                 270

Glu Arg Arg Arg Asn Glu Arg Leu Arg Arg Glu Ser Val Arg Pro Val
        275                 280                 285


<210>  25
<211>  105
<212>  PRT
<213>  Homo sapiens

<400>  25

Met Arg Gly Ala Thr Arg Val Ser Ile Met Leu Leu Leu Val Thr Val
1               5                   10                  15

Ser Asp Cys Ala Val Ile Thr Gly Ala Cys Glu Arg Asp Val Gln Cys
            20                  25                  30

Gly Ala Gly Thr Cys Cys Ala Ile Ser Leu Trp Leu Arg Gly Leu Arg
        35                  40                  45

Met Cys Thr Pro Leu Gly Arg Glu Gly Glu Glu Cys His Pro Gly Ser
    50                  55                  60

His Lys Val Pro Phe Phe Arg Lys Arg Lys His His Thr Cys Pro Cys
65                  70                  75                  80

Leu Pro Asn Leu Leu Cys Ser Arg Phe Pro Asp Gly Arg Tyr Arg Cys
                85                  90                  95

Ser Met Asp Leu Lys Asn Ile Asn Phe
            100                 105
```

<210> 26
<211> 200
<212> PRT
<213> Homo sapiens

<400> 26

```
Met Ala Ala Ala Trp Thr Val Val Leu Val Thr Leu Val Leu Gly Leu
1               5                   10                  15

Ala Val Ala Gly Pro Val Pro Thr Ser Lys Pro Thr Thr Thr Gly Lys
            20                  25                  30

Gly Cys His Ile Gly Arg Phe Lys Ser Leu Ser Pro Gln Glu Leu Ala
        35                  40                  45

Ser Phe Lys Lys Ala Arg Asp Ala Leu Glu Glu Ser Leu Lys Leu Lys
    50                  55                  60

Asn Trp Ser Cys Ser Ser Pro Val Phe Pro Gly Asn Trp Asp Leu Arg
65                  70                  75                  80

Leu Leu Gln Val Arg Glu Arg Pro Val Ala Leu Glu Ala Glu Leu Ala
                85                  90                  95

Leu Thr Leu Lys Val Leu Glu Ala Ala Ala Gly Pro Ala Leu Glu Asp
            100                 105                 110

Val Leu Asp Gln Pro Leu His Thr Leu His His Ile Leu Ser Gln Leu
        115                 120                 125

Gln Ala Cys Ile Gln Pro Gln Pro Thr Ala Gly Pro Arg Pro Arg Gly
    130                 135                 140

Arg Leu His His Trp Leu His Arg Leu Gln Glu Ala Pro Lys Lys Glu
145                 150                 155                 160

Ser Ala Gly Cys Leu Glu Ala Ser Val Thr Phe Asn Leu Phe Arg Leu
                165                 170                 175

Leu Thr Arg Asp Leu Lys Tyr Val Ala Asp Gly Asn Leu Cys Leu Arg
            180                 185                 190

Thr Ser Thr His Pro Glu Ser Thr
        195                 200
```

<210> 27
<211> 640

<212> PRT
<213> Homo sapiens

<400> 27

Met Ser Glu Arg Lys Glu Gly Arg Gly Lys Gly Lys Gly Lys Lys Lys
1 5 10 15

Glu Arg Gly Ser Gly Lys Lys Pro Glu Ser Ala Ala Gly Ser Gln Ser
20 25 30

Pro Ala Leu Pro Pro Arg Leu Lys Glu Met Lys Ser Gln Glu Ser Ala
35 40 45

Ala Gly Ser Lys Leu Val Leu Arg Cys Glu Thr Ser Ser Glu Tyr Ser
50 55 60

Ser Leu Arg Phe Lys Trp Phe Lys Asn Gly Asn Glu Leu Asn Arg Lys
65 70 75 80

Asn Lys Pro Gln Asn Ile Lys Ile Gln Lys Lys Pro Gly Lys Ser Glu
85 90 95

Leu Arg Ile Asn Lys Ala Ser Leu Ala Asp Ser Gly Glu Tyr Met Cys
100 105 110

Lys Val Ile Ser Lys Leu Gly Asn Asp Ser Ala Ser Ala Asn Ile Thr
115 120 125

Ile Val Glu Ser Asn Glu Ile Ile Thr Gly Met Pro Ala Ser Thr Glu
130 135 140

Gly Ala Tyr Val Ser Ser Glu Ser Pro Ile Arg Ile Ser Val Ser Thr
145 150 155 160

Glu Gly Ala Asn Thr Ser Ser Ser Thr Ser Thr Ser Thr Thr Gly Thr
165 170 175

Ser His Leu Val Lys Cys Ala Glu Lys Glu Lys Thr Phe Cys Val Asn
180 185 190

Gly Gly Glu Cys Phe Met Val Lys Asp Leu Ser Asn Pro Ser Arg Tyr
195 200 205

Leu Cys Lys Cys Gln Pro Gly Phe Thr Gly Ala Arg Cys Thr Glu Asn
210 215 220

Val Pro Met Lys Val Gln Asn Gln Glu Lys Ala Glu Glu Leu Tyr Gln
225 230 235 240

Lys Arg Val Leu Thr Ile Thr Gly Ile Cys Ile Ala Leu Leu Val Val
245 250 255

Gly Ile Met Cys Val Val Ala Tyr Cys Lys Thr Lys Lys Gln Arg Lys
260 265 270

Lys Leu His Asp Arg Leu Arg Gln Ser Leu Arg Ser Glu Arg Asn Asn
275 280 285

Met Met Asn Ile Ala Asn Gly Pro His His Pro Asn Pro Pro Pro Glu
290 295 300

Asn Val Gln Leu Val Asn Gln Tyr Val Ser Lys Asn Val Ile Ser Ser
305 310 315 320

Glu His Ile Val Glu Arg Glu Ala Glu Thr Ser Phe Ser Thr Ser His
325 330 335

Tyr Thr Ser Thr Ala His His Ser Thr Thr Val Thr Gln Thr Pro Ser
340 345 350

His Ser Trp Ser Asn Gly His Thr Glu Ser Ile Leu Ser Glu Ser His
355 360 365

Ser Val Ile Val Met Ser Ser Val Glu Asn Ser Arg His Ser Ser Pro
370 375 380

Thr Gly Gly Pro Arg Gly Arg Leu Asn Gly Thr Gly Gly Pro Arg Glu
385 390 395 400

Cys Asn Ser Phe Leu Arg His Ala Arg Glu Thr Pro Asp Ser Tyr Arg
405 410 415

Asp Ser Pro His Ser Glu Arg Tyr Val Ser Ala Met Thr Thr Pro Ala
420 425 430

Arg Met Ser Pro Val Asp Phe His Thr Pro Ser Ser Pro Lys Ser Pro
435 440 445

Pro Ser Glu Met Ser Pro Pro Val Ser Ser Met Thr Val Ser Met Pro
450 455 460

Ser Met Ala Val Ser Pro Phe Met Glu Glu Glu Arg Pro Leu Leu Leu
465 470 475 480

Val Thr Pro Pro Arg Leu Arg Glu Lys Lys Phe Asp His His Pro Gln

485 490 495

Gln Phe Ser Ser Phe His His Asn Pro Ala His Asp Ser Asn Ser Leu
500 505 510

Pro Ala Ser Pro Leu Arg Ile Val Glu Asp Glu Glu Tyr Glu Thr Thr
515 520 525

Gln Glu Tyr Glu Pro Ala Gln Glu Pro Val Lys Lys Leu Ala Asn Ser
530 535 540

Arg Arg Ala Lys Arg Thr Lys Pro Asn Gly His Ile Ala Asn Arg Leu
545 550 555 560

Glu Val Asp Ser Asn Thr Ser Ser Gln Ser Ser Asn Ser Glu Ser Glu
565 570 575

Thr Glu Asp Glu Arg Val Gly Glu Asp Thr Pro Phe Leu Gly Ile Gln
580 585 590

Asn Pro Leu Ala Ala Ser Leu Glu Ala Thr Pro Ala Phe Arg Leu Ala
595 600 605

Asp Ser Arg Thr Asn Pro Ala Gly Arg Phe Ser Thr Gln Glu Glu Ile
610 615 620

Gln Ala Arg Leu Ser Ser Val Ile Ala Asn Gln Asp Pro Ile Ala Val
625 630 635 640

<210> 28
<211> 482
<212> PRT
<213> Homo sapiens

<400> 28

Met Ala Ala Leu Met Thr Pro Gly Thr Gly Ala Pro Pro Ala Pro Gly
1 5 10 15

Asp Phe Ser Gly Glu Gly Ser Gln Gly Leu Pro Asp Pro Ser Pro Glu
20 25 30

Pro Lys Gln Leu Pro Glu Leu Ile Arg Met Lys Arg Asp Gly Gly Arg
35 40 45

Leu Ser Glu Ala Asp Ile Arg Gly Phe Val Ala Ala Val Val Asn Gly
50 55 60

Ser Ala Gln Gly Ala Gln Ile Gly Ala Met Leu Met Ala Ile Arg Leu

65 70 75 80

Arg Gly Met Asp Leu Glu Glu Thr Ser Val Leu Thr Gln Ala Leu Ala
85 90 95

Gln Ser Gly Gln Gln Leu Glu Trp Pro Glu Ala Trp Arg Gln Gln Leu
100 105 110

Val Asp Lys His Ser Thr Gly Gly Val Gly Asp Lys Val Ser Leu Val
115 120 125

Leu Ala Pro Ala Leu Ala Ala Cys Gly Cys Lys Val Pro Met Ile Ser
130 135 140

Gly Arg Gly Leu Gly His Thr Gly Gly Thr Leu Asp Lys Leu Glu Ser
145 150 155 160

Ile Pro Gly Phe Asn Val Ile Gln Ser Pro Glu Gln Met Gln Val Leu
165 170 175

Leu Asp Gln Ala Gly Cys Cys Ile Val Gly Gln Ser Glu Gln Leu Val
180 185 190

Pro Ala Asp Gly Ile Leu Tyr Ala Ala Arg Asp Val Thr Ala Thr Val
195 200 205

Asp Ser Leu Pro Leu Ile Thr Ala Ser Ile Leu Ser Lys Lys Leu Val
210 215 220

Glu Gly Leu Ser Ala Leu Val Val Asp Val Lys Phe Gly Gly Ala Ala
225 230 235 240

Val Phe Pro Asn Gln Glu Gln Ala Arg Glu Leu Ala Lys Thr Leu Val
245 250 255

Gly Val Gly Ala Ser Leu Gly Leu Arg Val Ala Ala Ala Leu Thr Ala
260 265 270

Met Asp Lys Pro Leu Gly Arg Cys Val Gly His Ala Leu Glu Val Glu
275 280 285

Glu Ala Leu Leu Cys Met Asp Gly Ala Gly Pro Pro Asp Leu Arg Asp
290 295 300

Leu Val Thr Thr Leu Gly Gly Ala Leu Leu Trp Leu Ser Gly His Ala
305 310 315 320

Gly Thr Gln Ala Gln Gly Ala Ala Arg Val Ala Ala Ala Leu Asp Asp
325 330 335

Gly Ser Ala Leu Gly Arg Phe Glu Arg Met Leu Ala Ala Gln Gly Val
340 345 350

Asp Pro Gly Leu Ala Arg Ala Leu Cys Ser Gly Ser Pro Ala Glu Arg
355 360 365

Arg Gln Leu Leu Pro Arg Ala Arg Glu Gln Glu Glu Leu Leu Ala Pro
370 375 380

Ala Asp Gly Thr Val Glu Leu Val Arg Ala Leu Pro Leu Ala Leu Val
385 390 395 400

Leu His Glu Leu Gly Ala Gly Arg Ser Arg Ala Gly Glu Pro Leu Arg
405 410 415

Leu Gly Val Gly Ala Glu Leu Leu Val Asp Val Gly Gln Arg Leu Arg
420 425 430

Arg Gly Thr Pro Trp Leu Arg Val His Arg Asp Gly Pro Ala Leu Ser
435 440 445

Gly Pro Gln Ser Arg Ala Leu Gln Glu Ala Leu Val Leu Ser Asp Arg
450 455 460

Ala Pro Phe Ala Ala Pro Ser Pro Phe Ala Glu Leu Val Leu Pro Pro
465 470 475 480

Gln Gln

<210> 29
<211> 487
<212> PRT
<213> Homo sapiens

<400> 29

Met Ala Ala Leu Met Thr Pro Gly Thr Gly Ala Pro Pro Ala Pro Gly
1 5 10 15

Asp Phe Ser Gly Glu Gly Ser Gln Gly Leu Pro Asp Pro Ser Pro Glu
20 25 30

Pro Lys Gln Leu Pro Glu Leu Ile Arg Met Lys Arg Asp Gly Gly Arg
35 40 45

```
Leu Ser Glu Ala Asp Ile Arg Gly Phe Val Ala Ala Val Val Asn Gly
    50                  55                  60

Ser Ala Gln Gly Ala Gln Ile Gly Ala Met Leu Met Ala Ile Arg Leu
65                  70                  75                  80

Arg Gly Met Asp Leu Glu Glu Thr Ser Val Leu Thr Gln Ala Leu Ala
                85                  90                  95

Gln Ser Gly Gln Gln Leu Glu Trp Pro Glu Ala Trp Arg Gln Gln Leu
            100                 105                 110

Val Asp Lys His Ser Thr Gly Gly Val Gly Asp Lys Val Ser Leu Val
        115                 120                 125

Leu Ala Pro Ala Leu Ala Ala Cys Gly Cys Lys Val Pro Met Ile Ser
    130                 135                 140

Gly Arg Gly Leu Gly His Thr Gly Gly Thr Leu Asp Lys Leu Glu Ser
145                 150                 155                 160

Ile Pro Gly Phe Asn Val Ile Gln Ser Pro Glu Gln Met Gln Val Leu
                165                 170                 175

Leu Asp Gln Ala Gly Cys Cys Ile Val Gly Gln Ser Glu Gln Leu Val
            180                 185                 190

Pro Ala Asp Gly Ile Leu Tyr Ala Ala Arg Asp Val Thr Ala Thr Val
        195                 200                 205

Asp Ser Leu Pro Leu Ile Thr Ala Ser Ile Leu Ser Lys Lys Leu Val
    210                 215                 220

Glu Gly Leu Ser Ala Leu Val Val Asp Val Lys Phe Gly Gly Ala Ala
225                 230                 235                 240

Val Phe Pro Asn Gln Glu Gln Ala Arg Glu Leu Ala Lys Thr Leu Val
                245                 250                 255

Gly Val Gly Ala Ser Leu Gly Leu Arg Val Ala Ala Ala Leu Thr Ala
            260                 265                 270

Met Asp Lys Pro Leu Gly Arg Cys Val Gly His Ala Leu Glu Val Glu
        275                 280                 285

Glu Ala Leu Leu Cys Met Asp Gly Ala Gly Pro Pro Asp Leu Arg Asp
    290                 295                 300
```

Leu Val Thr Thr Leu Gly Gly Ala Leu Leu Trp Leu Ser Gly His Ala
305 310 315 320

Gly Thr Gln Ala Gln Gly Ala Ala Arg Val Ala Ala Ala Leu Asp Asp
325 330 335

Gly Ser Ala Leu Gly Arg Phe Glu Arg Met Leu Ala Ala Gln Gly Val
340 345 350

Asp Pro Gly Leu Ala Arg Ala Leu Cys Ser Gly Ser Pro Ala Glu Arg
355 360 365

Arg Gln Leu Leu Pro Arg Ala Arg Glu Gln Glu Glu Leu Leu Ala Pro
370 375 380

Ala Asp Ala Pro Leu Pro Ala Gly Thr Val Glu Leu Val Arg Ala Leu
385 390 395 400

Pro Leu Ala Leu Val Leu His Glu Leu Gly Ala Gly Arg Ser Arg Ala
405 410 415

Gly Glu Pro Leu Arg Leu Gly Val Gly Ala Glu Leu Leu Val Asp Val
420 425 430

Gly Gln Arg Leu Arg Arg Gly Thr Pro Trp Leu Arg Val His Arg Asp
435 440 445

Gly Pro Ala Leu Ser Gly Pro Gln Ser Arg Ala Leu Gln Glu Ala Leu
450 455 460

Val Leu Ser Asp Arg Ala Pro Phe Ala Ala Pro Ser Pro Phe Ala Glu
465 470 475 480

Leu Val Leu Pro Pro Gln Gln
485

<210> 30
<211> 482
<212> PRT
<213> Homo sapiens

<400> 30

Met Ala Ala Leu Met Thr Pro Gly Thr Gly Ala Pro Pro Ala Pro Gly
1 5 10 15

Asp Phe Ser Gly Glu Gly Ser Gln Gly Leu Pro Asp Pro Ser Pro Glu
20 25 30

```
Pro Lys Gln Leu Pro Glu Leu Ile Arg Met Lys Arg Asp Gly Gly Arg
        35                  40                  45

Leu Ser Glu Ala Asp Ile Arg Gly Phe Val Ala Ala Val Val Asn Gly
    50                  55                  60

Ser Ala Gln Gly Ala Gln Ile Gly Ala Met Leu Met Ala Ile Arg Leu
65                  70                  75                  80

Arg Gly Met Asp Leu Glu Glu Thr Ser Val Leu Thr Gln Ala Leu Ala
                85                  90                  95

Gln Ser Gly Gln Gln Leu Glu Trp Pro Glu Ala Trp Arg Gln Gln Leu
            100                 105                 110

Val Asp Lys His Ser Thr Gly Gly Val Gly Asp Lys Val Ser Leu Val
        115                 120                 125

Leu Ala Pro Ala Leu Ala Ala Cys Gly Cys Lys Val Pro Met Ile Ser
    130                 135                 140

Gly Arg Gly Leu Gly His Thr Gly Gly Thr Leu Asp Lys Leu Glu Ser
145                 150                 155                 160

Ile Pro Gly Phe Asn Val Ile Gln Ser Pro Glu Gln Met Gln Val Leu
                165                 170                 175

Leu Asp Gln Ala Gly Cys Cys Ile Val Gly Gln Ser Glu Gln Leu Val
            180                 185                 190

Pro Ala Asp Gly Ile Leu Tyr Ala Ala Arg Asp Val Thr Ala Thr Val
        195                 200                 205

Asp Ser Leu Pro Leu Ile Thr Ala Ser Ile Leu Ser Lys Lys Leu Val
    210                 215                 220

Glu Gly Leu Ser Ala Leu Val Val Asp Val Lys Phe Gly Gly Ala Ala
225                 230                 235                 240

Val Phe Pro Asn Gln Glu Gln Ala Arg Glu Leu Ala Lys Thr Leu Val
                245                 250                 255

Gly Val Gly Ala Ser Leu Gly Leu Arg Val Ala Ala Ala Leu Thr Ala
            260                 265                 270

Met Asp Lys Pro Leu Gly Arg Cys Val Gly His Ala Leu Glu Val Glu
        275                 280                 285
```

```
Glu Ala Leu Leu Cys Met Asp Gly Ala Gly Pro Pro Asp Leu Arg Asp
    290                 295                 300

Leu Val Thr Thr Leu Gly Gly Ala Leu Leu Trp Leu Ser Gly His Ala
305                 310                 315                 320

Gly Thr Gln Ala Gln Gly Ala Ala Arg Val Ala Ala Ala Leu Asp Asp
                325                 330                 335

Gly Ser Ala Leu Gly Arg Phe Glu Arg Met Leu Ala Ala Gln Gly Val
            340                 345                 350

Asp Pro Gly Leu Ala Arg Ala Leu Cys Ser Gly Ser Pro Ala Glu Arg
        355                 360                 365

Arg Gln Leu Leu Pro Arg Ala Arg Glu Gln Glu Glu Leu Leu Ala Pro
    370                 375                 380

Ala Asp Gly Thr Val Glu Leu Val Arg Ala Leu Pro Leu Ala Leu Val
385                 390                 395                 400

Leu His Glu Leu Gly Ala Gly Arg Ser Arg Ala Gly Glu Pro Leu Arg
                405                 410                 415

Leu Gly Val Gly Ala Glu Leu Leu Val Asp Val Gly Gln Arg Leu Arg
            420                 425                 430

Arg Gly Thr Pro Trp Leu Arg Val His Arg Asp Gly Pro Ala Leu Ser
        435                 440                 445

Gly Pro Gln Ser Arg Ala Leu Gln Glu Ala Leu Val Leu Ser Asp Arg
    450                 455                 460

Ala Pro Phe Ala Ala Pro Ser Pro Phe Ala Glu Leu Val Leu Pro Pro
465                 470                 475                 480

Gln Gln


<210>  31
<211>  482
<212>  PRT
<213>  Homo sapiens

<400>  31

Met Ala Ala Leu Met Thr Pro Gly Thr Gly Ala Pro Pro Ala Pro Gly
1               5                   10                  15
```

```
Asp Phe Ser Gly Glu Gly Ser Gln Gly Leu Pro Asp Pro Ser Pro Glu
            20                  25                  30

Pro Lys Gln Leu Pro Glu Leu Ile Arg Met Lys Arg Asp Gly Gly Arg
        35                  40                  45

Leu Ser Glu Ala Asp Ile Arg Gly Phe Val Ala Ala Val Val Asn Gly
    50                  55                  60

Ser Ala Gln Gly Ala Gln Ile Gly Ala Met Leu Met Ala Ile Arg Leu
65                  70                  75                  80

Arg Gly Met Asp Leu Glu Glu Thr Ser Val Leu Thr Gln Ala Leu Ala
                85                  90                  95

Gln Ser Gly Gln Gln Leu Glu Trp Pro Glu Ala Trp Arg Gln Gln Leu
            100                 105                 110

Val Asp Lys His Ser Thr Gly Gly Val Gly Asp Lys Val Ser Leu Val
        115                 120                 125

Leu Ala Pro Ala Leu Ala Ala Cys Gly Cys Lys Val Pro Met Ile Ser
    130                 135                 140

Gly Arg Gly Leu Gly His Thr Gly Gly Thr Leu Asp Lys Leu Glu Ser
145                 150                 155                 160

Ile Pro Gly Phe Asn Val Ile Gln Ser Pro Glu Gln Met Gln Val Leu
                165                 170                 175

Leu Asp Gln Ala Gly Cys Cys Ile Val Gly Gln Ser Glu Gln Leu Val
            180                 185                 190

Pro Ala Asp Gly Ile Leu Tyr Ala Ala Arg Asp Val Thr Ala Thr Val
        195                 200                 205

Asp Ser Leu Pro Leu Ile Thr Ala Ser Ile Leu Ser Lys Lys Leu Val
    210                 215                 220

Glu Gly Leu Ser Ala Leu Val Val Asp Val Lys Phe Gly Gly Ala Ala
225                 230                 235                 240

Val Phe Pro Asn Gln Glu Gln Ala Arg Glu Leu Ala Lys Thr Leu Val
                245                 250                 255

Gly Val Gly Ala Ser Leu Gly Leu Arg Val Ala Ala Ala Leu Thr Ala
```

260 265 270

Met Asp Lys Pro Leu Gly Arg Cys Val Gly His Ala Leu Glu Val Glu
275 280 285

Glu Ala Leu Leu Cys Met Asp Gly Ala Gly Pro Pro Asp Leu Arg Asp
290 295 300

Leu Val Thr Thr Leu Gly Gly Ala Leu Leu Trp Leu Ser Gly His Ala
305 310 315 320

Gly Thr Gln Ala Gln Gly Ala Ala Arg Val Ala Ala Ala Leu Asp Asp
325 330 335

Gly Ser Ala Leu Gly Arg Phe Glu Arg Met Leu Ala Ala Gln Gly Val
340 345 350

Asp Pro Gly Leu Ala Arg Ala Leu Cys Ser Gly Ser Pro Ala Glu Arg
355 360 365

Arg Gln Leu Leu Pro Arg Ala Arg Glu Gln Glu Glu Leu Leu Ala Pro
370 375 380

Ala Asp Gly Thr Val Glu Leu Val Arg Ala Leu Pro Leu Ala Leu Val
385 390 395 400

Leu His Glu Leu Gly Ala Gly Arg Ser Arg Ala Gly Glu Pro Leu Arg
405 410 415

Leu Gly Val Gly Ala Glu Leu Leu Val Asp Val Gly Gln Arg Leu Arg
420 425 430

Arg Gly Thr Pro Trp Leu Arg Val His Arg Asp Gly Pro Ala Leu Ser
435 440 445

Gly Pro Gln Ser Arg Ala Leu Gln Glu Ala Leu Val Leu Ser Asp Arg
450 455 460

Ala Pro Phe Ala Ala Pro Ser Pro Phe Ala Glu Leu Val Leu Pro Pro
465 470 475 480

Gln Gln

<210> 32
<211> 482
<212> PRT
<213> Homo sapiens

<400> 32

Met Ala Ala Leu Met Thr Pro Gly Thr Gly Ala Pro Pro Ala Pro Gly
1               5                   10                  15

Asp Phe Ser Gly Glu Gly Ser Gln Gly Leu Pro Asp Pro Ser Pro Glu
            20                  25                  30

Pro Lys Gln Leu Pro Glu Leu Ile Arg Met Lys Arg Asp Gly Gly Arg
        35                  40                  45

Leu Ser Glu Ala Asp Ile Arg Gly Phe Val Ala Ala Val Val Asn Gly
    50                  55                  60

Ser Ala Gln Gly Ala Gln Ile Gly Ala Met Leu Met Ala Ile Arg Leu
65                  70                  75                  80

Arg Gly Met Asp Leu Glu Glu Thr Ser Val Leu Thr Gln Ala Leu Ala
                85                  90                  95

Gln Ser Gly Gln Gln Leu Glu Trp Pro Glu Ala Trp Arg Gln Gln Leu
            100                 105                 110

Val Asp Lys His Ser Thr Gly Gly Val Gly Asp Lys Val Ser Leu Val
        115                 120                 125

Leu Ala Pro Ala Leu Ala Ala Cys Gly Cys Lys Val Pro Met Ile Ser
    130                 135                 140

Gly Arg Gly Leu Gly His Thr Gly Gly Thr Leu Asp Lys Leu Glu Ser
145                 150                 155                 160

Ile Pro Gly Phe Asn Val Ile Gln Ser Pro Glu Gln Met Gln Val Leu
                165                 170                 175

Leu Asp Gln Ala Gly Cys Cys Ile Val Gly Gln Ser Glu Gln Leu Val
            180                 185                 190

Pro Ala Asp Gly Ile Leu Tyr Ala Ala Arg Asp Val Thr Ala Thr Val
        195                 200                 205

Asp Ser Leu Pro Leu Ile Thr Ala Ser Ile Leu Ser Lys Lys Leu Val
    210                 215                 220

Glu Gly Leu Ser Ala Leu Val Val Asp Val Lys Phe Gly Gly Ala Ala
225                 230                 235                 240

Val Phe Pro Asn Gln Glu Gln Ala Arg Glu Leu Ala Lys Thr Leu Val
245 250 255

Gly Val Gly Ala Ser Leu Gly Leu Arg Val Ala Ala Ala Leu Thr Ala
260 265 270

Met Asp Lys Pro Leu Gly Arg Cys Val Gly His Ala Leu Glu Val Glu
275 280 285

Glu Ala Leu Leu Cys Met Asp Gly Ala Gly Pro Pro Asp Leu Arg Asp
290 295 300

Leu Val Thr Thr Leu Gly Gly Ala Leu Leu Trp Leu Ser Gly His Ala
305 310 315 320

Gly Thr Gln Ala Gln Gly Ala Ala Arg Val Ala Ala Ala Leu Asp Asp
325 330 335

Gly Ser Ala Leu Gly Arg Phe Glu Arg Met Leu Ala Ala Gln Gly Val
340 345 350

Asp Pro Gly Leu Ala Arg Ala Leu Cys Ser Gly Ser Pro Ala Glu Arg
355 360 365

Arg Gln Leu Leu Pro Arg Ala Arg Glu Gln Glu Glu Leu Leu Ala Pro
370 375 380

Ala Asp Gly Thr Val Glu Leu Val Arg Ala Leu Pro Leu Ala Leu Val
385 390 395 400

Leu His Glu Leu Gly Ala Gly Arg Ser Arg Ala Gly Glu Pro Leu Arg
405 410 415

Leu Gly Val Gly Ala Glu Leu Leu Val Asp Val Gly Gln Arg Leu Arg
420 425 430

Arg Gly Thr Pro Trp Leu Arg Val His Arg Asp Gly Pro Ala Leu Ser
435 440 445

Gly Pro Gln Ser Arg Ala Leu Gln Glu Ala Leu Val Leu Ser Asp Arg
450 455 460

Ala Pro Phe Ala Ala Pro Ser Pro Phe Ala Glu Leu Val Leu Pro Pro
465 470 475 480

Gln Gln